(19) Europäisches Patentamt
European Patent Office
Office européen des brevets

(11)  **EP 2 780 470 B1**

(12)  **EUROPEAN PATENT SPECIFICATION**

(45) Date of publication and mention
of the grant of the patent:
**14.09.2016 Bulletin 2016/37**

(51) Int Cl.:
***C12Q 1/68*** (2006.01)

(21) Application number: **12805938.3**

(86) International application number:
**PCT/EP2012/004792**

(22) Date of filing: **19.11.2012**

(87) International publication number:
**WO 2013/072069 (23.05.2013 Gazette 2013/21)**

(54) **METHOD FOR PERFORMING QUANTITATION ASSAYS**

VERFAHREN ZUR DURCHFÜHRUNG VON QUANTIFIZIERUNGSTESTS

PROCÉDÉ POUR EFFECTUER DES DOSAGES DE QUANTIFICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2011 PL 39702811**
**17.11.2011 PL 39702711**
**17.11.2011 PL 39702611**
**26.06.2012 PL 39967312**
**11.07.2012 PL 39990812**

(43) Date of publication of application:
**24.09.2014 Bulletin 2014/39**

(73) Proprietor: **Curiosity Diagnostics Sp. z o.o.**
**01-224 Warszawa (PL)**

(72) Inventors:
 • **GARSTECKI, Piotr**
  **PL-01-494 Warsaw (PL)**
 • **DEBSKI, Pawel Rafal**
  **PL-03-984 Warsaw (PL)**
 • **OSZMANIEC, Michal**
  **PL-00-459 Warsaw (PL)**
 • **KAMINSKI, Tomasz**
  **PL-23-212 Wilkolaz (PL)**
 • **WARCHULSKI, Adam**
  **PL-05-822 Milanowek (PL)**

(74) Representative: **Prinz & Partner mbB**
**Patentanwälte**
**Friedrichstrasse 90**
**10117 Berlin (DE)**

(56) References cited:
 • **KREUTZ JASON E ET AL: "Theoretical design and analysis of multivolume digital assays with wide dynamic range validated experimentally with microfluidic digital PCR.", ANALYTICAL CHEMISTRY 1 NOV 2011, vol. 83, no. 21, 1 November 2011 (2011-11-01), pages 8158-8168, XP002693075, ISSN: 1520-6882**
 • **CHANDLER D P: "Redefining relativity: Quantitative PCR at low template concentrations for industrial and environmental microbiology", JOURNAL OF INDUSTRIAL MICROBIOLOGY AND BIOTECHNOLOGY, vol. 21, no. 3, September 1998 (1998-09), pages 128-140, XP002693076, ISSN: 1367-5435**
 • **COCHRAN W G: "Estimation of bacterial densities by means of the 'most probable number'", BIOMETRICS 1950, vol. 6, no. 2, 1950, pages 105-116, XP002693077, ISSN: 0006-341X**

**Description**

**Technical Field:**

The present invention relates to a method for determining an estimate of a concentration of particles $E(C)$, wherein a sample of predetermined volume is divided into a number ($N$) of compartments, the ($N$) compartments comprise or consist of different sample volumes ($v_i$) and/or different dilution factors ($d_i$) of the sample, at least part of the particles that are present in any of the ($N$) compartments provide a measurable signal and the estimated concentration of particles $E(C)$ is a function of measured signals, as well as an apparatus for use in the inventive method, uses of the inventive method, a sample holder and a kit for use in the inventive method.

**Background art:**

In analytical chemistry the standard (or 'analogue') method to quantify the concentration of an analyte particle is to calibrate how it relates to a measurable amplitude of a physical quantity, e.g. the absorbance of light passing through the sample. The estimate $E(C)$ of the concentration $C$ of the analyte particles is then retrieved as function of the signal.

The Polymerase Chain Reaction (PCR) comprises one method of the prior art for quantifying concentration of nucleic acids. The PCR procedure generally allows for replicating fragments of DNA chains. Typically, the length of a replicated fragment does not exceed 10 thousand base pairs (bp), and in the quantitative PCR usually a shorter fragments are replicated. According to methods of the prior art the replicated DNA copies are labelled, for instance, with fluorescent dyes displaying strong fluorescence only after complex formation with a double-stranded DNA (dye-DNA complex) - for example SYBR Green or EvaGreen. [Advan. Physiol. Educ. 29:151-159, 2005] - to provide a measureable signal (fluorescence level). The fluorescence level increases proportionally with multiplication of the DNA fragment.

When target DNA fragments are present in the sample, their number increases very rapidly during the PCR process - geometrically as a function of the number of completed cycles. Fast increase of the number of copies is attractive in preparative applications and in qualitative diagnostic assays aiming at detecting the presence of the target DNA (for instance, as a proof for the presence of specific microorganisms in the sample). At the same time, the exponential growth (as a function of the number of cycles) of replications of the target DNA significantly hampers the assay of the initial number of target DNA copies in the sample before the PCR process was carried out. According to the prior art, the so called real-time PCR, provides methods, where fluorescence activity of the sample (*i.e.,* signal intensity) is monitored after each PCR cycle, and subsequently the initial DNA concentration in the sample is approximated by comparing fluorescence intensity from the sample as a function of time with calibration curves. The accuracy of these procedures is limited. Their additional drawback is that additional comparative (reference) experiments must be performed for each quantitation.

In 1999 Vogelstein and Kinzler proposed a digital assay that uses the ability of the observer to detect a binary stochastic function $k(C)$ adopting either a positive value *(k = 1)* when the inspected volume happens to contain at least one analyte particle or a negative value ($k = 0$) otherwise. The concentration is estimated on the basis of the fraction of 'positive' compartments (i.e. ones that yield $k = 1$). As the assays require strong amplification of the presence of the analyte particle, their key applications are generally in quantitative PCR or Enzyme Linked Immunosorbent Assay (ELISA).

According to a digital PCR method of the prior art, a PCR mixture (sample and reagents) with a total volume V is partitioned into $N$ compartments with equal volumes $v = V/N.$ Then, PCR reaction cycles are carried out in all compartments (preferably simultaneously), e.g. by cyclic variation of temperature. Subsequently, typically fluorescence intensity is measured after completion of the PCR process (endpoint measurements) separately for each compartment, and to the number of compartments, where the reaction is completed, the compartments that yield a 'positive' signal are counted and the number of positive compartments $K$ together with $N$ are used to calculate the estimate $E(M)$ of the actual initial number $M$ of target DNA copies in the tested sample.

The development of the concept of digital assay offered a new paradigm in analytical chemistry. It allows the absolute quantification without calibration of the experimental set-up. Also, it benefits from simplified laboratory routines, *i.e.* end-point measurement, and relatively plain mathematical tools needed to interpret the experimental results. The digital assays of the prior art are, however, affected by limitations.

1. The maximum number of analyte particles $M$ to be determined is directly proportional to the number $N$ of compartments in the assay. In many applications and potential applications of diagnostic quantitation assays it is preferred that the span of the dynamic range ($\Omega = C^+/C^-$) (with $C^+$ representing the upper limit and $C^-$ representing the lower limit of estimated concentration of analyte particles in the assay) is large, for instance is equal to 1 million or more. To reach such a large span of the dynamic range in a standard digital PCR procedure, the sample must be partitioned into proportionally large number of compartments - in the said example - according to the state of knowledge in the field that overlooks unfavourably small precision at very small concentrations, into as many as 200,000 compartments or - actually - as shown in the description below, even 600,000 compartments. Partitioning a sample into such a huge number of compartments is although possible unfavourable, as such an assay requires specialized, complicated and expensive equipment to be performed. In particular, design of assays that aim to partition, amplify and inspect tens of thousands, or hundreds of thousands or millions of compartments require expensive technologies of micro-fabrication, automation and rapid and sensitive detection from multiple small volumes.

Furthermore, the precision and dynamic range achieved by the digital assays of the prior art cannot be independently tuned narrowing the range of applications and elevating the technical cost of the assays. Thus, it is not possible to obtain high precision (low standard deviation) in a narrow range of concentrations while using a small number $N$ of compartments using digital assays of the prior art.

The prior art provides a solution to increase the span of the dynamic range of the assay and reducing the number of compartments by combining classical digital quantitation assays with different dynamic ranges [Shen, F.; Sun, B.; Kreutz, J. E.; Davydova, E. K.; Du, W.; Reddy P. L.; Joseph, L. J.; Ismagilov, R. F., "Multiplexed Quantification of Nucleic Acids with Large Dynamic Range Using Multivolume Digital RT-PCR on a Rotational SlipChip Tested with HIV and Hepatitis C Viral Load", J. Am. Chem. Soc., Article ASAP (X 2011)]. The solution relies on a simultaneously performed assay for multiple sets of compartments. The compartments belonging to each set have the same volume. In the cited example, $N_z$ = 4 sets are used with $N_j$ = **160** compartments each ($j=1$ to $4$), with volumes of compartments equal to $v_j$=1, 5, 25 and 125 nL in each of four sets. The procedure consists in i) dividing the samples into sets and compartments, ii) performing simultaneously signal amplification, iii) counting separately the number $K_j$ of positive signals in each of $N_z$ sets and iv) calculating the most probable initial concentration of particles in the sample. The calculation is laborious and demanding, because of the requirement to calculate repeatedly the product

$$\prod_{j=1}^{N_z}\left\{\left[\frac{N_j!}{K_j!(N_j-K_j)!}\right][1-exp(-v_jC)]^{K_j}[exp(-v_jC)]^{N_j-K_j}\right\},$$ where the multiplication operator $\prod_{j=1}^{N_z}$ denotes the product of $N_z$ terms calculated for each of $N_z$ compartment families, each term comprising the probability of observing $K_j$ positive signals from the $j$-th family. Computation of the result requires an iterative calculation of the said product for each tested, hypothetical value of C within the test dynamic range, until a value C is found for which the above product assumes the maximum. The above procedure must be repeated after each measurement of signals from the sample, which hinders the analysis of the test result, requires a sufficiently fast electronic device or elongates the time needed to obtain the estimate of the number of particles in the sample. The above procedure would be in particular definitely unfavourable in the case of a large number (e.g. ten, or few tens, or hundred or more) of sets of compartments, characterised by that the compartments have the same volume within one set, but different volumes for each two different sets.

Furthermore, WO 2012/100198 A2 discloses methods for performing digital measurements with varying volumes and, thus, widening the dynamic range.

Although the aforementioned digital assays provide an increase in the dynamic range, the analysis is very laborious and the number of compartments is still high. Accordingly, it would be desirable to provide a method which is easier to be analyzed and which allows to use a smaller number of compartments to decrease the technological requirements for preparation of the sample holder, for the procedure of partitioning the sample, amplification and detection, for the apparatus that realizes these tasks and for the analysis of the result of the assay.

Accordingly, there exists a need of providing an improved method for determining an estimate of a concentration of particles E($C$), wherein the desired dynamic range and the desired precision of the assay can be independently tuned, and/or the mathematical analysis is easier, and/or wherein for a predetermined dynamic range and a predetermined standard deviation

> a) the total number (N) of compartments comprising the predetermined sample volume can be adjusted, preferably reduced and/or

> b) the total volume of a mixture comprising or consisting of sample, one or more reagents suitable for amplifying at least part of the particles to a measurable signal and optionally one or more diluents in all of the (N) compartments can be adjusted, preferably reduced and/or

> c) the volume, preferably the minimum or maximum volume of the mixture comprising or consisting of sample, one or more reagents suitable for amplifying at least part of the particles to a measurable signal and optionally one or more diluents in each of the (N) compartments can be predetermined.

**Brief Description of the Invention:**

The aforementioned needs are met in part or all by means of the claimed inventive subject matter. Preferred embodiments are in particular described in the dependent claims, the detailed description and/or the accompanying figures.

Accordingly a first aspect of the invention relates to a method for determining an estimate of a concentration of particles $E(C)$, wherein a sample of predetermined volume is divided into a number $(N)$ of compartments, at least part of the particles that are present in any of the $(N)$ compartments provide a measurable signal and the estimated concentration of particles $E(C)$ is a function of measured signals, characterized in that the method comprises or consists of

   a) determining the number $(N)$ of separate compartments, wherein the number $(N)$ is smaller or equal to the value of the function

$$N_{MAX} = A \cdot \ln\left(\frac{C^+}{C^-}\right)\Big/\sigma_{MAX}^2$$

   wherein $(A)$ represents a real number being the integer 6, wherein $(C^+)$ represents a predetermined upper limit of the interval of concentration $(C)$ to be estimated by the method, wherein $(C^-)$ represents a predetermined lower limit of the interval of concentration $(C)$ to be estimated by the method, wherein $(\sigma_{MAX})$ represents a predetermined maximum allowable relative standard deviation of the estimate of concentration $(C)$ of particles, wherein $C^- < C < C^+$, and

   b) determining a modulation factor $(z_i)$, wherein $(z_i)$ is a function of volumes $(v_i)$ and dilution factors $(d_i)$ of the sample in at least part of or all of the $(N)$ compartments and partitioning the sample into the $(N)$ compartments, wherein at least part of or all of the two or more of the $(N)$ compartments comprise or consist of different sample volumes $(v_i)$ and/or different dilution factors $(d_i)$ of the sample, wherein $(i)$ represents an index number of the $(N)$ compartments represented by the integers 0 to $N - 1$, and wherein $(v_i)$ represents the volume and $(d_i)$ represents the dilution factor of the sample in the compartment $(i)$.

Accordingly a second aspect of the invention relates to an apparatus for use in determining a concentration of particles in accordance with the inventive method characterized in that the apparatus is configured to

   a) determining a number $(N)$ of separate compartments, wherein the number $(N)$ is smaller or equal to the value of the function

$$N_{MAX} = A \cdot \ln\left(\frac{C^+}{C^-}\right)\Big/\sigma_{MAX}^2$$

   wherein $(A)$ represents a real number being the integer 6, Wherein $(C^+)$ represents a predetermined upper limit of the interval of concentration $(C)$ to be estimated by the method, Wherein $(C^-)$ represents a predetermined lower limit of the interval of concentration $(C)$ to be estimated by the method, Wherein $(\sigma_{MAX})$ represents a predetermined maximum allowable relative standard deviation of the estimate of concentration $(C)$ of particles, wherein $C^- < C < C^+$, and

   b) determining a modulation factor $(z_i)$, wherein $(z_i)$ is a function of volumes $(v_i)$ and dilution factors $(d_i)$ of the sample in at least part of or all of the $(N)$ compartments so that at least part of or all of the two or more of the $(N)$ compartments comprise or consist of different sample volumes $(v_i)$ and/or different dilution factors $(d_i)$ of the sample, wherein $(i)$ represents an index number of the $(N)$ compartments represented by the integers 0 to $N - 1$, and wherein $(v_i)$ represents the volume and $(d_i)$ represents the dilution factor of the sample of predetermined volume in the compartment $(i)$.

Accordingly a third aspect of the invention relates to a use of the inventive method or the inventive apparatus for

   a) adjusting, preferably reducing the total number $(N)$ of compartments comprising the predetermined sample volume and/or

   b) adjusting, preferably reducing the total volume of a mixture comprising or consisting of sample, one or more reagents suitable for amplifying at least part of the particles to a measurable signal and optionally one or more diluents in all of the $(N)$ compartments and/or

c) predetermining the volume, preferably the minimum or maximum volume of the mixture comprising or consisting of sample, one or more reagents suitable for amplifying at least part of the particles to a measurable signal and optionally one or more diluents in each of the ($N$) compartments and/or

d) predetermining the modulation factor ($z_i$), preferably the minimum or the maximum suitable modulation factor ($z_i$) for partitioning the predetermined sample volume into the ($N$) compartments.

Accordingly a fourth aspect of the invention relates to a use of a sample holder in an inventive method, characterized in that the sample holder is configured to,

a) comprising or consisting of a predetermined number ($N$) of compartments wherein the number ($N$) is smaller or equal to the value of the function

$$N_{MAX} = A \cdot \ln\left(\frac{C^+}{C^-}\right)/\sigma_{MAX}^2$$

wherein ($A$) represents a real number being the integer 6, Wherein ($C^+$) represents a predetermined upper limit of the interval of concentration ($C$) to be estimated by the method, Wherein ($C^-$) represents a predetermined lower limit of the interval of concentration ($C$) to be estimated by the method, Wherein ($\sigma_{MAX}$) represents a predetermined maximum allowable relative standard deviation of the estimate of concentration ($C$) of particles, wherein $C^- < C < C^+$, and

b) wherein the ($N$) compartments are configured to comprising the predetermined sample volume with a predetermined modulation factor ($z_i$), wherein ($z_i$) is a function of volumes ($v_i$) and dilution factors ($d_i$) of the sample in at least part of or all of the (N) compartments so that at least part of or all of the two or more of the ($N$) compartments can comprise or consist of different sample volumes ($v_i$) and/or different dilution factors ($d_i$) of the sample, wherein ($i$) represents an index number of the ($N$) compartments represented by the integers 0 to $N$ -1, and wherein ($v_i$) represents the volume and ($d_i$) represents the dilution factor of the sample in the compartment ($i$).

Accordingly a fifth aspect of the invention relates to a use of a kit comprising the inventive sample holder and one or more reagents suitable for amplifying at least part of particles comprised in the compartments of the sample holder to a measurable signal and optionally one or more suitable diluents for determining a concentration of particles, preferably for determining the concentration of particles in accordance with the inventive method.

The aforementioned inventive embodiments can - as far it is reasonable in view of a technical expert - comprise any possible combination of the preferred inventive embodiments, which are disclosed in the following and in particular in the dependent claims.

## Brief Description of the Drawings:

**Fig. 1a), 1b) and 1c):** Graphs displaying the combinations of the dynamic range and precision that can be achieved with assays comprising identical compartments and the inventive method, comparison of the number $N$ of compartments required to yield a given dynamic range and precision in accordance with the present invention.

**Fig. 2a) and 2b):** Graphs displaying the dynamic range $C^+/C^- = \Omega$ and relative standard deviation $\sigma$ provided by the inventive quantitation assay and the relation between the common ratio $x$ of geometric sequence and the number $N'$ of copies of compartments in accordance with the present invention

**Fig. 3a), 3b), 3c), 3d):** Inventive concept of active stripe, microstate and the precision of estimates derived from the assays in accordance with the present invention

**Fig. 4a), 4b), 4c):** Graphs displaying the standard deviation of the estimate of concentration of particles $E(C)$, for two different approaches (summation and microstate) of analyzing the signals from the compartments in accordance with the inventive method.

**Fig. 5a) and 5b):** Graphs displaying the probability distributions $p(\mu|C)$ for the first 10 (Fig 5a) and first 90 (Fig. 5b) most frequently occurring microstates in two different assays designed in accordance with the present invention

**Fig. 6:** Graph displaying the calibration correction function $f_{corr} = C/E(C)$ of initial concentration $C = M/V$ of particles for the inventive quantitation assay

**Fig. 7a), 7b) and 7c):** Graph displaying the sum of values $K = \sum w_i \cdot k_i$, with weights $w_i = 1/d_i$ as a function of the number of particles $M$ (Fig. 7a); Graph displaying the value of the estimate $E(C)$ of initial concentration of particles as a function of $K$ (Fig. 7b); Graph displaying the relative standard deviation $\sigma(C)$ of the estimate $E(C)$ as a function of the logarithm of initial concentration $C = M/V$ of particles for the inventive quantitation assay (Fig. 7c)

**Fig. 8a) and 8b):** Graphs displaying the results of 100 Monte Carlo (MC) simulations for inventive assays and 7 or 8 experiments as experimental and numerical verification of the performance of the inventive method.

**Fig. 9a) and 9b):** Graphs showing relative standard deviation, dynamic range and the estimate of concentration as a function of signal from assays comprising identical compartments and analyzed for the concentration of finite number of particles in the sample (analysis with non-independent random variables) and for the concentration of particles in the sample being scaled concentration of the reservoir (identical and independent random variables)

## Detailed Description of the Invention:

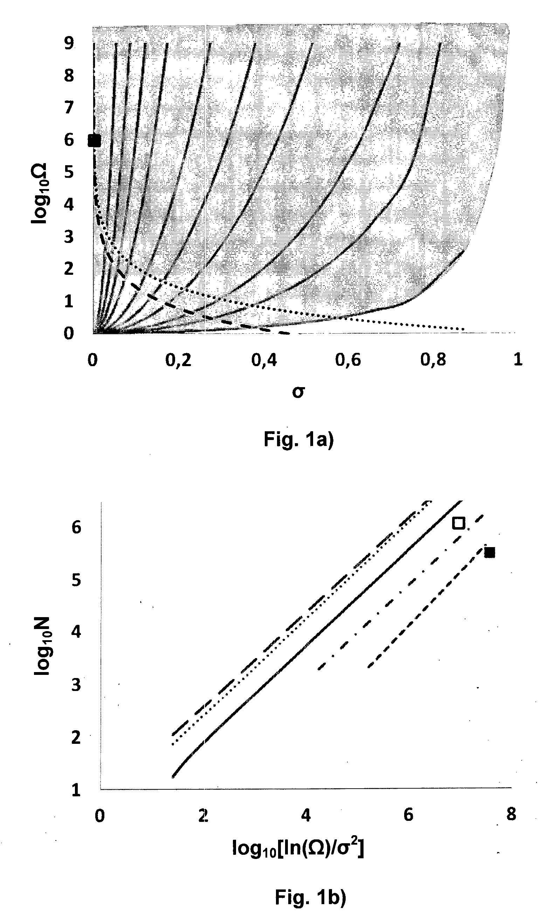

The inventors have surprisingly identified that by use of the embodiments of the present invention (parts A, B, C, D, E, and/or F, wherein the preferred inventive embodiments of part A, B, C, D, E and F can independently from each other be combined), in particular the inventive method, the inventive apparatus, the inventive sample holder and/or the inventive kit one or more needs underlying the present invention can be met (see in particular Figs. 1a), 1b) and 1c) and respective parts in detailed figure description).

Generally, the invention relies on the unexpected discovery that it is possible to preferably and significantly reduce the number of partitions required to perform an assay in a defined concentration range with specified precision and specified accuracy, provided that the estimated values of the number of target particles in individual partitions are different. Formally, the estimated number of particles in the $i$-th compartment is $m_i = C \cdot d_i \cdot v_i$, where $v_i$ means the volume of the $i$-th compartment, $d_i$ means the dilution factor of the $i$-th compartment and $C$ is the unknown concentration of in the sample. The essence of the present invention is that imposing the requirement that the values $m_i$ are not equal to each other, preferably that they are essentially different, and most preferably that they span approximately the same range of values as the required range of concentrations, for which the assay should provide an accurate estimate of the initial concentration of target particles, allows one to preferably reduce the number of partitions required to perform a quantitation assay of the initial number of particles in a defined range of concentrations and with a specified precision.

The inventors of the present invention have surprisingly discovered that by partitioning the sample in accordance with the inventively determined number $N$ of compartments and the determined modulation factor $z_i$ the span of the assay dynamic range $C^+/C^-$ and the maximum value of the relative standard deviation ($\sigma_{Max}$) of the estimate of the concentration $E(C)$ of particles can independently be predetermined, or in other words controlled or tuned (see in particular Fig. 1a) and respective part in the detailed figure description).

The inventive determination of estimate of concentration $E(C)$ of particles generally comprises determination of estimate of concentration $E(C_{sample})$ of particles in the sample as well as the determination of estimate of concentration $E(C_{reservoir})$ of particles in the reservoir or the determination of estimate of concentration $E(C_{specimen})$ of particles in the specimen, which is in detail explained in the following. The estimate of concentration E(C) is a function of the estimated number $E(M)$ of particles in the respective sources, e.g., sample volume, reservoir volume or specimen volume. Accordingly, in case estimate of concentration $E(C)$ of particles is used with respect to the present invention the term generally comprises the estimate of concentration $E(C_{sample})$ of particles in the sample, the estimate of concentration $E(C_{reservoir})$ of particles in the reservoir and the estimate of concentration $E(C_{specimen})$, unless stated otherwise.

Thus, in a preferred embodiment the present invention relates to the determination of the concentration of particles in the sample $E(C_{sample}) = E(M)/V_{sample}$, wherein $E(M)$ is the estimated number of particles in a sample of volume $V_{sample}$. In the procedure the volume of the sample is partitioned and mixed with appropriate volumes of reagents, to allow for amplification of the presence of particles in the compartments to a measurable signal and to obtain the desired volumes $v_i$ and dilution factors $d_i = (m_i/v_i)/(M/V_{sample})$, where $m_i$ is the estimated number of particles in $i$-th compartment. The quotients $(M/V_{sample})$ and $(m_i/v_i)$ can be coded as concentrations of particles in the sample ($C_{sample}$) and of particles in the $i$-th compartment ($C_{sample}$), yet as there is a finite number of particles in the sample, and placement of a particle in one compartment affects the probability of finding it in another compartment it has to be understood that these are not true concentrations as they only represent the estimated number of molecules scaled by the volume of either sample or compartment. As will be discussed below, the appropriate - and known in the prior art - approach to the analysis of the signals from compartments is based on the dependant random variables of placement of particles in the compartments. Such analysis yields the estimated number $E(M)$ of particles in the sample, that can be represented as the estimated concentration $E(C_{sample})$ of particles in the sample.

In an alternative preferred embodiment it is preferred that the sample volume $V_{sample}$ containing the particles of interest is prepared (e.g. by isolation or purification of particles) from a volume $V_{specimen}$ of a specimen. In such a case, the estimate $E(M)$ of the number of particles in the sample is the same as the estimate of the number of particles in the specimen. These can be represented either by the estimated concentration of particles in the sample $E(C_{sample})$ = $E(M)/V_{sample}$ or by estimated concentration of particles in the specimen $E(C_{specimen}) = E(C_{sample})(V_{sample}/V_{specimen})$ = $E(M)/V_{specimen}$.

In an alternative preferred embodiment of the present invention the volume $V_{specimen}$ of the specimen may be taken from a much larger volume of a reservoir (e.g. an organism, or environment). It may be then of interest, and is possible with the use of the present invention, to estimate the concentration $E(C_{reservoir})$ of particles in the reservoir. In such a case the placement of particles in the compartments formed by partitioning and optionally also diluting the sample is treated as a set of independent random variables. The probability of finding a particle in any given compartment is, within this approach, only a function of the volume of the compartment, the dilution ratio of the sample in the compartment and of the concentration $C_{sample}$ of particles in the sample which is in turn equal to the concentration of particles in the reservoir, scaled by the appropriate quotients of volumes of sample and of specimen. As a result of calculation based on the signals from the compartments the invention allows to retrieve the estimate of concentration of particles in the sample $E(C_{sample})$. The estimate of the concentration of particles in the reservoir is the same as the estimate of concentration of particles in the specimen: $E(C_{reservoir}) = E(C_{specimen}) = E(C_{sample})(V_{sample}/V_{specimen})$.

Alternatively, the estimate of the concentration $E(C_{reservoir})$ of the particles in the reservoir can also be preferably determined by transforming the estimate $E(M)$ of the number of particles in the sample obtained from a calculation based on a distribution of dependent variables. In most general terms, the probability distribution of concentration in the reservoir $\rho(C_{reservoir})$ is equal to an integral transform of the distribtuion of probability of the number of particles in the sample $\rho(M/V_{sample})$. In a simplified protocol, the estimate of $E(C_{reservoir})$ can preferably be determined by multiplying $E(M)/V_{sample}$ by a correction function $f_{corr}(E(M))$ and by scaling it by the quotient $V_{sample}/V_{specimen}$.

Thus, the inventive method of determining the estimate of concentration $E(C)$ of particles is also advantageous over the prior art, as it provides solutions for determining the estimate of concentration of particles in the sample $E(C_{sample})$ being an expression of the estimate of a finite number of particles distributed non-independently between compartments, divided by the volume of the sample, or for determining the estimate $E(C_{reservoir})$ of concentration of particles in the reservoir from which a specimen was taken, from which the sample was prepared, with $E(C_{reservoir})$ calculated from an estimate of concentration of particles on the basis of their independent placing in compartments.

**Embodiments of the invention part A:**

The present invention is particularly preferred, as it teaches how to modify the volume and/or dilution factor of the sample in the $i$-th compartment and, thus, allowing to independently tune the dynamic range and the precision of a quantification assay.

Furthermore, the inventive method teaches how to determine the number (N) of separate compartments and the modulation factor ($z_i$) for a predetermined upper limit ($C^+$) and lower limit ($C^-$) of the interval of concentration (C) to be estimated by the method as well as for a predetermined maximum allowable relative standard deviation ($\sigma_{MAX}$) of the estimate of concentration $E(C)$ of particles, so that

a) the total number ($N$) of compartments comprising the predetermined sample volume can be adjusted, preferably reduced and, *e.g.,* a wide dynamic range with good precision and small $N$ (e.g. $\Omega = 10^9$ with $\sigma < 50\%$ can be realized with $N = 60$), ii) a wide dynamic range with high precision and small $N$ (e.g. $\Omega = 10^9$, $\sigma < 10\%$, $N = 1615$) or iii) a narrow dynamic range with high precision and again small $N$ (e.g. $\Omega = 10^3$ with the benefit of obtaining $\sigma < 30\%$ with $N = 80$ and $\sigma < 10\%$ with $N = 631$) can be realized and/or

b) the total volume of a mixture comprising or consisting of sample, one or more reagents suitable for amplifying at least part of the particles to a measurable signal and optionally one or more diluents in all of the ($N$) compartments can be adjusted, preferably reduced and/or

c) the volume, preferably the minimum or maximum volume of the mixture comprising or consisting of sample, one or more reagents suitable for amplifying at least part of the particles to a measurable signal and optionally one or more diluents in each of the ($N$) compartments can be predetermined.

Furthermore the inventive method is preferably easier with respect to carrying out the mathematical analysis and may preferably provide feedback regarding the precision of the estimate of concentration $E(C)$ prior to carrying out the inventive procedural steps of amplification, measurement and assignment as well as determination of concentration, which will be in detail described with respect to the inventive concept of active stripe and microstate $\mu$ further down in the following description.

With respect to step a) of the inventive method the number ($N$) of compartments represents an integer 0 to $N$ - 1, wherein the integer is generally predetermined in accordance with the given function, wherein the integer is larger than the real results of the function, preferably wherein the integer is the smallest integer larger than the real result of the function.

In step b) of the inventive method the value of the modulation factor ($z_i$) is preferably determined based on a well defined power sequence, an exponential sequence, preferably a geometrical sequence; a polynomial sequence or based on a distribution in the set of compartments, preferably predetermined by Gaussian distribution or a combination thereof. More preferably the value of the modulation factor ($z_i$) is determined based on the exponential sequence, such as a geometric sequence (volumes and or dilutions of subsequent compartments represent terms of a geometric series ($v_i d_i \sim e^i$), as thereby a uniform fractional precision regarding the estimated concentration $E(C)$ of particles over the dynamic range $C^+/C^-$ can be achieved. Further preferred embodiments are also disclosed in the following description. More generally, the set of values of the products $v_i d_i$ can be determined by any function that is non-uniform over the range of variation of the index $i$ and may be a function of the dynamic range of the assay, of the precision of the assay and of other important parameters of the assays and its technical realization.

The inventive method is generally suitable to determine the concentration of any particle which optionally after suitable amplification exhibits a suitable measurable signal in the compartment. In a preferred embodiment the inventive method can be used to determine suitable particles preferably selected from the group consisting of a virus, a bacterium, a nucleic acid (NA), preferably a deoxyribonucleic acid (DNA) or a ribonucleic acid (RNA), a peptide, a protein, or another particle, or a nanoparticle or a colloidal particle or a combination thereof.

The inventive method is furthermore generally applicable to all quantitative determination methods of the estimate of a concentration of particles $E(C)$ according to the prior art (e.g. analytic and/or diagnostic quantitation assays), wherein at least part of the particles that are present in any of the ($N$) compartments provide a measurable signal or are amplified to provide a measurable signal by common procedures, and the estimated concentration of particles $E(C)$ is a function of measured signals determined by common statistical models.

Preferably the inventive method is applicable to assays amplifying the presence of an inventively suitable particle to a measurable signal, preferably wherein the particles are amplified by means of polymerase chain reaction (PCR), preferably comprising cyclic temperature variation; loop-mediated isothermal amplification (LAMP); rolling circle amplification (RCA); cascade rolling circle amplification (Cascade RCA); helicase-dependent amplification; nucleic acid sequence based amplification (NASBA); nicking enzyme amplification reaction (NEAR); single-molecule enzyme-linked immunoabsorbent assay (digital ELISA); or any other procedure for amplification of the presence of a particle to a measurable signal, or a combination thereof.

With respect to the inventive determination of the estimate concentration $E(C)$ of particles any common statistical method for determination of estimate concentration $E(C)$ of particles in quantitative assays can be used. Preferred embodiments of the present invention are disclosed further down in the following description.

In accordance with the present invention the partitioning of the predetermined sample volume in step b) into $N$ compartments can be conducted by any suitable common method. In a preferred method, the sample is partitioned according to the determined number $N$ of compartments and according to the determined modulation factor $z_i$ by suitable means of pipetting the sample volume; by suitable means of droplet generation by microfluidic systems, preferably active and/or passive droplet generation; by suitable means of digital microfluidic techniques (also referred to as "electro-wetting on dielectric" EWOD), or by any other suitable method for obtaining the partitioned set of compartments (such as, e.g. disclosed in WO 2012/109600 A2, which disclosure in context with partitioning is incorporated herein in its entirety).

With respect to the pipetting technique a common serial dilution through repetitive mixing, splitting and adding diluents (suitable buffers) generally enables the inventive partitioning of the predetermined sample volume according to the determined number $N$ of compartments and according to the determined modulation factor $z_i$. As an example, the first compartment of the series may comprise a certain volume $v_0$ of the predetermined sample volume with a concentration $C_0$, then the second compartment of the series comprises a fraction $x$ of the predetermined sample volume which is then diluted by adding suitable diluents to reach the volume $v_0$, so that the second compartment comprises $C_1 = C_0 x^1$. Repeating this procedure yields a geometric progression of concentrations $C_i = C_0 x^i$. A suitable apparatus for conducting such a pipetting procedure is for example a hand pipette, or any of the commonly known robotic pipetting stations, or apparatus dedicated to pipetting ultra small volumes, as for example the Mosquito apparatus marketed by TTP Labtech.

In general the aforementioned pipetting method can also be used to prepare a set of compartments presenting a geometric progression of the estimated number of particles: $(d_i v_i)/(d_0 v_0) = z_i = x^i$ by modulating either the concentration, or the volume of each compartment, or both, in any sequence or combination.

With respect to the microfluidic system technique all suitable common techniques can be applied to the present invention, in particular, common active droplet microfluidic systems and passive microfluidic systems, which enable the inventive partitioning of the predetermined sample volume in accordance with the determined number $N$ of compartments and according to the determined modulation factor $z_i$.

With respect to the active droplet microfluidic system technique the present inventors have in particular developed a range of techniques for on-demand execution of operations on microdroplets in microfluidic chips. The operations include i) generation of a small droplet of precisely determined volume, ii) translating the droplet, iii) merging droplets, iv) splitting droplets. These operations allow executing practically any protocol of dilutions and inventive partitioning. The aforementioned active microfluidic technique has been disclosed in particular in the following patent applications PCT/PL2011/050002 "System and method for automated generation and handling of liquid mixtures", filed 21 January 2011 and the subsequent national applications; PL395776 "Sposób dzielenia kropel na żądanie w złączu mikro-przepływowym" filed 2011-07-27; PL395777 "Sposób dzielenia kropel na żądanie w złączu mikroprzepływowym" filed 2011-07-27; PL395778 "Sposób dzielenia kropel na żądanie w złączu mikroprzepływowym" filed 2011-07-27, wherein the content of each application in relation to the active microfluidic techniques is incorporated herein with respect to the present invention in their entirety.

With respect to the passive microfluidic systems (traps) the present inventors (for Scope Fluidics) in particular have developed a system and method for execution of precise operations on droplets in a passive manner. The concept is based on the construction of well geometrically defined 'traps' in the microfluidic channels. The traps, depending on their exact geometry, can i) trap a droplet smaller than a given volume and hold it in place, ii) trap a droplet of precisely set volume and hold it in place, iii) exchange a portion of liquid in the trapped droplet (i.e. accept a small additional volume and release the same volume of the original mixture), iv) trap, wait for arrival of a new droplet, then merge them and release, v) trap, wait for arrival of subsequent drop, then release just the first, while locking the second, etc. These functionalities allow building a lot of different liquid handling protocols, which can be used in accordance with the present invention. The aforementioned passive microfluidic technique (so called "TRAPS" system and method) has been disclosed in particular in the following patent application [PL-398979 "Urządzenie mikroprzepływowe i układ mikroprzepływowy obejmujący jedno lub więcej urządzeń mikroprzepływowych" filed 2012-04-25], wherein the content thereof in relation to the passive microfluidic technique is incorporated herein with respect to the present invention in its entirety.

Furthermore, the well established technological platform for manipulations of droplets on planar substrates called either 'digital microfluidics' or 'electro-wetting on dielectric' (EWOD) enables the inventive partitioning of the predetermined sample volume in accordance with the determined number $N$ of compartments and according to the determined modulation factor $z_i$. The digital microfluidic technique / electro-wetting on dielectric (EWOD) technique allows to generate drops on demand, move them, merge, mix and split them.

The predetermined sample volume is in accordance with the present invention partitioned into $N$ compartments, wherein any common sample holder suitable for holding the separate volumes so that the volumes can be further processed and/or analyzed in accordance with the inventive digital assays can be used.

In a preferred embodiment the predetermined sample volume is partitioned into an inventive sample holder according to the fourth aspect of the present invention. The sample holder of the present invention can comprise e.g. suitable test tubes, arrays of wells on a microarray, or a microfluidic chip, a microfluidic chip configured to generating droplets as well as other commercially available or otherwise generally known devices capable of holding discrete volumes suitable for carrying out the inventive method. In a furthermore preferred embodiment the predetermined sample volume is partitioned using the inventive kit in accordance with the fifth aspect of the present invention. All preferred embodiments of the fourth and fifth aspect of the present invention can independently from each other be combined with the present inventive method.

In a preferred embodiment of the present inventive method the modulation factor ($z_i$) is determinable, preferably is determined by the function $z_i = (v_i d_i)/(v_0 d_0)$ or analogue thereof, wherein the analogue conveys information about pairwise differences in the estimated number of particles in compartments,
wherein ($i$), ($v_i$) and ($d_i$) is defined as set out hereinbefore and
wherein ($v_0$) represents the volume and ($d_0$) represents the dilution factor of the sample in a reference compartment, wherein the reference compartment is different to compartment ($i$), preferably wherein the reference compartment represents the first compartment in the series of ($N$) compartments. The term "first compartment in the series of ($N$) compartments" is defined with respect to the present invention as the compartment comprising part of the predetermined sample volume with the starting volume and starting dilution factor.

In a preferred embodiment of the inventive method in step b) 1%, preferably 5 %, more preferably 25 %, even more preferably 50 %, even more preferably 75% and most preferably 100 % of the ($N$) compartments differ from each other by the value of the modulation factor ($z_i$). The more of the ($N$) compartments differ from each other by the value of the modulation factor ($z_i$), the broader is the range of concentrations of particles in the sample that can be assayed.

However, the reduction to practice of partitioning the predetermined sample volume into the determined ($N$),

wherein the ($N$) compartments comprise or consist of different sample volumes ($v_i$) and/or different dilution factors ($d_i$) of the sample and, thus, differ by the modulation factor ($z_i$) may be for some laboratories too challenging, as the differences caused by the determined modulation factor ($z_i$) may result in only minute changes with respect to volumes and/or dilutions of the different compartments when partitioning the sample. In this case the inventive method provides an alternative preferred embodiment to partition the sample into a number ($N_{LIB}$) of two or more separate sets of compartments (libraries), each library set is indexed with ($j$) and contains $N_j' > 0$ compartments, each compartment in the same library comprising or consisting of a part of the sample volume with the same value of a modulation factor ($z_j$), wherein $z_j$ is a function of volumes ($v_j$) and dilution factors ($d_j$) of the sample in the library set ($j$), and wherein the $N_{LIB}$ separate library sets $j$ are distinguishable from each other by different values of the modulation factor ($z_j$), wherein $j$ represents the index number of the number ($N_{LIB}$) library sets represented by the integers 0 to $N_{LIB}$ - 1. The library concept is further down described in detail with further preferred embodiments.

The inventive method is cumulatively or alternatively preferred, wherein in step b) the sample is partitioned into ($N$) compartments with such volumes ($v_i$) and dilution factors ($d_i$) as to fulfil the condition: $v_i d_i = v_0 d_0 z_i$ wherein $z_i > 0$, and at least for one value of ($i$) the quotient $z_{i+1}/z_i$ is different from 1.

In accordance with a preferred method of the present invention in step b) the modulation factor $z_i$ is based on en exponential series and the sample is partitioned into ($N$) compartments with such volumes($v_i$) and/or dilutions ($d_i$) as to fulfil the condition: $v_i = A \cdot exp^{(B \cdot \frac{i}{N})}$ and/or $d_i = A \cdot exp^{(B \cdot \frac{i}{N})}$, wherein ($A$) and ($B$) represent independently of each other arbitrary real numbers and wherein preferably $A = 1/C^+$ and/or $B = [1.95 \cdot ln\{(C^+/C^-)/N\}]^{0.856}$. In this case it is even more preferred to divide the sample into ($N$) compartments

$$N = 2 \cdot \sigma_{MAX}^{-1.9} \cdot (\frac{C^+}{C^-})^{0.25} \text{, preferably } N = 2 \cdot \sigma_{MAX}^{-1.94} \cdot (\frac{C^+}{C^-})^{0.22}.$$

with ($C^+$), ($C^-$), and ($\sigma_{MAX}$) as hereinbefore with respect to the first aspect of the invention.

The case predetermined sample volume is in accordance step b) of the inventive method is partitioned with a geometric series, the inventive partitioning fulfil the following condition:

$$(v_{i+1} d_{i+1})/(v_i d_i) = z_{i+1}/z_i = x$$

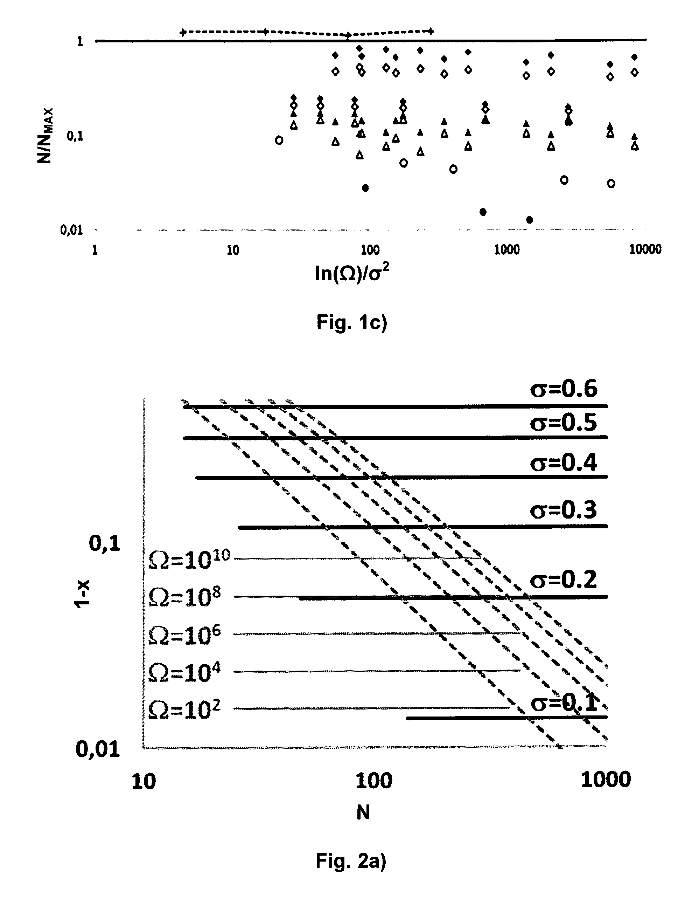

wherein x > 0 and $x \neq 1$, preferably wherein the value of ($x$) is represented by about 0.1, 0.5, 0.8, 0.9, 0.91, 0.92, 0.93, 0.94, 0.95, 0.96, 0.97, 0.98, 0.99, 1.01, 1.02, 1.03, 1.04, 1.05, 1.06, 1.07, 1.08, 1.09, 1.1, 1.2, 1.3, 1.4, 1.5, 2, or 10. In this preferred embodiment the modulation factors of two consecutive compartments ($i$ + 1) and ($i$) are compared and thereby representing a geometric series, which is preferred as already mentioned hereinbefore a uniform fractional precision over the dynamic range can be provided (see Fig. 2a) and respective part in the detailed figure description).

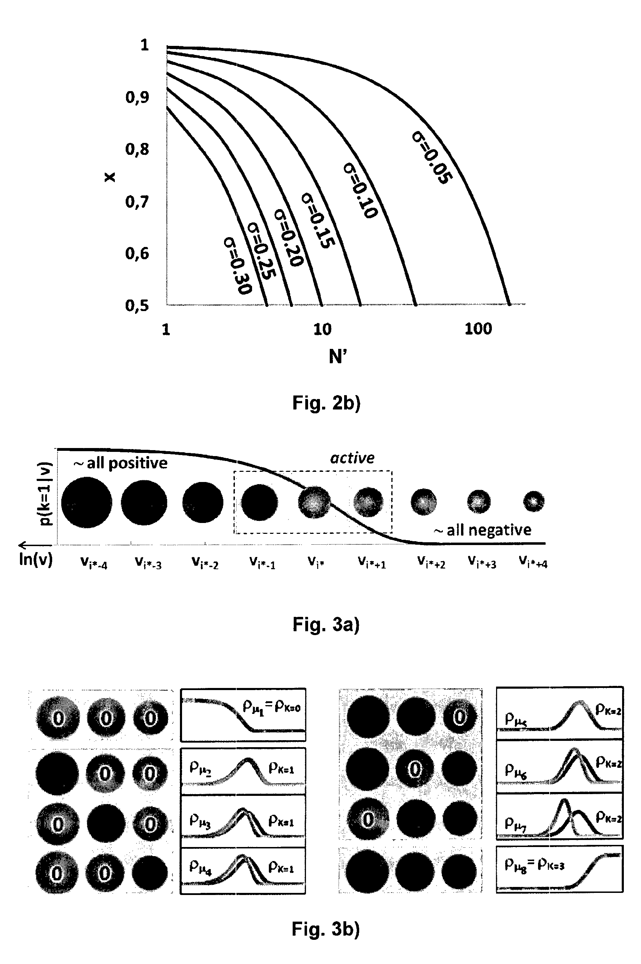

The inventors have surprisingly identified that the inventive method comprising a geometric series with quotient $x = (v_{i+1} d_{i+1})/(v_i d_i)$ can preferably be gauged for the required precision of the estimate of concentration of particles $E(C)$. For any particular value of the input concentration $C^*$ a compartment ($i^*$) can be predetermined that is characterized by the product $v_i^* d_i^* \approx ln(2) /C^*$. One can then consider the potential outcomes (presence or absence of particles) in compartments neighboring with $i^*$ in the geometric series. In particular one can consider the signals from $\Delta N/2$ compartments on either "side": $(v_{i^*+n} d_{i^*+n}) = (v_0 d_0) x^{i^*+n}$, $n \in (-\Delta N/2, \Delta N/2)$. As the probability of obtaining a signal depends on the volume $v_{i^*+n}$ and/or dilution factor $d_{i^*+n}$ of the compartment $i^* + n$: $p_{i^*+n} = 1 - e^{-cv0d0xi^*+n}$, the inventors identified that compartments with much larger or much smaller volumes and/or dilutions than $v_i^* d_i^*$ will not introduce significant information about the estimate of the concentration $E(C) \approx C^*$ of particles (see Fig. 3a of respective part in the detailed figure description). Thus, the inventors found out that the relevant information for determining the concentration $C \approx C^*$ of particles should preferably be derived from a finite set of compartments, that is in accordance with the present invention be called "active stripe" and comprises or consists of $\Delta N$ compartments.

The inventors have unexpectedly found, that in order for the assay to assess any concentration within a required dynamic range $C \in (C^-, C^+)$ it is possible to prepare i) a main set of compartments presenting a geometric series of the factors $v_i d_i$ spanning values from $ln(2) /C^-$ to $ln(2) /C^+$ and ii) supplement this set with margins of $\Delta N/2$ compartments presenting the same geometric progression, and placed on each end of the aforementioned main set.

According to the aforementioned preferred embodiment the inventive method is additionally optimized, as the inventors furthermore found out that the entirety of information that is conveyed by any outcome of the experiment is contained in the particular arrangement - in the context of the present invention called "microstate ($\mu$)" - of the signals, which lead to an assignment of a first value ($k_i$) to the compartments ($i$) that comprise or consists of a predetermined threshold number of particles or more (positive value). In other words, the detailed distribution of signals accounting for the state of each compartment is referred to as a microstate ($\mu$) in the context of the present invention.

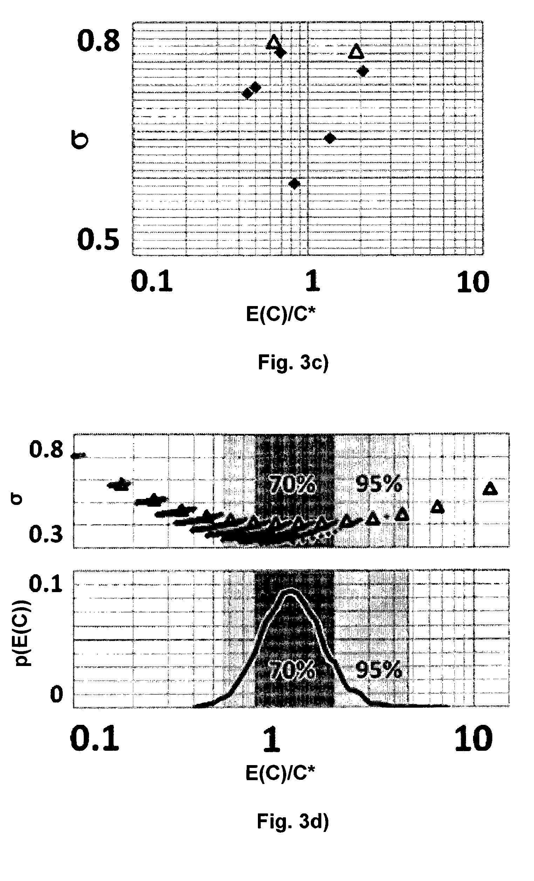

Both the number and the localization of the respective compartments ($i$) assigned with the first value ($k_i$) within the active stripe are stochastic variables. Repetitive experiments with the same initial concentration of the particles will yield different answers. Each such outcome has a different probability of happening from different input concentrations of the particle. Thus, the observation of each particular microstate ($\mu$) carries a different expectation on the estimated concentration $E(C)$ of particles (see Fig. 3a, 3b, 3c, 3d of respective part in the detailed figure description).

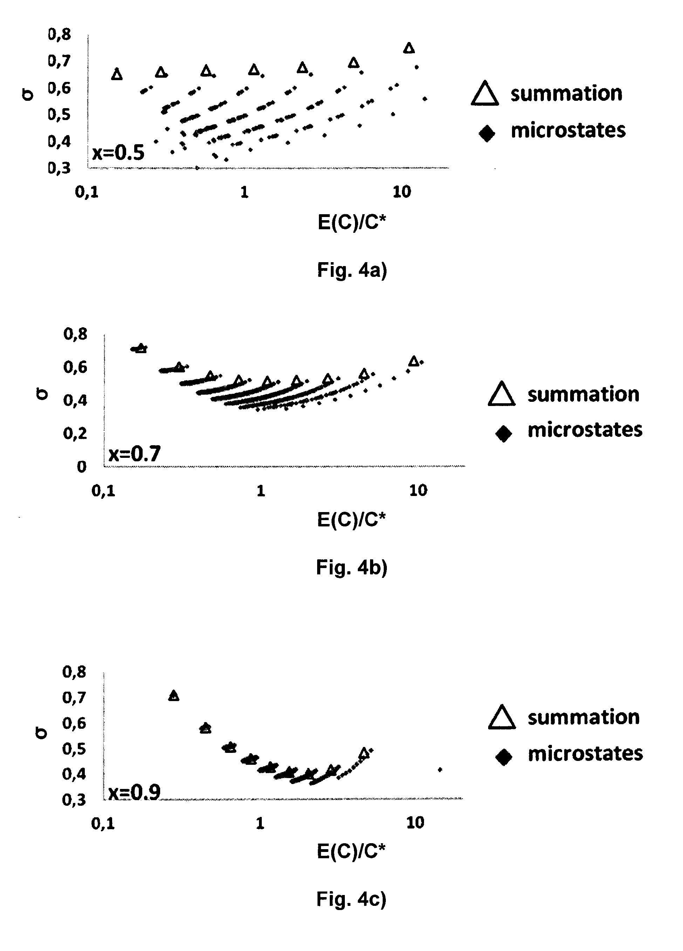

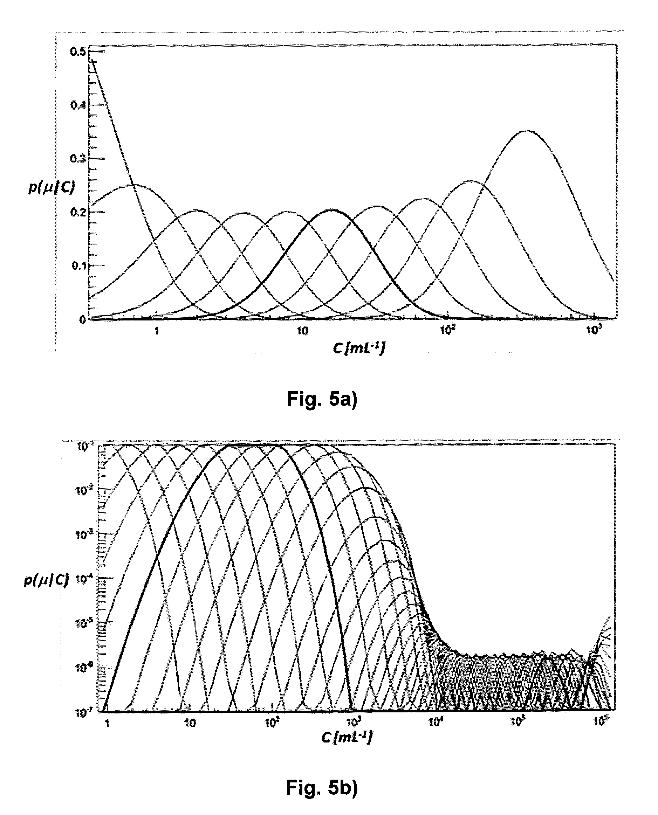

Using geometric series the precision of the estimate concentration $E(C)$ of particles generally depends on the common factor of the geometric series and on the number of compartments. Two important observations can then be made. First, in accordance with the values of the standard deviations most of the outcomes of the estimated concentration $E(C)$ fall very close to the input concentration ($C^*$) (see Fig. 3d of respective part in the detailed figure description). Second the standard deviations offered by the analysis of the microstates are smaller than those obtained from the analysis of the sum $K$ of positive values (see Fig. 4a), 4b) and 4c)) of respective part in the detailed figure description. Further the standard deviations associated with the microstates have a well defined envelope from above. This limit is given by the probability of the standard deviation $\rho(C|\mu)$ calculated for the most probable microstate using the Bayes theory transforming $\rho(\mu|C)$. This microstate has all the compartments for which $v_id_i > v_i^*d_i^*$ yielding the first value ($k_i$) and all the others including $v_{i^*}d_{i^*}$ having assigned a second value ($k_i$), wherein the second value ($k_i$) is assigned, in case the compartment ($k_i$) comprises or consists of less than the threshold number of particles indicating the first value (negative value) (see Fig. 5a) and 5b) and respective part in the detailed figure description).

An inventive method containing a long sequence of compartments will yield in the vast majority of the uses of the assay the precision of the estimate of the concentration either same, or more likely higher, or most likely significantly higher than the precision as the estimate calculated on the basis of the most probable inventive microstate. In addition the inventors surprisingly found out, that, especially (i.e. most likely), for concentrations close to the limits of the dynamic range of the method (i.e. for $C \approx C^-$ or $C \approx C^+$) - the inventive method may return with a small probability an unlikely set of signals/values that yield a low precision of the estimate and, thus, will inform the user (by providing the value of standard deviation higher than requested) that the unfortunate event has happened and that the experiment should be repeated. Although the digital assays of the prior art also allow small probability of unlikely events that provide misinformed estimates of the concentration, only the aforementioned preferred inventive feature using the microstate approach can provide the feedback to the user on such an unfortunate unlikely event occurring.

Increasing the number $\Delta N$ of partitions in the active stripe according to the present invention obviously improves the estimate of $E(C)$ yet only to a certain limit, because the much larger and much smaller compartments than $v_i^*d_i^*$ progressively carry less and less information. Indeed, for any value of $x$ the standard deviation of the estimate concentration falls with increasing $\Delta N$ only to a limit $\sigma_{max}(x) \approx 1.2739(1 - x)^{1.9895}$. The corresponding value of $\Delta N$ that yields compromise between high precision and small number of compartments can preferably be determined by the function $\Delta N(x) = 4.5637(1 - x)^{-0.798}$.

One way of carrying out the aforementioned preferred inventive method is that the inventive method with the geometric series with *quotient* $x = (v_{i+1}d_{i+1})/(v_id_i)$ is determinable, preferably determined by the function:

$$x = 1 - \left(\frac{\sigma_{max}}{0.8955}\right)^{1/0.513},$$

the number (N) of compartments is preferably set to an integer not smaller than the value determinable by the function:

$$N = \Delta N + \log_x\left(\frac{C^-}{C^+}\right),$$

wherein ($\Delta N$) is preferably an integer not smaller than the value determinable, preferably determined by the function:

$$\Delta N = 4.5637(1 - x)^{-0.798}$$

and wherein the value for $d_0v_0$ is determinable, preferably determined by the function

$$d_o v_o = \ln(2) \cdot x^{-\Delta N/2} \left(\frac{1}{C^-}\right)$$

with $(C^+)$, $(C^-)$, $(\sigma_{MAX})$, $(d_0)$ and $(v_0)$ as defined hereinbefore with respect of the first aspect of the invention.

The partitioning of the sample according to the aforementioned preferred inventive method can be designed in the following steps: (i) for given for a given $(C^+)$, $(C^-)$ and $(\sigma_{MAX})$ the values for $N$, $\Delta N$ and $x$ are determined, ii) using $x$ and $\Delta N$ the volume $v_0$ and dilution factor $d_0$ of the first compartment in the sequence is determined and (iii) the sequence of $N$ compartments with $(v_i d_i) = (v_0 d_0) x^i$ wherein $(i)$ represents an index number of the $N$ compartments with integers from 0 to $N - 1$.

The simple equations above of the inventive method create a powerful analytical tool. First, the dynamic range $\left(\frac{C^+}{C^-} = \Omega\right)$ can be tuned completely independently of the precision. Second, for a given required minimum precision and dynamic range, the inventive method requires even $10^2$ to $10^4$ times less compartments than the standard digital assays using equal volumes in the compartments.

For example, with the classic assay, to address dynamic ranges of $\Omega = 10^4$ or $\Omega = 10^7$ one needs, according to the state of knowledge in the field $N \geq 2 \cdot 103$ or $N \geq 2 \cdot 10^6$ test volumes respectively. If a precision of the estimate of $E(\sigma) < 50\%$ is satisfactory, the same $\Omega$s can be assessed by the inventive method comprising only as few as 35 or 47 compartments. If a more precise experiment is needed (i.e. $\sigma < 25\%$ or $\sigma < 10\%$), only 140 and 795, or 192 and 1120 test-volumes respectively are needed with respect to the inventive method. These numbers are orders of magnitude smaller than in the case of the classical assay qualitatively changing the technical requirements to run them.

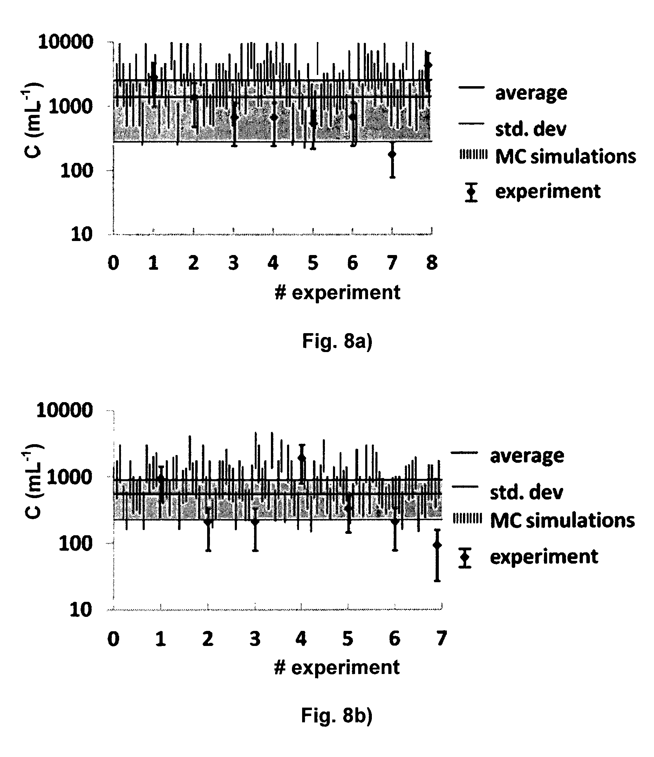

The operation of the algorithms was verified both numerically and experimentally on assays, with varied dynamic ranges and precision: (i) $\Omega = 10^3$, $\sigma \leq 80\%$, $N = 10$, $x = 0.50$, and (ii) $\Omega = 10^2$, $\sigma \leq 60\%$, $N = 10$, $x = 0.60$ (see Examples as well as Figs. 8a) and Fig. 8b) and respective parts in the detailed figure description).

The inventive method furthermore proves to be superior over the multivolume digital assay approach of the prior art [Shen, F.; Sun, B.; Kreutz, J. E.; Davydova, E. K.; Du, W.; Reddy P. L.; Joseph, L. J.; Ismagilov, R. F., "Multiplexed Quantification of Nucleic Acids with Large Dynamic Range Using Multivolume Digital RT-PCR on a Rotational SlipChip Tested with HIV and Hepatitis C Viral Load", J. Am. Chem. Soc., Article ASAP (X 2011)], which already increased the span of the dynamic range in comparison to the standard digital assays using equal volumes. The multivolume method of Ismagilov shows a 3-fold resolution (corresponding to sigma (standard deviation of the estimate) of 30%), the following dynamic range: (a) - about $10^4$ (between $5.2 \cdot 10^2$ and $4.0 \cdot 10^6$) while using 640 compartments (4 families, design 1) and (b) - ca $10^5$ (between $1.7 \cdot 10^2$ and $2.0 \cdot 10^7$) while using 880 compartments (6 families, design 2B).

Comparing the method of Ismagilov with the inventive method, the inventive method obtains the same dynamic range and precision with: (a) - $N = 98$ compartments ($x = 0.881373264$) and (b) - $N = 117$ ccompartments ($x = 0.881373264$)

From a different side, if allowed to use 640 compartments, the inventive method can yield the following combinations of dynamic range and precision: a dynamic range of $2 \cdot 10^3$ with $\sigma < 10\%$; a dynamic range of $5 \cdot 10^7$ with $\sigma < 15\%$; and a dynamic range $> 10^{10}$ with $\sigma < 30\%$. If allowed to use 880 compartments, the inventive method can yield a dynamic range of $3 \cdot 104$ with $\sigma < 10\%$; a dynamic range of $1010$ with $\sigma < 15\%$; and a dynamic range $>> 1010$ with $\sigma < 30\%$. Thus, the above comparison shows that quantitatively the inventive method can i) reduce the number of compartments from 640 to 98, and from 880 to 117, ii) for the same number of compartments can increase the dynamic range by a factor of million or better, and iii) provides the option to freely tune the dynamic range and precision.

In diagnostics the often considered parameter of the assay is its resolution in statistically significant determination that two samples have different concentrations. The resolution of the inventive method is closely approximated by the standard multiplication of the standard deviations, i.e. 70, 90, 95% confidence of distinguishing two samples is realized for samples differing in concentration by a factor of $r\sigma$ with $r = 1,2$ or $3$. Thus, if one knows the required resolution of the assay $R = C_1/C_2$ and the required confidence, then the standard deviation to be inserted into the equations designing the assays is $\sigma = (R - 1)/r$.

One additional benefit of the inventive method is that it can yield the complete information from any single stochastic realization of the experiment. In other words, the preferred scalings that are listed above provide the likely highest standard deviation (i.e. the minimum precision guaranteed by the assay): the declared standard deviation $\sigma$ of the estimate $E(C)$ is the likely highest value $\sigma_{\mu i}$ for all the microstates observed in the assay for $C$'s within a range $C \in (C^-, C^+)$. It might, however, happen that the method returns a lucky answer (microstate) $\mu_i$ that yields a smaller standard deviation $\sigma_{\mu i}$ of the estimation $E_{\mu i}(C)$ and therefore yielding higher precision, wherein here $i$ indexes the microstates $i = 0$ to $2^N - 1$. With the increase of $x$ the distribution of $\sigma_{\mu i}$ becomes narrower and closer to the value of maximum standard deviation. Interestingly, the expected value of the standard deviation $\sigma_{expected}(x)$ can be preferably represented as a polynomial function of $x$. This could be used as a basis for designing the assay in place of the maximum standard deviation $\sigma_{max}(x)$.

Using the inventive concept it is further preferred to construct assays that provide different required precision in different ranges of concentration. This could be useful in diagnostics, where e.g. at low and high concentrations precision can be sacrificed for the dynamic range, while in the intermediate range of concentration the exact estimate is clinically informative.

Accordingly another preferred inventive method relates to dividing the interval $(C^-, C^+)$ into $q$ subintervals $(C_n^-, C_n^+)$, preferably disjoint ones, in each subinterval the set of values $d_i v_i$ is a geometric series with quotient $x_n$, whereas:

1) For $\sigma_{n_{max}} < \sigma_{\text{n-1}_{max}}$

$$x_n = 1 - \left(\frac{\sigma_{n_{max}}}{0.8955}\right)^{1/0.513}$$

$$\Delta N_n = 4.5637(1 - x_n)^{-0.798}$$

$$N_n = \Delta N_n + \log_{x_n}\left(\frac{C_n^-}{C_n^+}\right)$$

$$d_{n_0} v_{n_0} = ln2 \cdot x_n^{-\Delta N_n/2}(1/C_n^-)$$

2) For $\sigma_{n_{max}} > \sigma_{\text{n-1}_{max}}$

$$x_n = 1 - \left(\frac{\sigma_{n_{max}}}{0.8955}\right)^{1/0.513}$$

$$\Delta N_n = 4.5637(1 - x_n)^{-0.798}$$

$$N_n = 0.5\Delta N_n + 0.5\Delta N_{n-1} + \log_{x_n}\left(\frac{C_n^-}{C_n^+}\right)$$

$$d_{n_0} v_{n_0} = ln2 \cdot x_{n-1}^{-\Delta N_{n-1}/2}(1/C_n^-)$$

2. where: $C^-$ means the lower limit of the interval for the determination of the unknown concentration $C$, $C^+$ means the upper limit of the interval for the determination of the unknown concentration $C$, $C_n^-$ means the lower limit of the subinterval with the number $n$, $C_n^+$ means the upper limit of the subinterval with the number n, $\sigma_{n_{max}}$ means the maximum permitted standard deviation of the estimate $E(C)$ of the unknown concentration $C$ of particles in the sample in the subinterval $(C_n^- C_n^+)$, $n$ is the subinterval number, running over integers from $1$ to q, and $i$ is the number of compartment in n-th interval, running over integers from $0$ to $N_n$ - 1.

According to a further cumulatively or alternatively preferred inventive method, the inventive method comprises or consists of the following steps:

c) optionally amplifying the particles of the compartments with one or more suitable reagents and optionally one or more diluents for obtaining a measurable signal indicating the presence of a predetermined threshold number of particles, preferably one, two, three or more particles in a compartment,

d) measuring the signals in each of the ($N$) compartments and assigning to at least part, preferably all compartments a value ($k_i$), wherein ($i$) represents the index number of the compartments represented by integers 0 to $N$ - 1 and compartment (*i*) is assigned a first value ($k_i$), if the compartment (*i*) comprises or consists of the predetermined threshold number of particles or more, and compartment (*i*) is assigned a second value ($k_i$), if the compartment ($k_i$) comprises or consists of less than the threshold number of particles indicating the first value and

e) determining the estimated concentration of particles $E(C)$, wherein the estimated concentration of particles $E(C)$ is determinable by a function of

> i. a *sum K* of first and/or second values ($k_i$), wherein the function preferably is corrected using a calibration correction function, and/or

> ii. a *sum K* of first and/or second weighted values ($w_i k_i$), wherein at least part of the values ($k_i$), preferably each value ($k_i$) is modulated with a weight value ($w_i$), wherein (*i*) represents the index number of the compartments represented by the integers 0 to *N* - 1, and wherein the weight value ($w_i$) preferably is inversely proportional to the degree of volume and/or dilution of the number (*i*) of the compartments, and wherein the function is preferably corrected using a calibration correction function, and/or

> iii. a number, vector, matrix, tensor of any order or any other unambiguous representation of a *microstate* $\mu$, wherein the number, vector, matrix, tensor of any order or any other representation comprises or consists of information reflecting at least part of the values ($k_i$) measured in step e), preferably comprises or consists of a vector $k \equiv \{k_i\}$, representing the *microstate* $\mu$, wherein the vector ($k$) comprises or consists of at least part, preferably all of the values ($k_i$) measured in step e).

In case the particles to be measured do not provide as such a measurable signal, it is according to inventive method step c) preferred to amplify the particles so that at least part of the particles, preferably all of the particles provide a measurable signal. A suitable measurable signal is any signal which can be detected visually and/or by suitable means. In a preferred method the particles are labeled with a suitable component, e.g. a reporter molecule, which bind, form complexes or interact with the particles in another way so that they excite light of specific wavelength and/or electricity upon suitable excitation, preferably they exhibits fluorescence (up and downconverting), exhibit light in the infrared (IR) range (near and/or far infrared wavelength, NIR/FIR), or exhibit electricity. Reporter molecules in particular suitable for use in any of the hereinafter mentioned amplification methods are commonly known. In a preferred method two reporter molecules can be used, which compete for a single binding site of the particle to be determined and, thus, furthermore extend the dynamic range of the inventive method. An example of suitable reporter molecules is given in WO 2012/049316 A1, wherein the different reporter molecules (markers) are used to interact (directly or indirectly) with the particles of the sample, wherein the reporter molecules are preferably nucleic acid molecules, e.g. oligonucleotides. Such reporter molecules are preferably used in proximity probe-based detection assays and the disclosure of WO 2012/049316 A1 concerning the suitable reporter molecules (markers) is incorporated herein in its entirety.

The inventive method is according to step c) applicable to any amplification method for quantitatively determining the presence of the particles. Preferably the inventive method is applicable to methods amplifying the presence of a particle to a measurable signal, preferably wherein the particles are amplified by means of polymerase chain reaction (PCR), preferably by cyclic temperature variation, loop-mediated isothermal amplification (LAMP), rolling circle amplification (RCA), cascade rolling circle amplification (Cascade RCA), helicase-dependent amplification, nucleic acid sequence based amplification (NASBA), nicking enzyme amplification reaction (NEAR), single-molecule enzyme-linked immunoabsorbent assay (digital ELISA), or any other procedure for amplification of the presence of a particle to a measurable signal, or a combination thereof. The aforementioned amplification methods are known in the prior art and a person skilled in the art will readily know which suitable one or more reagents and optionally one or more diluents can be used.

With respect to the inventive method step d) the mixture in every compartment containing the particles and optionally one or more reagents and optionally one or more diluents is generally suitably excited so that a compartment comprising at least a particle exhibits a signal indicating the presence of the particle, e.g. by fluorescence and/or phosphorescence and/or electricity intensity. The value of the signal in at least a part, preferably all compartments is measured with suitable means. Subsequently the value of signal is respectively transformed by suitable means, such as detectors suitable for measuring fluorescence, phosphorescence intensity and transforming the signal into the first or second value ($k_i$) respectively assigned at least part, preferably all of the compartments (*i*). Preferably, the threshold number of particles is 1, 2, 3, 4, 5, 6, 7 or more particles, more preferably 1 particle in a compartment. The first and second valued ($k_i$) can be any suitable values, preferably values wherein the first value ($k_i$) represents a positive value indicating the presence of the threshold number of particles in the compartment (*i*) and the second value ($k_i$) represents a negative value indicating that the threshold number of particles in the compartment is not present in the compartment (*i*). Preferably the first and second values ($k_i$) comprise binary values, wherein the first value ($k_i$) is preferably represented by "1" or any other integer, real number or sign and the second value ($k_i$) is preferably represented by "0" or any other integer, real number or sign different from the integer representing the first value ($k_i$).

According to step e) of the inventive method preferred methods of determining the estimated concentration of particles $E(C)$ are disclosed.

According to a first alterative i) of the inventive method the *sum K* of first and/or second values ($k_i$), preferably the set of $\{k_i\}$, $N$ and $\{v_i d_i\}$ or $P(v_i d_i)$ values, wherein $P(v_i d_i)$ is preferably a function of the volume and/or dilution, is transformed by known statistical algorithms, preferably by use of the Bayes theorem into the estimate of concentration.

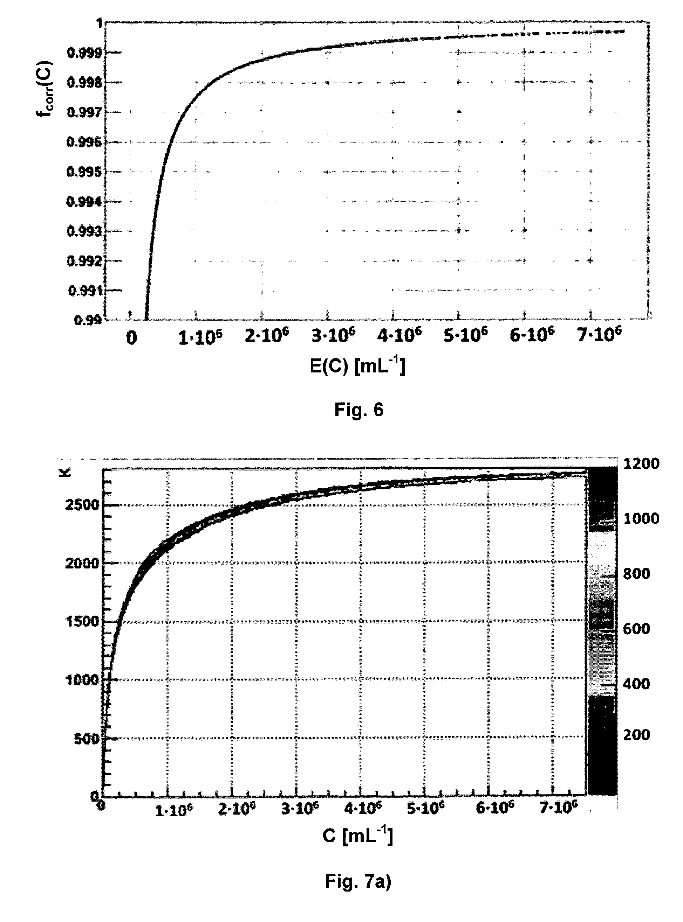

Preferably the aforementioned function is corrected using a calibration correction function. The suitable calibration correction function may be determined experimentally, using standard samples with concentrations of target particles within the dynamic range of the test. The calibration correction can also be computed numerically based on the dependence of the estimate $E(C)$ of the concentration of particles in the sample and the actual number of particles M randomly distributed in compartments divided by the volume of the sample: $C = M/V_{sample}$: $f_{corr}(C)= C/E(C)$. Having computed the calibration correction $f_{corr}(C)$, the corrected estimate of the number of particles is determinable, preferably determined with the formula $E_{corr}(C) = E(C) \cdot f_{corr}(C)$. For instance, for a distribution of 474 partitions with volumes given by the formula $v_i = e^{0.0115 \cdot i}$ for index i running over the values from 0 to 473, the correction is $f_{corr}(C) = (-1.48 \cdot 10^{-5} \cdot C + 1.09)^{-1}$ (see Fig. 6 and respective part in the detailed figure description).

To overcome the aforementioned drawbacks in the analysis of multi-volume assays the inventors have surprisingly identified to inventively determine the estimate concentration $E(C)$ of particles by use of a simple *sum K* of first and/or second weighted values ($w_i k_i$) applying common statistical methods, wherein at least part of the values ($k_i$), preferably each value ($k_i$) is modulated with a weight value ($w_i$), wherein the weight value ($w_i$) preferably a function on dilution factors and/or volumes of compartments ($w_i = f(d_i, v_i)$) and more preferably is inversely proportional to the degree of dilution factors and/or volumes of the number ($i$) of the compartments (see Fig. 7a), 7b) and 7c) of respective part in the detailed figure description). Preferably the determination of concentration $C$ of particles is carried out using the set of preferably the set of $\{w_i k_i\}$, $N$ and $\{v_i d_i\}$ or $P(v_i d_i)$ values, wherein $P(v_i d_i)$ is preferably a non-increasing function of the volume and/or dilution, is transformed by known statistical algorithms, preferably by use of the Bayes theorem into the estimate of concentration. In case, the weight value ($w_i$) is dependent on the compartment volume and dilution a distribution of weights $w(d_i v_i)$ is employed, said distribution being selected from the group comprising:

a. $w(d_i v_i) \equiv 1$,

b. $w(d_i v_i)$ $(d_i v_i)^{\beta}$, where the exponent $\beta$ represents an arbitrary real number,

c. $w(d_i v_i) = \exp(\beta d_i v_i)$, where the multiplier $\beta$ is an arbitrary real number.

Preferably the aforementioned method according to alternative ii) is corrected using a calibration correction function as described hereinbefore with alternative i) (see Fig. 6 and respective part in the detailed figure description).

The aforementioned methods of determining the estimate of concentration $E(C)$ of particles of alternative i) and ii) relate to determining the estimate of concentration $E(C_{samples})$ of particles in the predetermined sample volume, as stochastic distribution of particles between compartments is a distribution of dependant variables. Hereinbefore it is described how to transform the functions to result at the estimate of concentration $E(C_{reservoir})$ of particles in the reservoir or the estimate of concentration $E(C_{specimen})$ of particles in the specimen.

The inventive summation procedures according to methods i) and ii) result, however, in a loss of some information, as the sum $K$ of values, preferably binary values or the weighted sum $K$ of values, preferably binary values does not contain unambiguous information on which compartments contained at least one target particle, and which did not.

In case of using the preferred inventive concept of the number, vector, matrix, tensor of any order or any other unambiguous representation of the *microstate* $\mu$ is preferably used to determine the estimate concentration $E(C)$ of particles, wherein the number, vector, matrix, tensor of any order or any other representation comprises or consists of information reflecting at least part of the values ($k_i$) measured in step e), preferably comprises or consists of a vector $k \equiv \{k_i\}$, representing the *microstate* $\mu$, wherein the vector ($k$) comprises or consists of at least part, preferably all of the values ($k_i$) measured in step e). The preferred embodiments of the first and/or second values of ($k_i$) as described hereinbefore with respect to step e) of the inventive method also apply with respect to the aforementioned preferred method. The surprising benefit of using the microstate $\mu$ approach is that the computational complexity of the procedure for computation of the estimate concentration $E(C)$ of particles is reduced and the information on the probable concentration that caused the observed compartments contain at least a threshold number of particles is increased. The microstate $\mu$ approach provides preferably (i.e. most likely), for concentrations close to the limits of the dynamic range of the method (i.e. for $C \approx C^-$ or $C \approx C^+$) a feedback, and the inventive method will inform the user (by providing the value of standard deviation higher than requested) that the unfortunate event has happened and that the method should be repeated. As mentioned hereinbefore the inventive method may be used either to estimate the concentration of particles in the sample, or in a reservoir from which the specimen and sample were prepared.

Preferably, in step e) the calculations of the microstate $\mu$ are performed with the use of a neural network mapping the vector *k* into the estimate *E(C)*.

According to a preferred embodiment of the microstate $\mu$ approach, in step e) of the inventive method the estimate concentration *E(C)* of particles is a function of the conditional probability $p(\mu|C)$ transformed by Bayes theorem into $p(C|\mu)$, preferably determined by the following operations are performed:

e1) calculation of conditional probability $p(\mu|C)$ from the formula:

$$p(\mu|C) = \prod\nolimits_{i=0}^{N-1} \left[ \left(1 - e^{-Cv_0 d_0 z_i}\right)^{k_i} \left(e^{-Cv_0 d_0 z_i}\right)^{(1-k_i)} \right]$$

e2) calculation of conditional probability $p(C|\mu)$ from the formula:

$$p(C|\mu) = p(\mu|C) \cdot \left[ \int_{C_{min}}^{C_{max}} dC \cdot p(\mu|C) \right]^{-1}$$

where:

$C_{min} = -C^-\ln(p_{TR})/\ln(2)$, $C_{max} = -C^+\ln(1 - p_{TR})/\ln(2)$, and: $C^-$ means the lower limit of the interval for the determination of the unknown concentration *C*, $C^+$ means the upper limit of the interval for the determination of the unknown concentration *C*, $0 < p_{TR} < 1$ is a parameter specifying the assay precision, preferably $C_{min} = 0$ and $C_{max} = \infty$;

in the case the series of values $v_i d_i$ is a geometric series with quotient $x < 1$, $C_{min}$ and $C_{max}$ are equal to: $C_{min} = C^- x^{\Delta N/2}$, $C_{max} = C^+ x^{-\Delta N/2}$, where $\Delta N = 4.5637(1 - x)^{-0.798}$.

e3) determination of the concentration C as the estimate *E(C)*, where:

$$E(C) = \int_{C_{min}}^{C_{max}} dC \cdot C \cdot p(C|\mu)$$

with standard deviation $\sigma(C) = \sqrt{E(C^2) - E^2(C)} \cdot [E(C)]^{-1}$, where:

$$E(C^2) = \int_{C_{min}}^{C_{max}} dC \cdot C^2 \cdot p(C|\mu)$$

As already described hereinbefore the inventive method also provides preferred solutions in case the determined number *N* of compartments and the determined modulation factor $z_i$ leads to a partitioning of the predetermined sample volume, which cannot be handled in a respective laboratory in practice, e.g. as the suitable means of partitioning the sample are not present there. In this case the inventive method recommends to use the library approach, wherein in step b) the sample is partitioned into a number ($N_{LIB}$) of two or more separate sets of compartments (libraries), each library set is indexed with (*j*) and contains $N_j' > 0$ compartments, each compartment in the same library comprising or consisting of a part of the sample volume with the same value of a modulation factor ($z_j$), wherein $z_j$ is a function of volumes ($v_j$) and dilution factors ($d_j$) of the sample in the library set (*j*), and wherein the $N_{LIB}$ separate library sets *j* are distinguishable from each other by different values of the modulation factor ($z_j$), wherein *j* represents the index number of the number ($N_{LIB}$) library sets represented by the integers 0 to $N_{LIB}$ - 1.

With respect to the library approach the modulation factor ($z_j$) is preferably determined by the function *of* $z_j = (v_j d_j)/(v_0 d_0)$ or an analogue thereof, wherein ($v_j$) represents the volume and ($d_j$) represents the dilution factor of the sample in the compartments of library set (*j*) and wherein ($v_0$) represents the volume and ($d_0$) represents the dilution

factor of the sample in the compartments of a selected reference library set ($j$).

According to another preferred embodiment of the library approach in step b) of the inventive method at least part, preferably all of the number ($N_{LIB}$) library sets ($j$) comprise or consist of $N'_j = N'$ compartments for each library set ($j$), and fulfil the condition:

$$z_{j+1}/z_j = x$$

wherein $x > 0$ and $x \neq 1$. By using the geometric series with quotient $x = (v_{j+1}d_{j+1})/(v_jd_j)$ the precision of the estimate of the concentration $E(C)$ is optimized, preferably the precision is only worst by the factor 20% in comparison to the microstate $\mu$ approach described hereinbefore.

According to a further cumulatively or alternatively preferred embodiment of the inventive method the set of values $v_jd_j = v_0d_0z_j$ represents a geometric series with quotient $x = (v_{j+1}d_{j+1})/(v_jd_j)$, wherein the function:

$$x_{max} = 1 - \left(\frac{\sigma_{max}}{0.8955}\right)^{1/0.513}$$

represents the maximum preferred value of the quotient ($x$), wherein the quotient ($x$) can be tuned to the technical preferences for execution of the method, and the number $N'_j$ of compartments in each library set ($j$) is set to an integer not smaller than the value determinable by the function:

$$N'_j = \left(\frac{0.8955}{\sigma_{max}}\right)^2 (1 - x)^{1.026}$$

and the number ($N_{LIB}$) of the separate library sets ($j$) is preferably set to an integer not smaller than the value determinable by the function

$$N_{LIB} = log_x\left(\frac{C^-}{C^+}\right) + \Delta N_{LIB}$$

wherein $\Delta N_{LIB}$ is determinable, preferably determined by the function

$$\Delta N_{LIB} = 4.5637(1 - x)^{-0.798}$$

and wherein $v_0d_0$ is determinable, preferably determined by the function

$$v_0d_0 = ln2 \cdot x^{-\Delta N_{LIB}/2} \cdot \frac{1}{C^-}.$$

with ($C^+$), ($C^-$) and ($\sigma_{MAX}$), as hereinbefore with respect to the first aspect of the invention and ($d_0$) and ($v_0$) as defined hereinbefore with respect to the inventive library approach.

With respect to the present invention the number $N_{LIB}$ of compartments and $\Delta N_{LIB}$ represent an integer, wherein the integer is determined, which is close, preferably closest to the real result of the underlying function.

According to a furthermore preferred inventive method at least part or preferably each of the number ($N_{LIB}$) library sets ($j$) is assigned a value ($k_i^j$) being a function of at least part of, preferably all the values ($k_i$) assigned to each of the compartments contained in library set ($j$) in step d), and subsequently mapping the values ($k_i^j$) into a number, vector, matrix, tensor of any order or any other unambiguous representation of a *microstate* $\mu$, wherein the number, vector, matrix, tensor of any order or any other representation comprises or consists of information reflecting at least part of the values ($k_i^j$), preferably comprises or consists of a vector $k_{LIB} \equiv \{k_i^j\}$, representing the *microstate*

$\mu$, wherein the vector ($k_{LIB}$) comprises or consists of at least part, preferably all of the values ($k_i^j$), wherein preferably the value ($k_i^j$) comprises or consists of the *sum* $K_j$ of the values ($k_i$) assigned to each of the compartments contained in library set (*j*) in step d).

3. According to a preferred embodiment of the present invention the determination of estimate of concentration $E(C)$ of particles is characterized in that the following operations are performed:

e1) calculation of conditional probability $p(\mu|C)$ from the formula:

$$p(\mu|C) = \prod_{j=0}^{N_{LIB}-1} \left\{ \binom{N'_j}{K_j} \left(1 - e^{-z_j v_0 d_0 C}\right)^{K_j} \left(e^{-z_j v_0 d_0 C}\right)^{N'_j - K_j} \right\}$$

e2) calculation of conditional probability $p(C|\mu)$ from the formula:

$$p(C|\mu) = p(\mu|C) \cdot \left[ \int_{C_{min}}^{C_{max}} dC \cdot p(\mu|C) \right]^{-1}$$

where: $C_{min} = -C^- \ln(p_{TR})/\ln(2)$, $C_{max} = -C^+ \ln(1 - p_{TR})/\ln(2)$, and: $C^-$ means the lower limit of the interval for the determination of the unknown concentration $C$, $C^+$ means the upper limit of the interval for the determination of the unknown concentration $C$, $0 < p_{TR} < 1$ is a parameter specifying the assay precision, preferably $p_{TR} \to 1$; in the case the series of values $v_j d_j$ is a geometric series with quotient $x < 1$, $C_{min}$ and $C_{max}$ are preferably equal to: $C_{min} = C \cdot x^{\Delta N/2}$, $C_{max} = C^+ x^{-\Delta N/2}$, where $\Delta N = 4.5637(1 - x)$ -0.798

e3) determination of the concentration $C$ as the estimate $E(C)$, where:

$$E(C) = \int_{C_{min}}^{C_{max}} dC \cdot C \cdot p(C|\mu)$$

with standard deviation $\sigma(C) = \sqrt{E(C^2) - E^2(C)} \cdot [E(C)]^{-1}$, where:

$$E(C^2) = \int_{C_{min}}^{C_{max}} dC \cdot C^2 \cdot p(C|\mu)$$

According to a further preferred embodiment of the inventive method, the number of copies of compartments in each library is equal $N'_j = N'$, providing a convenient method for adjusting the technically preferable value $x$ of the common ratio of the geometric progression and $N'$ while warranting the requested dynamic range and precision of the assay (see Fig. 2b and the respective part of the detailed figure description).

According to a further preferred embodiment of the inventive method, the signal is preferably measured in each compartment of the library with number $i$ using the Real-Time PCR method and that compartment is assigned a real value that is dependent on the initial number of copies of target particle in that compartment, and subsequently the library with number $i$ is assigned a value $k_i$ specifying how many times more copies of the target particle are present in all compartments belonging to that library, than in certain arbitrarily selected positive compartment $v_{smin} d_{smin}$, and the measured signal is mapped in the form of a vector $k \equiv \{k_i\}$ specifying a *microstate* $\mu$, containing consecutively the values of the parameter $k_i$, where $i$ is the library number, running over integers from 0 to $N$ - 1.

4. According to a furthermore preferred inventive method according to the first aspect of the present invention, the method is characterised in that in step d) the total signal in all compartments from all libraries is measured, while each compartment $i$ in the $j$-th library used in the assay is assigned a parameter $k_i^j$ being a function of the signal measured in that compartment, and then the measured signal is mapped into a form of a value $K_j = \sum_i w_i^j k_i^j$

specifying a *microstate* $\mu$, containing consecutively the values of the parameter $K_j$, where $j$ is the library number, running over integers from $0$ to the number $N_{lib}$ - 1, and $w_i^j$ is a summation weight, and preferably $w_i^j = 1$ for each $i$ and each $j$. In case of this preferred inventive method, the following operations are preferably performed in step e):

e1) calculation of conditional probability $p(K|C)$ from the formula:

$$p(K|C) = \sum_{\{k_i\}} \left[ \prod_{i=0}^{N_{LIB}-1} \left\{ \binom{N'_i}{K_i} \left( 1 - e^{-z_i v_0 d_0 C} \right)^{K_i} \left( e^{-z_i v_0 d_0 C} \right)^{N'_i - K_i} \right\} \right]$$

e2) calculation of conditional probability $p(C|K)$ from the formula:

$$p(C|K) = p(K|C) \cdot \left[ \int_{C_{min}}^{C_{max}} dC \cdot p(K|C) \right]^{-1}$$

where: $C_{min}$ = -$C^-$ln($p_{TR}$)/ln(2), $C_{max}$ = -$C^+$ ln(1 - $p_{TR}$)/ln(2), and: $C^-$ means the lower limit of the interval for the determination of the unknown concentration $C$, $C^+$ means the upper limit of the interval for the determination of the unknown concentration $C$, 0 < $p_{TR}$ < 1 is a parameter specifying the assay precision; , preferably $p_{TR} \to 1$; in case the series of values $v_i d_i$ is a geometric series with quotient $x$ < 1, $C_{min}$ and $C_{max}$ are equal to: $C_{min}$ = $C^-$ $x^{\Delta N/2}$, $C_{max}$ = $C^+ x^{-\Delta N/2}$, where $\Delta N$ = 4.5637(1 - $x$)$^{-0.798}$.

e3) determination of the concentration C as the estimate $E(C)$, where:

$$E(C) = \int_{C_{min}}^{C_{max}} dC \cdot C \cdot p(C|K)$$

with standard deviation $\sigma(C) = \sqrt{E(C^2) - E^2(C)} \cdot [E(C)]^{-1}$, where:

$$E(C^2) = \int_{C_{min}}^{C_{max}} dC \cdot C^2 \cdot p(C|K)$$

Furthermore, in case the inventive method allows different standard deviations for different dynamic ranges, the following preferred inventive method is carried out, wherein the method is characterised in that the interval ($C^-, C^+$) is divided into $q$ subintervals $(C_n^-, C_n^+)$, preferably disjoint ones, in each subinterval the set of values $d_i v_i$ is a geometric series with quotient $x_n$, and the libraries have constant cardinalities $N'_n$, whereas:

a) For $\sigma_{n_{max}} < \sigma_{n-1_{max}}$

$$x_n(N'_n) = 1 - \left( \frac{\sigma_{n_{max}}}{0.8955} \sqrt{N'_n} \right)^{1/0.513}$$

$$N'_n(x_n) = \left[ \frac{0.8955}{\text{ó}_{n_{max}}} (1 - x_n)^{0.513} \right]^2$$

$$\Delta N_{LIB_n} = 4.5637(1 - x_n)^{-0.798}$$

$$N_{LIB_n} = \Delta N_{LIB_n} + \log_{x_n}\left(\frac{C_n^-}{C_n^+}\right)$$

$$d_{n_0} v_{n_0} = ln2 \cdot x_n^{-\Delta N_{LIBn}/2}(1/C_n^-)$$

b) For $\sigma_{n_{max}} > \sigma_{n-1_{max}}$

$$x_n(N'_n) = 1 - \left(\frac{\sigma_{n_{max}}}{0.8955}\sqrt{N'_n}\right)^{1/0.513}$$

$$N'_n(x_n) = \left[\frac{0.8955}{\sigma_{n_{max}}}(1 - x_n)^{0.513}\right]^2$$

$$\Delta N_{LIB_n} = 4.5637(1 - x_n)^{-0.798}$$

$$N_{LIB_n} = 0.5\Delta N_{LIB_n} + 0.5\Delta N_{LIB_{n-1}} + \log_{x_n}\left(\frac{C_n^-}{C_n^+}\right)$$

$$d_{n_0} v_{n_0} = ln2 \cdot x_{n-1}^{-\Delta N_{LIB_{n-1}}/2}\left(\frac{1}{C_n^-}\right)$$

Where $C^-$ means the lower limit of the interval for the determination of the unknown concentration $C$, $C^+$ means the upper limit of the interval for the determination of the unknown concentration $C$, $C_n^-$ means the lower limit of the subinterval with the number $n$, $C_n^+$ means the upper limit of the subinterval with the number $n$, $\sigma_{n_{max}}$ means the maximum permitted standard deviation of the estimate $E(C)$ of the concentration $C$ of particles in the subinterval $(C_n^-, C_n^+)$, $n$ is the subinterval number, running over integers from $1$ to $q$, and $i$ is the library number, running over integers from $0$ to $N_{LIB_n}$ - 1.

Preferably, the aforementioned calculations are performed with the use of a neural network.

According to another cumulatively or alternatively preferred embodiment of the present invention the inventive method can be used with respect to a multiplex PCR method. Accordingly, the inventive method is preferably used wherein the concentration of two or more different particles and/or two or more different parts of essentially the same particles are determined, wherein at least part, preferably all of the different particles and/or different parts of essentially the same particles that are present in any of the *(N)* compartments provide, preferably are amplified to provide two or more distinguishable measurable signals, wherein the two or more distinguishable signals in any of the (*N*) compartments are measured and wherein to each of the (*N*) compartments two or more different values are respectively assigned, wherein the different values independently from each other indicate that a threshold of a number of same particles and/or same parts of essentially the same particles is present in the compartment or not.

Another preferred embodiment of the present inventive method according to the first aspect of the present invention can be described as follows:

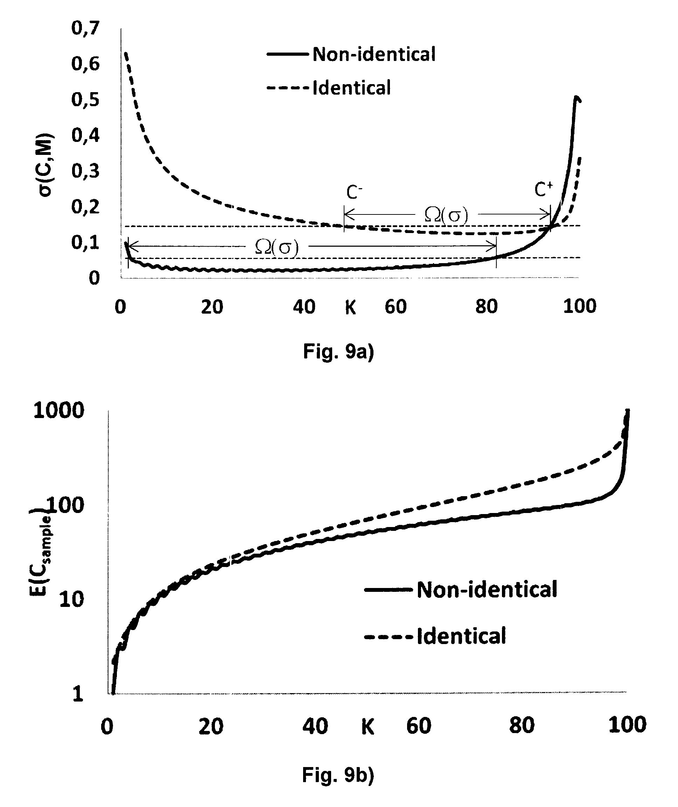

In potential applications of the digital quantitation assays the user is preferably interested in obtaining the estimate $E(M)$ of number of particles in the sample. If the sample is divided into a set of $N$ identical compartments, the appropriate algorithm for analysis of the outcome of the assay is to treat the stochastic distribution of particles between compartments as a distribution of dependant variables. The standard deviation of the estimate given by such analysis depends on the concentration $E(C_{sample}) = E(M)/V_{sample}$ of particles in the sample and the estimate itself depends on the signal from the assay (see Figs. 9a) and 9b) as well as the detailed figure description). In applications of the inventive method it is preferred that the relative standard deviation $\sigma$ of the estimate is not larger than a known threshold within a given range of concentrations $C_{sample} = M/V_{sample}$. The relation between the dynamic range of concentrations $\Omega = C^+/C^-$ and the maximum value of the relative standard deviation $\sigma$ of the estimates within the range $C_{sample} \in (C^-, C^+)$, $\Omega(\sigma)$ has a characteristic deflection point $\frac{d^2\Omega}{d\sigma^2} = 0$ that defines the optimum combination of the dynamic range $\Omega(N)$ and precision $\sigma(N)$ as a function of the number of compartments $N$. A single assay comprising $N$ compartments has the disadvantage that it does not allow to tune its dynamic range and precision independently of each other.

The inventors have unexpectedly found a solution to this problem that allows minimization of the total number of compartments for the required and independently predetermined dynamic range and precision of the estimate of concentration of particles $E(C)$. The solution to this technical problem is to use a combination of assays, each comprising a number of identical compartments, with the volume and/or dilution factor of the compartments different between the assays. According to the invention, given the required values of the dynamic range ($\Omega_{tot}$) of the combined assay and the maximum relative standard deviation ($\sigma$) of the estimate of the number of particles $E(M)$ that can be expressed by the estimate of concentration $E(C_{sample})$ returned by the assay, the parameters of the assay can be calculated with the use of the following simple scheme. The number $N_{lib}$ of sets of identical compartments is given by:

$$N_{lib} = \frac{\log{(\Omega_{tot})}}{\log(\alpha \cdot \sigma^\beta)}.$$

The number ($N'$) of compartments in each set is preferably given by:

$$N' = \gamma \cdot \sigma^\delta,$$

and the common factor $x = d_{i+1}v_{i+1}/d_i v_i$ of the geometric series of the modulation factor $z_i = d_i v_i/d_0 v_0 = x^i$, where $i \in (0, N_{lib} - 1)$, characterizing compartments in different assays is preferably given by:

$$x = \Omega_{tot}^{1/N_{lib}}.$$

The value of the reference modulation factor $z_0 = d_0 v_0$ is preferably given by the expression:

$$z_0 = (\varepsilon/C^-)N^\varphi.$$

where the constants have the following values: $\alpha = 0.1624$, $\beta = 2.2307$, $\gamma = 0.0328$ and $\delta = -2.361$, $\varepsilon = 3.2605$ and $\varphi = -0.6166$.

In alternatively preferred applications of the inventive method it may be preferred to estimate the concentration of particles in a large reservoir (e.g. of liquid or bodily fluids etc.) from which the specimen was taken. Then the placement of particles in the compartments is treated as a set of independent random variables being functions of the concentration of particles in the sample $C_{sample} = C_{reservoir}(V_{specimen}/V_{sample})$ and of the volumes and dilution ratios of the compartments. Then the estimate of concentration of particles delivered by the assay will describe the estimate $E(C_{reservoir}) = E(C_{sample})(V_{sample}/V_{specimen})$ and the relative standard deviation $\sigma$ of such an estimate. The appropriate algorithm for analysis of the outcome of the assay, is to treat the stochastic distribution of particles between compartments as a distribution of identical independently distributed variables. The standard deviation of the estimate given by such analysis depends on the concentration $C_{sample} = C_{reservoir}(V_{specimen}) V_{sample})$ and the estimate itself depends on the signal from the assay (see Fig. 9a) and 9c) and respective part of the detailed figure description).

In alternatively preferred applications of the inventive method it is preferred that the relative standard deviation $\sigma$ of the estimate is not larger than a known threshold within a given range of concentrations $C_{reservoir}$. The relation between the dynamic range of concentrations $\Omega_{tot} = C_{reservoir}^+/C_{reservoir}^-$. The maximum value of the relative

standard deviation $\sigma$ of the estimates within the range $C_{sample} \in (C^-, C^+)$, $\Omega(\sigma)$ has a characteristic deflection point $\frac{d^2\Omega}{d\sigma^2} = 0$ that defines the optimum combination of the dynamic range $\Omega(N)$ and precision $\sigma(N)$ as a function of the number of compartments $N$. A single assay comprising $N$ compartments has the disadvantage that it does not allow to tune its dynamic range and precision independently of each other. The solution to this technical problem is to use a combination of assays, each comprising a number of identical compartments, with the volume and/or dilution factor of the compartments different between the assays. Ismagilov et al [Shen, F.; Sun, B.; Kreutz, J. E.; Davydova, E. K.; Du, W.; Reddy P. L.; Joseph, L. J.; Ismagilov, R. F., "Multiplexed Quantification of Nucleic Acids with Large Dynamic Range Using Multivolume Digital RT-PCR on a Rotational SlipChip Tested with HIV and Hepatitis C Viral Load", J. Am. Chem. Soc., Article ASAP (X 2011)] have shown such a combination yet the solution provided did not minimize the number of compartments required to achieve a given set of values of the dynamic range and the relative standard deviation of the estimate of concentration. The present inventors have unexpectedly found a solution to this problem that allows minimization of the total number of compartments for the required and independently predetermined dynamic range and precision of the estimate of concentration of particles. According to the invention, given the required values of the dynamic range $\left(\Omega_{\text{tot}} = C^+_{reservoir}/C^-_{reservoir}\right)$ of the combined assay and the maximum relative standard deviation ($\sigma$) of the estimate of concentration $E(C_{sample})$ returned by the assay, the parameters of the inventive method can be determined with the use of the following simple scheme. The number $N_{lib}$ of sets of identical compartments is preferably given by:

$$N_{lib} = \frac{\log(\Omega_{\text{tot}})}{\log(\alpha \cdot \sigma^\beta)}.$$

The number ($N'$) of compartments in each set is preferably given by:

$$N' = \gamma \cdot \sigma^\delta,$$

and the common factor $x = d_{i+}1v_{i+1}/d_iv_i$ of the geometric series of the modulation factor $z_i = d_iv_id_0v_0 = x^i$, where $i \in (0, N_{lib} - 1)$, characterizing compartments in different assays is preferably given by:

$$x = \Omega_{\text{tot}}^{1/N_{lib}}.$$

The value of the reference modulation factor $z_0 = d_0v_0$ is preferably given by the expression:

$$z_0 = \left[\varepsilon/\left(C^-_{reservoir}(V_{specimen}/V_{sample})\right)\right]N^\varphi$$

where the constants have the following values: $\alpha = 0.6813$, $\beta = -2.0966$, $\gamma = 0.9925$ and $\delta = -2.065$, $\varepsilon = 0.746$ and $\varphi = -0.2357$.

In accordance with the second aspect of the present invention an apparatus for use in determining a concentration of particles in accordance with the inventive method is provided. All of the embodiments of the first aspect of the present invention can be combined independently from each other with respect to the second aspect of the invention.

The inventive apparatus enables the determination of the number ($N$) and the determination of the modulation factor ($z_i$) by suitable means. Suitable means are common apparatus, preferably comprising a computer with a memory device configured with executable instructions stored thereon, the instructions - when executed by a processor, cause the processor to determine the number ($N$) and the modulation factor ($z_i$) in accordance with the inventive method of the first aspect of the present invention. Thus, the inventive apparatus enables meeting the needs underlying the present invention at least partly.

In accordance with a cumulatively or alternatively preferred embodiment of the inventive apparatus the apparatus is configured to

c) partitioning at least part of the predetermined sample volume into the determined number ($N$) of separate compartments, wherein at least part, preferably all of the compartments comprise the predetermined sample volume with the determined modulation factor ($z_i$)

d) optionally amplifying the particles of the compartments with one or more suitable reagents and optionally one or more diluents for obtaining a measurable signal indicating the presence of a predetermined threshold number of particles, preferably one, two, three or more particles in a compartment,

e) measuring the signals in each of the ($N$) compartments and assigning to at least part, preferably all compartments a value ($k_i$), wherein ($i$) represents the index number of the compartments represented by integers 0 to $N$ - 1 and compartment ($i$) is assigned a first value ($k_i$), if the compartment ($i$) comprises or consists of the predetermined threshold number of particles or more, and compartment ($i$) is assigned a second value ($k_i$), if the compartment ($k_i$) comprises or consists of less than the threshold number of particles indicating the first value and

f) determining the estimated concentration of particles $E(C)$, wherein the estimated concentration of particles $E(C)$ is determined by a function of

i. a *sum K* of the values ($k_i$), wherein the function preferably is corrected using a calibration correction function, and/or

ii. a *sum K* of weighted values ($w_i k_i$), wherein at least part of the values ($k_i$), preferably each value ($k_i$) is modulated with a weight value ($w_i$), wherein ($i$) represents the index number of the compartments represented by the integers 0 to $N$ - 1*,* and wherein the weight value ($w_i$) preferably is inversely proportional to the degree of volume and/or dilution of the number ($i$) of the compartments, and wherein the function is preferably corrected using a calibration correction function, and/or

iii. a number, vector, matrix, tensor of any order or any other unambiguous representation a *microstate* μ, wherein the number, vector, matrix, tensor of any order or any other representation comprises or consists of information reflecting at least part of the values ($k_i$) measured in step e), preferably comprises or consists of a vector $k \equiv \{k_i\}$, representing the *microstate* μ, wherein the vector ($k$) comprises or consists of at least part, preferably all of the values ($k_i$) measured in step e).

The preferred inventive apparatus enables carrying out both the determination of the number (N) and the determination of the modulation factor ($z_i$) by suitable means as described hereinbefore as well as the determination of the estimate of concentration $E(C)$ by carrying out the optional amplification reaction with suitable means, the measurement of the values with suitable means, assignment of the first and second values $k_i$ with suitable means as well as the actual determination of the final determination of the estimate of concentration $E(C)$ by suitable means, such as a computer with a memory device configured with executable instructions stored thereon, the instructions - when executed by a processor, cause the processor to determine the estimate of concentration $E(C)$ in accordance with the present invention.

Suitable means for configuring the preferred apparatus have been disclosed with respect to the first aspect of the present invention and can be combined independently from each other with the aforementioned preferred apparatus of the present invention.

According to a third aspect of the present invention the use of the inventive method or the inventive apparatus is provided for

a) adjusting, preferably reducing the total number (N) of compartments comprising the predetermined sample volume and/or

b) adjusting, preferably reducing the total volume of a mixture comprising or consisting of sample, one or more reagents suitable for amplifying at least part of the particles to a measurable signal and optionally one or more diluents in all of the (N) compartments and/or

c) predetermining the volume, preferably the minimum or maximum volume of the mixture comprising or consisting of sample, one or more reagents suitable for amplifying at least part of the particles to a measurable signal and optionally one or more diluents in each of the (N) compartments and/or

d) predetermining the modulation factor ($z_i$), preferably the minimum or the maximum suitable modulation factor ($z_i$) for partitioning the predetermined sample volume into the (N) compartments.

All the preferred embodiments of the first and second aspect of the present invention can independently from each other be combined with the embodiments of the third aspect of the present invention.

According to a fourth aspect of the present invention a use of the sample holder in an inventive method or with an inventive apparatus is provided, wherein the sample holder is inventively configured to,

a) comprising or consisting of a predetermined number (N) of compartments wherein the number (N) is smaller or equal to the value of the function

$$N_{MAX} = A \cdot \ln\left(\frac{C^+}{C^-}\right)/\sigma^2_{MAX}$$

wherein ($A$) represents a real number being the integer 6, wherein ($C^+$) represents a predetermined upper limit of the interval of concentration (C) to be estimated by the method, wherein ($C^-$) represents a predetermined lower limit of the interval of concentration (C) to be estimated by the method, wherein ($\sigma_{MAX}$) represents a predetermined maximum allowable relative standard deviation of the estimate of concentration (C) of particles, wherein $C^- < C < C^+$, and

b) wherein the ($N$) compartments are configured to comprising the predetermined sample volume with a predetermined modulation factor ($z_i$), wherein ($z_i$) is a function of volumes ($v_i$) and dilution factors ($d_i$) of the sample in at least part of or all of the ($N$) compartments so that at least part of or all of the two or more of the ($N$) compartments can comprise or consist of different sample volumes ($v_i$) and/or different dilution factors ($d_i$) of the sample, wherein ($i$) represents an index number of the ($N$) compartments represented by the integers 0 to $N$ - 1, and wherein ($v_i$) represents the volume and ($d_i$) represents the dilution factor of the sample in the compartment ($i$).

All the preferred embodiments of the first, second and third aspect of the present invention can independently from each other be combined with the embodiments of the fourth aspect of the present invention.

Inventive sample holders can preferably comprise suitable test tubes, arrays of wells on a microarray, or a microfluidic chip, a microfluidic chip configured to generating droplets as well as other commercially available or otherwise generally known devices capable of holding discrete volumes and allowing amplification and measurement of respective signals.

The fifth aspect of the present invention relates to a use of a kit comprising the inventive sample holder and one or more reagents suitable for amplifying at least part of particles comprised in the compartments of the sample holder to a measurable signal and optionally one or more suitable diluents for determining a concentration of particles, preferably for determining the concentration of particles in accordance with the inventive method.

All the preferred embodiments of the first, second, third and fourth aspect of the present invention can independently from each other be combined with the embodiments of the fifth aspect of the present invention.

### Detailed Description of the Figures:

### Figures 1a), 1b) and 1 c)

Figures 1 a), 1b) and 1c) show the comparison of the performance of different digital assays designs.

In figure 1a) the shaded area shows the combinations of dynamic range and precision of the estimation of the concentration (expressed as the relative standard deviation of the estimate of concentration) achieved by inventive quantitation assays with volume of compartments determined by the geometrical sequence designed with $x = 1 - \alpha \cdot \sigma^\beta_{max}$, $\Delta N = \gamma \cdot (1 - x)^{-\delta}$, $N = 2 \cdot \Delta N - \log_x(\Omega)$ and $v_o = \ln 2 \cdot x^{-\Delta N} 1/C^-$, where $\alpha = 1.24$, $\beta = 1.9493$, $\gamma = 4.5637$ and $\delta = 0.798$. The grey lines show exemplary traces for a constant number of compartments in the geomtric assay. The black dotted line shows the possible combinations of relative standard deviation and dynamic range of the assay with identical compartments treated as identical and independent random variables, while the black dashed line indicates the same for the assay comprising identical compartments treated as dependant random variables. Open square ($\square$) indicates the assay comprising identical compartments treated as independent random variables and covering concentration range $\Omega = 10^6$. Grey solid square ($\blacksquare$) indicates the assay comprising identical compartments treated as dependent random variables and covering concentration range $\Omega = 10^6$.

Figure 1b) shows the possible tuning of assays designed with the present inventive method while keeping the dynamic range constant and equal $\Omega = 10^6$. The graph shows the minimum number of compartments as a function of the parameter $p = \ln(\Omega)/\sigma^2$ for the present inventive designs and designs from the state-of-art.

Long-dashed line represents inventive quantitation assays comprising sequence of volumes given by $v_i = A \cdot exp^{(B \cdot \frac{i}{N})}$ whereas $A = 1/C^+$ and $= [1.95 \cdot ln\{(C^+/C^-)/N\}]^{0.856}$ while the number of compartment in such assays are equal to $N = 2 \cdot \sigma_{MAX}^{-1.9} \cdot (\frac{C^+}{C^-})^{0.25})$, and covering concentration range $\Omega = 10^6$.

Dotted line represents inventing quantitation assays comprising more preferable $N = 2 \cdot \sigma_{MAX}^{-1.94} \cdot (\frac{C^+}{C^-})^{0.22}$ compartments, and covering concentration range $\Omega = 10^6$.

Solid line represents inventive quantitation assays with volume of compartments determined by the geometrical sequence designed with $x = 1 - \alpha \cdot \sigma_{max}^{\beta}$, $\Delta N = \gamma \cdot (1 - x)^{-\delta}$, $N = 2 \cdot \Delta N - log_x(\Omega)$ and $v_o = ln2 \cdot x^{-\Delta N}1/C^-$, where $\alpha = 1.24$, $\beta = 1.9493$, $\gamma = 4.5637$ and $\delta = 0.798$, and covering concentration range $\Omega = 10^6$.

Dashed-dotted line represents inventive multivolume assays designed to provide the estimate of concentration ($C_R$) of particles in a reservoir of particles that is larger than the sample drawn from that reservoir. The assay is designed with the use of the following equations: $N_{lib} = \frac{\log(\Omega_{tot})}{\log(\alpha \cdot \sigma_{tot}^{\beta})}$, $N' = \gamma \cdot \sigma_{tot}^{\delta}$ and $x = \Omega^{1/Nlib}$, where $\alpha = 0.6813$, $\beta = -2.0966$, $\gamma = 0.9925$ and $\delta = -2.065$, and covering concentration range $\Omega = 10^6$.

Short-dashed (2) line represent multivolume quantitation assays designed to provide an estimate of the concentration ($C_{sample}$) of particles in the sample. The assay is designed with the use of the following equations:

$$N_{lib} = \frac{\log(\Omega_{tot})}{\log(\alpha \cdot \sigma_{tot}^{\beta})}, N' = \gamma \cdot \sigma_{tot}^{\delta} \text{ and } x = \Omega^{1/Nlib}, \text{ where } \alpha = 0.1624, \beta = 2.2307, \gamma = 0.0328 \text{ and } \delta = -2.361, \text{ and covering}$$

concentration range $\Omega = 10^6$.

Open square (□) indicates assay comprising identical compartments treated as independent random variables and covering concentration range $\Omega = 10^6$.

Grey solid square (■) indicates the assay comprising identical compartments treated as dependent random variables and covering concentration range $\Omega = 10^6$.

Figure 1c) shows a comparison of the number $N$ of compartments required yielding a given dynamic range and precision in accordance with different designs of the assays relating to the present invention as well as relating to the prior art in comparison thereto. The number of compartments normalized by the function $N_{max} = 6p$ is plotted as a function of the parameter $p = ln(\Omega)/\sigma^2$.

Open (◊) and solid (♦) black rhombs show the performance of the inventive assays in which compartment volumes are determined by geometric progression of the modulation factors, with $v_i = A \cdot exp^{(B \cdot \frac{i}{N})}$ whereas $A = 1/C^+$ and $= [1.95 \cdot ln\{(C^+/C^-)/N\}]^{0.856}$. The number of compartments in such assays are equal to $N = 2 \cdot \sigma_{MAX}^{-1.9} \cdot (\frac{C^+}{C^-})^{0.25}$ (assays marked with solid black rhombs ♦), or preferably $N = 2 \cdot \sigma_{MAX}^{-1.94} \cdot (\frac{C^+}{C^-})^{0.22}$ (assays marked with open black rhombs ◊).

Solid triangles (▲) represent inventive quantitation assays with volume of compartments determined by the geometrical sequence designed with $x = 1 - \alpha \cdot \sigma_{max}^{\beta}$, $\Delta N = \gamma \cdot (1 - x)^{-\delta}$, $N = 2 \cdot \Delta N - log_x(\Omega)$ and $v_o = ln2 \cdot x^{-\Delta N}1/C^-$, where $\alpha = 1.24$, $\beta = 1.9493$, $\gamma = 4.5637$ and $\delta = 0.798$.

Open triangles (△) represent inventive quantitation assays with volume of compartments determined by the geometric sequence with repetitions (or $N'$ copies of each compartment), where for a given number of copies ($N'$) the common factor of the geometric series has a value $x = 1 - \alpha \cdot (\sigma_{max}\sqrt{N'})^{\beta}$, and all other parameters are defined in the same way as for the geometric series without repetitions.

Open black circles (○) represent the number of compartments of an inventive multivolume assays designed to provide the estimate of concentration ($C_{reservoir}$) of particles in a reservoir of particles that is larger than the sample drawn from that reservoir. The assay is designed with the use of the following equations: $N_{lib} = \frac{\log(\Omega_{tot})}{\log(\alpha \cdot \sigma_{tot}^{\beta})}$, $N' = \gamma \cdot \sigma_{tot}^{\delta}$ and $x = \Omega^{1/Nlib}$, where $\alpha = 0.6813$, $\beta = -2.0966$, $\gamma = 0.9925$ and $\delta = -2.065$.

Solid black circles (●) represent multivolume quantitation assays designed to provide an estimate of the concentration ($C_{sample}$) of particles in the sample. The assay is designed with the use of the following equations:

$$N_{lib} = \frac{\log(\Omega_{tot})}{\log(\alpha \cdot \sigma_{tot}^{\beta})}, N' = \gamma \cdot \sigma_{tot}^{\delta} \text{ and } x = \Omega^{1/Nlib}, \text{ where } \alpha = 0.1624, \beta = 2.2307, \gamma = 0.0328 \text{ and } \delta = -2.361.$$

Plus-signs and with dashed line represent the performance of the multivolume assays of the prior art described by R.F. Ismagilov in F. Shen, R. F. Ismagilov et al., JACS 2011 133: 17705-17712.

The black solid line shows the clear demarcation line between the solution provided by Ismagilov and the more technically favorable solutions disclosed in this application. The distinction between the quantitation assays described here and multivolume assays presented previously is clearly seen.

### Figures 2a) and 2b)

Fig. 2 a) shows a graph of the dynamic range $C^+/C^- = \Omega$ and relative standard deviation $\sigma$ provided by the inventive quantitation assay comprising $N$ compartments with volumes determined by the geometrical sequence with common ratio (or quotient) $x$. The dynamic range $C^+/C^- = \Omega$ can be tuned independently of the precision $\sigma$, and for a given required dynamic range, the assays proposed require even $10^2$, to $10^4$ times less compartments than the standard digital assays.

Fig. 2b) shows a graph displaying the relation between the common ratio $x$ of the geometrical sequence determining the volumes of the compartments of the inventive assay and number of copies of each compartment in inventive assays that provide the maximum relative standard deviations $\sigma(C)$ of the estimate $E(C)$. For any predetermined dynamic range, and precision, the factor $x$ of geometric progression of volumes can be interchanged with the number $N'$ of copies of the compartments allowing to tuning the design of the assay to the technical requirements in its realization.

### Figures 3a), 3b), 3c) and 3d)

Figure 3a) shows an inventive assay comprising compartments of different volumes, each compartment addresses its own characteristic concentration $C$. Equivalently, for any arbitrary input concentration $C^*$ only one of the compartments addresses this concentration most closely. The progressively larger (or smaller) compartments carry progressively less information about the input concentration. This is reflected in that the probability of obtaining a signal from these compartments approaches unity (or zero). The active stripe (here marked with the grey dashed rectangle and comprising compartments with $v_{i^*-1}$, $v_{i^*}$, $v_{i^*+1}$) is defined as a finite set of compartments characterized by probability of giving a signal significantly different than zero or one. Compartments outside of the active stripe with a larger volume than in the active stripe (dark grey marked compartments with $v_{i^*-2}$, $v_{i^*-3}$, $v_{i^*-4}$) comprise a probability that always a positive signal, i.e., indicating that at least the threshold amount will be measured, whereas for compartments outside the active stripe comprising a smaller volume than in the active stripe (light grey marked compartments with $v_{i^*+2}$, $v_{i^*+3}$, $v_{i^*+4}$) comprise a probability that always a negative signal, i.e., indicating that less than the threshold amount will be measured.

Figure 3b) shows an active stripe in accordance with the present invention comprising a set of test-volumes yields stochastic combinations of signals (microstates) in every run of the assay, wherein the integer 1 indicates that at least the threshold amount of particles, preferably at least 1 particle is present in the compartment and the integer 0 indicates that less than the threshold amount, preferably no particle is present in that compartment. Each microstate yields $\rho_{\mu_i} = \rho(C|\mu_i)$ that provide more information than that $(\rho_K)$ retrieved from the sole number of values $K$. Each of these distributions are characterized by a different standard deviation (i.e. different information about C). The envelope (i.e. the maximum value) of the standard deviation of the distribution $\rho(C)$ is given by the most probable microstate. In accordance with the summation ($\Delta$) and microstate ($\blacklozenge$) approach this is illustrated for an active stripe comprising three compartments in figure 3c) and sixteen compartments in figure 3d). Interestingly, many of the less probable microstates carry significantly more information about $C$ than the most probable ones.

### Figures 4a), 4b) and 4c)

Figures 4a), 4b), 4c) show graphs displaying the standard deviation of the estimate of concentration of particles, for two different approaches (summation ($\Delta$) and microstate ($\blacklozenge$) approach) of analyzing the signals from the compartments in accordance with the inventive method. The triangles show the standard deviation of the estimate of concentration of particles derived from the sum of the value $K = \sum_{i=0}^{N-1} k_i$ (summation), while the rhombs show the standard deviation of the estimate of concentration of particles derived from each unique microstate $\mu = \{k_i\}$ that is possible for a given value of $K$. For the vast majority of the possible microstates the standard deviation of the concentration of the particles derived from the microstates is smaller than that based on the sum of values. Each graph corresponds to a different assay with a different quotient of the geometric series $d_{i+1}v_{i+1} = x(d_i v_i)$.

### Figures 5a) and 5b)

Figure 5a) shows a graph displaying the probability distributions $p(\mu|C)$ for the ten most frequently occurring microstates (grey lines) of the inventive quantitation assay of the sample with a volume of 20 $\mu$L, designed so as to offer the estimate of the concentration of particles with a relative standard deviation less than $\sigma_{max}$ = 62% in the concentration range from $C^-$ = 1 mL$^{-1}$ to $C^+$ = $10^2$ mL$^{-1}$, i.e., $C^+/C^-$ = $10^2$. The required dynamic range is accomplished by partitioning the sample into 10 partitions with dilution ratios $C_i/C_S$ = $d_i$ = $x^i$, where $x$ = 0.5. The black line marks the microstate, in which the first five compartments show the presence of at least one copy of the target particle. The width of the distribution defines the measurement precision, in this case equal to 62%. Each curve shown in the Figure results from the product of terms (1 - $e^{-v_iC}$) for positive partitions and $e^{-v_iC}$ for negative partitions that form a steep ridge on the left side (positive partitions), and on the right side (negative partitions), respectively.

Figure 5b) shows a graph displaying the probability distributions $p(\mu|C)$ for the first 90 most frequently occurring microstates (grey lines) of the inventive quantitation assay of the sample with a volume of 20 $\mu$L, designed so as to offer the estimate of the concentration of DNA particles in the said sample with a relative standard deviation less than $\sigma_{max\_1}$=62% in the concentration range from $C_1^-$ =$10^2$ [1/mL] to $C_1^+$=$10^4$ [1/mL], i.e., $(C_1^+/C_1^-)$=$10^2$, $\sigma_{max\_2}$=25% in the concentration range from $C_2^-$=$10^4$ [1/mL] to $C_2^+$=$10^6$ [1/mL], i.e., $(C_2^+/C_2^-)$=$10^2$, and $\sigma_{max\_3}$=62% in the concentration range from $C_3^-$=$10^6$ [1/mL] to $C_3^+$=$10^8$ [1/mL], i.e., $(C_3^+/C_3^-)$=$10^2$. The required dynamic range is accomplished by partitioning the sample into consecutively 14 compartments with dilution ratios $C_i/C_S$ = $d_i$ = $x^i$, where $x$ = 0.5, 86 compartments with $x$ = 0.917, and 14 compartments with $x$ = 0.5. Using numerical algorithms presented in the description of the invention, the estimate $E(C_S)$ is calculated based on the conditional probability distribution $p(C_S|\mu)$, where $\mu$ is a microstate of the system of partitions used to perform the analytical assay. The black line marks two microstates; in the first one (on the left) the first seven 7 partitions show the presence of at least one copy of the target particle, while the remaining ones are negative, in the second case (on the right) - the first 57 partitions show the presence of at least one copy of the target particle, while the remaining ones are negative. One can easily differentiate the microstates from the first group ($x$ = 0.5), and from the second group ($x$ = 0.917). The microstates from the second group are clearly narrower (which results in a higher precision of the assay) and occur more densely on the concentration axis.

**Figure 6**

Figure 6 shows a graph displaying the calibration correction function $f_{corr}$ = $C/E(C)$ of initial concentration $C = \dfrac{M}{V}$ of particles for the inventive quantitation assay comprising 2,814 compartments with dilution factors $d_i = \dfrac{c_i}{c} = x^i$, where $x$ = 0.99814, where signals from compartments are weighted by the factor $w_i$ = 1/$d_i$.

**Figures 7a), 7b) and 7c)**

Figure 7a) shows a graph displaying the sum of values $K = \sum w_i k_i$, with weights $w_i$ = 1/$d_i$ as a function of the number of particles M randomly distributed over N=2,814 compartments in accordance with the inventive method. The compartments, indexed $i$, are characterized by dilution factors $d_i = \dfrac{c_i}{c} = x^i$, with the quotient of the geometric series $x$=0.99814. The number of compartments and the quotient x are selected so that the quantitation assay offers an estimate of concentration of particles $c$ = $E(C)$ with a maximum relative standard deviation less than $\sigma_{MAX}$=10% within a dynamic range of concentrations $C^+/C^-$ = $10^5$. The results presented in Fig. 1 are obtained form 100 iterations of the numerical algorithm.

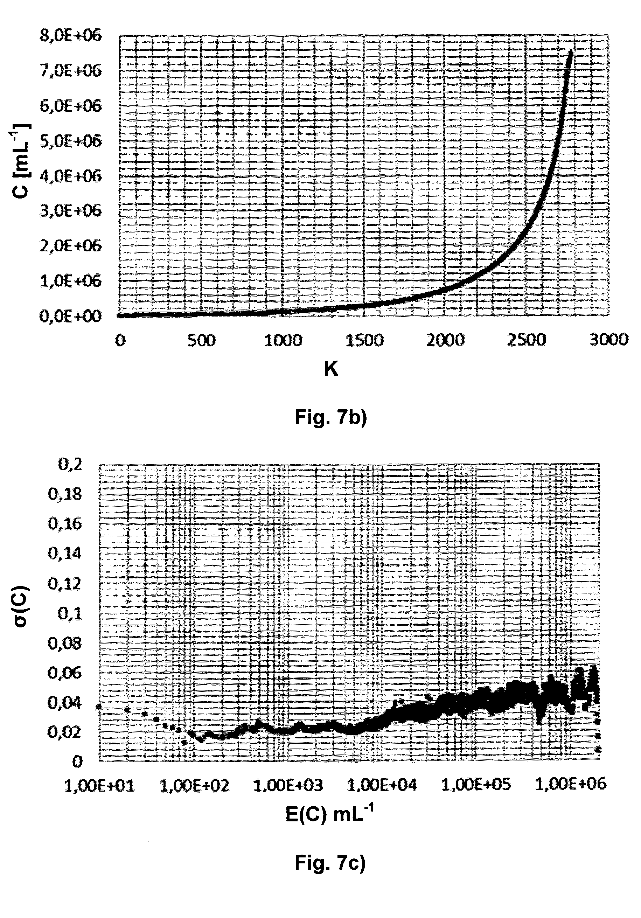

Figure 7b) shows a graph displaying the value of the estimate $E(C)$ of initial concentration of particles as a function of $K$, where $K$ is the sum of weighted signals $K = \sum w_i k_i$. with weights $w_i$ = 1/$d_i$ for the inventive quantitation assay comprising 2,814 compartments with dilution factors $d_i = \dfrac{c_i}{c} = x^i$, where $x$ = 0.99814.

Figure 7c) shows a graph displaying the relative standard deviation $\sigma(C)$ of the estimate $E(C)$ as a function of the logarithm of initial concentration $C = M/V$ of particles for the inventive quantitation assay comprising 2,814 compartments with dilution factors $d_i = \dfrac{c_i}{c} = x^i$, where $x$ = 0.99814, where signals from compartments are weighted by the factor $w_i$ = 1/$d_i$.

**Figures 8a), 8b) and 8c)**

The graphs of figures 8a), 8b) and 8c) show the results of 100 Monte Carlo (MC) simulations for each of the following assays and 7 or 8 experiments as experimental and numerical verification of the performance of the inventive method (see also example section). In accordance with the present invention a set of geometric assays, with varied dynamic ranges and precision: (i) $\Omega = 10^3$, $\sigma \leq 80\%$, $N = 10$, $x = 0.50$, (ii) $\Omega = 10^2$, $\sigma \leq 60\%$, $N = 10$, $x = 0.60$, and (iii) $\Omega = 10^1$, $\sigma \leq 25\%$, $N = 30$, $x = 0.92$ has been designed. Each of the above inventive assays was verified with a Monte Carlo simulation. In addition, the inventive quantitation assays were run experimentally. For each inventive assay the trails were done with a single fixed value of the input concentration $C_{input}$: 1420, 568 and 66 [particles/mL] for a, b and c, respectively. The results from MC are shown as black lines showing the 70% confidence interval ($E(C) \pm \sigma$). The expected values retrieved from the signals from the experimental assays are shown with red rhombs and the 70% confidence intervals with the error bars. The grey stripes show the range of $C_{input} \pm \sigma_{max}$.

**Figures 9a) and 9b)**

The graphs of figures 9a) and 9b) show the standard deviation of the estimate of concentration/number of molecules in the sample based on i.i.d. (dashed line) and non-i.i.d. approach (solid line) and the estimate of concentration itself as a function of signal. Dotted lines show the exemplary threshold values of the standard deviation of the estimate. For each threshold one can univocally determine the dynamic range $\Omega = C^+/C^-$, where $C^-$ is the minimum and $C^+$ is the maximum concentration assayed with at least the required precision. The 'wavy' behavior of the sigma distribution for non-i.i.d. approach is caused by computing accuracy.

**Further embodiments of the invention part B:**

   1. A method for determination of the concentration of particles in a sample, comprising the following steps:

   a) division of the sample into $N$ disjoint sub-volumes, each having volume of $v_i$, where $i$ is the index of the sub-volume, wherein

   •   either the sample is divided into sub-volumes in a deterministic way, i.e. such that the volumes $v_i$ are given by a sequence of values of an analytical function $f(i)$ or by a predefined set of values,

   •   or the sample is divided into sub-volumes in a random way, i.e. such that the distribution of volumes of the sub-volumes corresponds to a probability distribution $P(v)$, meaning that the number of sub-volumes having the volume of $v_i$ in the range ($v$, $v + \Delta v$) is given by the expression: $N \int_{v}^{v+Dv} P(v)dv$;

   b) conducting in each of the thus obtained sub-volumes the process of amplification of the presence of at least one said particle to a measurable signal;

   c) measuring said signal in each of the sub-volumes and assigning to this sub-volume the parameter $k_i$, wherein the parameter $k_i$ has the value equal to 1, if the result of said signal measurement indicates that in the given sub-volume there is at least one said particle and has the value equal to 0 otherwise, and $i$ is the number of the sub-volume;

   d) calculating the sum $K = \sum k_i \cdot w_i(v_i)$, where $i$ is the number of the sub-volume and the sum is over all the sub-volumes into which the sample was divided $i = 1 ... N$, and $w_i(v_i)$ is a weight, which may depend on the volume of the sub-volume;

   e) determination of an estimate $E(C)$ of the concentration of said particles in the sample based on the values of $K$, $N$ and $\{v_i\}$ or $P(v)$, with a known statistical algorithm based on the Bayes formalism,

   characterized in that, the division of the sample in step a) includes at least two sub-volumes having different volumes.

The disclosure with respect to the invention underlying Polish patent application PL 397028 (filed: 17 November 2011; title:" Sposób przeprowadzania cyfrowych oznaczeń analitycznych i diagnostycznych") in particular including all preferred embodiments and examples thereof and the relating figures are incorporated herein in its entirety and display also further inventive embodiments with respect to the present invention.

**2. <u>Further embodiments of the invention part C:</u>**

1. A method for determination of the concentration of particles in a sample, comprising the following steps:

a) division of the sample into $N$ disjoint sub-volumes, each having volume of $v_i$, where $i$ is the index of the sub-volume, wherein

- either the sample is divided into sub-volumes in a deterministic way, i.e. such that the volumes $v_i$ are given by a sequence of values of an analytical function $f(i)$ or by a predefined set of values,

- or the sample is divided into sub-volumes in a random way, i.e. such that the distribution of volumes of the sub-volumes corresponds to a probability distribution $P(v)$, meaning that the number of sub-volumes having the volume of $v_i$ in the range $(v, v + \Delta v)$ is given by the expression: $N \int_{v}^{v+\Delta v} P(v) dv$;

b) conducting in each of the thus obtained sub-volumes the process of amplification of the presence of at least one said particle to a measurable signal;

c) measuring said signal in each of the sub-volumes and assigning to this sub-volume the parameter $k_i$, wherein the parameter $k_i$ has the value equal to 1, if the result of said signal measurement indicates that in the given sub-volume there is at least one said particle and has the value equal to 0 otherwise, and $i$ is the number of the sub-volume;

d) determination of an estimate m of the concentration of said particles in the sample based on the set of values $\{k_i\}$, $N$ and $\{v_i\}$ or $P(v)$ with known statistical algorithms;

2. characterized in that the volume $v_i$ of each of the $N$ sub-volumes into which the sample is divided in the step a) is different.

The disclosure with respect to the invention underlying Polish patent application PL 397027 (filed: 17 November 2011; title:" Sposób przeprowadzania cyfrowych oznaczeń analitycznych i diagnostycznych") in particular including all preferred embodiments and examples thereof and the relating figures is incorporated herein in its entirety and display also further inventive embodiments with respect to the present invention.

**3. <u>Further embodiments of the invention part D:</u>**

1. A method for determination of an unknown concentration $C_P$ of particles in a sample having a known volume $V_P$, comprising the following steps:

a) mixing the sample having the volume of $V_P$ with the volume of $V_R$ of a solution of reagents required for conducting a reaction of amplification of the presence of at least one of said particles to a measurable signal, as a result of which a reactive solution having the volume of $V_S$ containing unknown concentration $C_S = C_P \cdot V_P/(V_P+V_R)$ of said particles is obtained,

b) creating, from the reactive solution having the volume of $V_P$ and of the additional solution of reagents required for conducting of the reaction of amplification of the presence of at least one of said particles to a measurable signal, $N$ of disjoint sub-volumes having the volume of u each, in such a way that each of those sub-volumes comprises the required concentration of reagents and a given dilution $d_n$ of said particles, where $d_n = \dfrac{c_n}{c_0}$, where the index n indexes the sub-volumes and goes over the integer numbers from $0$ to $N-1$, and $C_n$ denotes

the unknown concentration of said particles in the sub-volume having the number n,

c) conducting in each of the thus obtained sub-volumes the process of amplification of the presence of at least one of said particles to a measurable signal;

d) measuring said signal in each of the sub-volumes and assigning to this sub-volume the parameter $k_n$, wherein the parameter $k_n$ has the value equal to 1, if the result of said signal measurement indicates that in the given sub-volume there is at least one said particle and has the value equal to 0 otherwise, and n is the number of the sub-volume;

e) determination of the estimate $E(C_S)$ of the concentration of said particles in the reactive solution having the volume of $V_S$, based on the set of values of $\{k_n\}$, $N$ and $\{d_n\}$, using a known statistical algorithm, preferably based on the Bayes formalism,

f) determination of the concentration $C_P$ from the formula: $C_P = C_S \cdot (V_P + V_R)/V_P$

2. characterized in that for at least two of the sub-volumes mentioned in the step b) the dilutions $d_n$ are different.

The disclosure with respect to the invention underlying Polish patent application PL 397026 (filed: 17 November 2011; title:" Sposób przeprowadzania cyfrowych oznaczeń analitycznych i diagnostycznych") in particular including all preferred embodiments and examples thereof and the relating figures is incorporated herein in its entirety and display also further inventive embodiments with respect to the present invention.

### 3. <u>Further embodiments of the invention part E:</u>

1. A method for determining unknown concentration $C_S$ of particles in a sample of known volume $V_S$, comprising the following steps:

a) partitioning the said sample into $N$ separate compartments of volume $v_i$ and dilution $d_i$ each, by mixing of at least a fraction of the sample with certain volume of the solution of reagents required to carry out the reaction amplifying the presence of at least one target particle to a measurable signal, where $0 < d_i \leq 0$ means the dilution factor, i.e., the ratio of the concentration $C_i$ of particles in a given compartment to the unknown concentration $C_S$ of particles in the sample, $d_i = C_i/C_S$, and $i$ is the compartment number, running over integers from $0$ to $N - 1$;

b) performing in each compartment so produced a process amplifying the presence of at least one said particle to a measurable signal;

c) measurement of the said signal in each compartment and assigning to the said compartment a parameter $k_n$, said parameter $k_i$ assuming a value of 1, if the result of the said measurement indicates that a given compartment contains at least one said particle, and 0 in the opposite case;

d) constructing a vector $k \equiv \{k_i\}$, specifying a *microstate* $\mu$, containing in sequence the values of $k_i$ parameter measured in step c);

2. characterised in that

e) the unknown concentration $C_S$ of particles in a sample is calculated as an estimate being a function of the vector $k$ constructed in step d) and specifying the microstate $\mu$: $E(C_S) = f(\mu)$, whereas at least two from among the compartments produced in step a) differ from each other by the value of the modulation factor $z_i$, equal to the quotient $z_i = (v_i d_i)/(v_0 d_0)$.

The disclosure with respect to the invention underlying Polish patent application PL 399673 (filed: 26 June 2012; title:" Sposób przeprowadzania cyfrowych oznaczeń analitycznych i diagnostycznych") in particular including all preferred embodiments and examples thereof and the relating figures is incorporated herein in its entirety and display also further inventive embodiments with respect to the present invention.

### 3. **Further embodiments of the invention part F:**

1. A method for determining the unknown concentration $C_S$ of particles in a sample of known volume $V_S$, comprising the following steps:

a) partitioning the sample into $N$ sets of separate compartments, referred to as libraries, whereas each set contains $N'_i > 0$ compartments and within each set the value of the expression $d_i v_i$ is set by mixing of at least a fraction of the sample with certain volume of the solution of reagents required to carry out the reaction amplifying the presence of at least one target particle to a measurable signal, where: $v_i$ means the volume of a given compartment, $d_i > 0$ means the dilution factor, i.e., the ratio of the concentration $C_i$ of particles in a given compartment to the unknown concentration $C_S$ of particles in the sample, $d_i = C_i/C_S$, and $i$ is the library number, running over integers from $0$ to $N - 1$;

b) performing in each compartment of each library so produced a process amplifying the presence of at least one said particle to a measurable signal;

c) measurement of the said signal in each compartment Nano and mapping the measured signal in a form of a number, vector, matrix, or tensor of any order, specifying a *microstate* $\mu$, in which the indexes unambiguously identify each compartment with given dilution factor $d_i$ and with given volume $v_i$, and the values of components with given set of indexes map the signal value from that compartment;

2. characterised in that

d) the unknown concentration $C_S$ of particles in a sample is calculated as the estimate being a function of the said number, vector, matrix, or tensor of any order, obtained in step c) and specifying the microstate $\mu$: $E(C_S) = f(\mu)$, whereas at least two from among the libraries produced in step a) differ from each other by the value of the modulation factor $z_i$, equal to the quotient $z_i = (v_i d_i)/(v_0 d_0)$.

The disclosure with respect to the invention underlying Polish patent application PL 399908 (filed: 11 July 2012; title:" Sposób przeprowadzania cyfrowych oznaczeń analitycznych i diagnostycznych") in particular including all preferred embodiments and examples thereof and the relating figures is incorporated herein in its entirety and display also further inventive embodiments with respect to the present invention.

### **Examples:**

In the preferred embodiments of the invention the assays to determine quantitatively the Epstein-Barr virus DNA are performed. The replicated sequence is a fragment of a highly conservative EBNA-1 gene with a length of 218 base pairs. The primers with the following sequences were used: forward primer: *CTATATGCCTGCTTCCTCCGG* CGGACCCGGCCCACAACCTGGC, and reverse primer: *CGCCGGAGGAAGCAGGCATATAG* CGACTCAATGGTGTAAGACGAC. The method was validated by analysing the consistency of assays to determine the DNA concentration for calibrators (plasmid solutions containing EBNA-1 amplicon) used in the real-time PCR reaction with concentration from 10 copies to 10,000 copies per reaction.

A reaction mixture of volume $V_{sample}$ = 20 $\mu$L contains each 4 $\mu$l water, 1 $\mu$l Primer 10x, 10 $\mu$l Master Mix (Hot start DNA, Taq polymerase, dNTP mix, MgCl$_2$), 5 $\mu$l DNA template at concentration $(20/5)C_{sample}$ [copies/mL]. For each experiment, the required volume of a mixture used to dilute the said reaction mixture is also prepared.

In a non-limiting example, the reaction mixture of volume $V_S$ = 20 $\mu$L and the required volume of the mixture of reagents used to dilute the sample are deposited in a microfluidic system made of PDMS or polycarbonate. Then, droplets containing the sample solution of volumes $\{u_{Si}\}$ and droplets containing the reaction reagents of volumes $\{u_{Ri}\}$ are generated on-demand so as to obtain, after merging the droplets with the same indices ($u_{Si} + u_{Ri} \equiv u$), a series of droplets of constant volume u and of a defined progression of unknown concentrations of target molecules $C_i = C_{sample}(u_{Si}/u)$.

In a non-limiting example, the mixture so prepared is pipetted after dilution to test tubes in strips 20 $\mu$l each. After centrifugation, the strips are placed in a LightCycler Nano apparatus.

The following reaction protocol is used: 10 minutes initial denaturation at 95°C, followed by 45 cycles composed of a 30 s denaturation at 95°C, 30 s annealing at 55°C and 30 s elongation at 72°C. The entire procedure is completed by cooling the chip down to 20°C. The partitions displaying high fluorescence level in the channel 530-548 nm are counted.

**Executive Example 1**

In a preferred embodiment, a quantitation assay for a sample with a volume of 20 $\mu$L is designed so as to offer the estimate of the concentration of DNA molecules in the said sample with a relative standard deviation less than $\sigma_{max}$ = 62% in the concentration range from $C^-$ = 1 mL$^{-1}$ to $C^+$ = 10$^2$ mL$^{-1}$, i.e., $C^+/C^-$ = 10$^2$. The assay returns the concentration of the sample calculated with the use of independent random variables, thus, via scaling the estimate of concentration in the sample can be translated into the estimate of concentration in a reservoir. The required dynamic range is accomplished by partitioning the sample into 10 partitions with dilution ratios $C_i/C_{sample}$ = $d_i$ = $x^i$, where $x$ = 0.5. Using numerical algorithms presented in the description of the invention, the estimate $E(C_{sample})$ is calculated based on the conditional probability distribution $f(C_{sample}|\mu)$, where $\mu$ is a microstate of the system of partitions used to perform the analytical assay (see also Fig. 5a) and respective part of the detailed figure description).

In a preferred embodiment, the number of Epstein-Barr DNA copies is determined using a sample with a volume of 20 $\mu$L. The reaction mixture is placed in a system made of PDMS/polycarbonate and 10 droplets on demand with dilution ratios $d_i$ = $x^i$, where $x$ = 0.5, are generated. The system with droplets is placed in a thermocycler, the PCR reaction is carried out, and subsequently the signals from each droplet (microstate of the system) are read out.

For example, a 20 $\mu$L sample containing gene copies at a concentration of $C_{sample}$ = 55.5 mL$^{-1}$ was used. In five repeated experiments the following results were obtained: in the first trial $\mu_1$ = {1,1,1,1,1,0,0,0,0,0}, in the second one $\mu_2$ = {1,1,1,1,0,0,0,0,1,0}, in the third one $\mu_3$ = {1,1,1,0,0,0,0,0,0,0}, in the fourth one $\mu_4$ = {1,1,1,0,0,0,0,0,0,0}, and in the fifth one $\mu_5$ = {1,1,0,1,0,0,0,0,0,0}, where the index numbers the repetition of the experiment. Using the statistical model obtained for a given distribution of dilutions, these values are recomputed into the estimates $c_i$ = $E_i(C_{sample})$ of the initial concentration of DNA copies in the sample $c_1$ = 56.8, $c_2$ = 45.2, $c_3$ = 34.8, $c_4$ = 34.8 and $c_5$ = 27.9 mL$^{-1}$.

**Executive Example 2**

In a preferred embodiment, a quantitation assay for a sample with a volume of 20 $\mu$L is designed so as to offer the estimate of the concentration of DNA molecules in the said sample with a relative standard deviation less than $\sigma_{max}$ = 25% in the concentration range from $C^-$ = 5 mL$^{-1}$ to $C^+$ = 5 $\cdot$ 10$^2$ mL$^{-1}$, i.e., $C^+/C^-$ = 10$^2$. The assay returns the concentration of the sample calculated with the use of independent random variables, thus, via scaling the estimate of concentration in the sample can be translated into the estimate of concentration in a reservoir. The required dynamic range is accomplished by partitioning the sample into 86 partitions with dilution ratios $d_i$ = $x^i$, where $x$ = 0.917. Using numerical algorithms presented in the description of the invention, the estimate $E(C_{sample})$ is calculated based on the conditional probability distribution $f(C_{sample}|\mu)$, where $\mu$ is a microstate of the system of partitions used to perform the analytical assay. In a preferred embodiment, the number of Epstein-Barr DNA copies is determined using a sample with a volume of 20 $\mu$L. The reaction mixture is placed in a system made of PDMS/polycarbonate and 86 droplets on demand with dilution ratios $d_i$ = $x^i$, where $x$ = 0.917, are generated. The system with droplets is placed in a thermocycler, the PCR reaction is carried out, and subsequently the signals from each droplet (microstate of the system) are read out.

For example, a 20 $\mu$L sample containing gene copies at a concentration of $C_{sample}$ = 1.5 $\cdot$ 10$^2$ mL$^{-1}$ was used. In five repeated experiments the following results were obtained: in the first trial $\mu_1$ = {*1,1,1,1,1,1, 1,1,1, 1,1,1,1, 1,1,1,1,1,1,1,1,1,1,1,1,1,1,1,1, 1,1,0,1,1,1,1 ,1,1,1,0,0,1,1,0,1,1,1,1,1,1,0,1,1,0,1,1,1,0,0,0,0,1,0,0,0,0, 0,0,0,1,0,0,1,0,1,0,1*}, in the second one $\mu_2$ = {*1,1,1,1,1,1,1,1,1,1,1, 1,1,1,1,1,1,1,1,1,1,1,1, 1,1,1,1,1,1,1,1,1, 1,1, 1,1,1,1,1,0,1,1,1,1, 1,1,0,0,1,1,0,1,1,1,1,1,1,0,1,1,1,0,1,0,0,0,0,1,0,0,1,0,0,0,1,1,0,0,0,1,0*}, in the third one $\mu_3$ = {*1,1,1,1,1,1,1,1,1,1,1,1,1,1,1,1,1,1,1,1,1,1,1,1,1,1,1,1,1,1,1,1,1,1,1,1,1,1,1,1,1,1,1,1,0,0,1, 1,1,0,1,1,1,1,1,1,1, 1,0, 1,1,0,1,0,1,1,0,1,0,0,1,0,0,0,0,1,0,0,0,0,0,0,0*}, in the fourth one $\mu_4$ = {*1,1,1,1,1,1,1,1, 1,1,1,1,1, 1,1,1, 1,1,1, 1,1, 1,1, 1,1,1,1,1,1,1,1,1,1,1,1,1,1,1,1,0,1,1,1,0,1,1,0,1,1, 1,1,0,1,1,1,1,0,1,1,1,1,0,0,0, 1,1,1,1,0,0,0,0, 0,0,1,0, 0,0, 0,0, 0,1,1*}, and in the fifth one $\mu_5$ = {*1,1,1,1,1,1,1,1,1,1,1, 1,1,1,1,1,1,1,1,1,1,1,1,1,1,1, 1,1,1,1,1,1, 1,1, 1,1, 1,1, 1,1, 1,1,1,0,0, 1,1,0,0,0,1,0,0,1,0,0,1,1,1,1,1,0,1,1,1,1,0,0,1,0,0,1,0,1,1,1,0,0,1,0,1,1*}, where the index numbers the repetition of the experiment. Using the statistical model obtained for a given distribution of dilutions, these values are recomputed into the estimates $c_i$ = $E_i(C_{sample})$ of the initial concentration of DNA copies in the sample $c_1$=$1.51 \cdot 10^2$, $c_2$=$1.72 \cdot 10^2$, $c_3$=$1.52 \cdot 10^2$, $c_4$=$1.61 \cdot 10^2$ and $c_4$=$1.84 \cdot 10^2$ [mL$^{-1}$].

**Executive Example 3**

In a preferred embodiment, a quantitation assay for a sample with a volume of 20 $\mu$L is designed so as to offer the estimate of the concentration of DNA molecules in the said sample with a relative standard deviation less than $\sigma_{max\_1}$=62% in the concentration range from $C_1^-$=10$^2$ [1/mL] to $C_1^+$=10$^4$ [1/mL], i.e., $(C_1^+/C_1^-)$=10$^2$, $\sigma_{max\_2}$=25% in the concentration range from $C_2^-$=10$^4$ [1/mL] to $C_2^+$=10$^6$ [1/mL], i.e., $(C_2^+/C_2^-)$=10$^2$, and $\sigma_{max\_3}$=62% in the concentration range from $C_3^- = 10^6$ [1/mL] to $C_3^+ = 10^8$ [1/mL], i.e., $(C_3^+/C_3^-) = 10^2$. The assay returns the concentration of the sample calculated with the use of independent random variables, thus, via scaling the estimate of concentration in the sample can be translated into the estimate of concentration in a reservoir. The required dynamic range is accomplished by partitioning the sample into consecutively 14 partitions with dilution ratios $d_i = x^i$, where $x = 0.5$, 86 partitions with $x = 0.917$, and 14 partitions with $x = 0.5$. Using numerical algorithms presented in the description of the invention, the estimate $E(C_{sample})$ is calculated based on the conditional probability distribution $p(C_{sample}|\mu)$, where $\mu$ is a microstate of the system of partitions used to perform the analytical assay (see also Figure 5b) and respective part of the detailed figure description).

In a preferred embodiment, the number of Epstein-Barr DNA copies is determined using a sample with a volume of 20 $\mu$L. The reaction mixture is placed in a system made of PDMS/polycarbonate and 14 droplets on demand with dilution ratios $d_i = x^i$, where $x = 0.5$, 86 droplets with $x = 0.917$, and 14 droplets with $x = 0.5$ are generated. The system with droplets is placed in a thermocycler, the PCR reaction is carried out, and subsequently the signals from each droplet (microstate of the system) are read out.

For example, a 20 $\mu$L sample containing gene copies at a concentration of $C_{sample}=5\cdot10^3$ mL$^{-1}$ was used. In five repeated experiments the following results were obtained: in the first trial $\mu_1=\{1,1,1,1,\ 1,0,1,\ 0,0,0,\ 0,0,0,\ 0,0,$ $0,0,0,0,0,0,0,0,0,0,0,0,1,0,0,0,0,0,0,0,0,0,0,0,0,0,0,0,0,0,0\ ,0,0,0,0,0,0,0,0,0,0,0,0,0,0,0,0,0,0,0,0,0,$ $0,0,0,0,0,0,0,0,0,0,0,0,0,0,0,0,0,0,0,0,\ 0,0,0,0,0,0,0,0,0,0,0,0,0,0,0\}$, in the second one $\mu2=\{1,1,1,1,1,1,1,0,0,0,0,$ $0,0,\ 0,0,0,0,0,0,0,0,0,0,0,0,0,1,0,0,0,0,0,0,0,0,0,0,0,0,0,0,0,0,0,0,0,0,0,0,0,0,0,0,0,0,0,0,0,0,0\ ,0,0,0,0,0,0,\ 0,0,$ $0,0,0,0,0,0,0,0,0,0,0,0,0,0,0,0,0,0,0,0,0,0,0,0,0,0,0,0,0,0,0,0,0,0,0,0,0\}$, in the third one $\mu_3=\{1,1,1,\ 1,1,0,0,\ 1,0,$ $0,0,0,0,1,0,0,0,0,0,0,0,0,0,0,0,0,0,0,0,0,0,0,0,\ 0,0,0,0,0,0,0,0,0,0,0,0,0,0,0,0,0,0,0,0,0,0,0,0,0,0,0,0,0,0,0,\ 0,0,$ $0,0,0,0,0,0,0,0,0,0,0,0,0,0,0\ ,0,0,0,0,0,0,0,0,0,0,0,0,0,0,0,0,0,0,0,0,0,0,0\}$, in the fourth one $\mu_4=\{1,1,1,1,0,$ $1,1,1,1,\ 0,0,0,0,0,1,0,0,0,0,1,0,0,0,0,0,0,0,0,0,0,0,0,0,0,1,0,0,0,0,0,0,0,0,0,0,0,0,0,0,0,0,0,0,0,0,0\ ,0,\ 0,0,\ 0,0,0,\ 0,$ $0,0,0,0,0,0,0,0,0,0,0,0,0,0,1,0,0,0,0,0,0,0,0,0,0,0,0,0,0,0,0,0,\ 0,0,0,0\}$, in the fifth one $\mu_5=\{1,\ 1,1,$ $1,1,0,1,0,0,0,0,0,0,0,0,0,0,0,0,0,0,0,0,0,0,0,0,0,\ 0,0,0,0,0,0,0,0,0,0,0,0,0,0,0,0,0,0,0,\ 0,1,0,0,0,\ 0,0,$ $0,0,0,0,0,0,0,0,0,0,0,0,0\ ,0,0,0,0,0,0,0,0,0,0,0,0,0,0,0,0,0,0,0,0,0,0,0,0,0,0,0\}$, where the index numbers the repetition of the experiment. Using the statistical model obtained for a given distribution of dilutions, these values are recomputed into the estimates $c_l = E_l(C_{sample})$ of the initial concentration of DNA copies in the sample $c_1=2.30\cdot10^3$, $c_2=6.12\cdot10^3$, $c_3=2.14\cdot10^3$, $c_4=2.90\cdot10^3$ and $c_5=2.30\cdot10^3$ [mL$^{-1}$].

For example, a 20 $\mu$L sample containing gene copies at a concentration of $C_{sample}=1.5\cdot10^5$ mL$^{-1}$ was used. In five repeated experiments the following results were obtained: in the first trial $\mu_1=\{1,1,1,1,\ 1,1,1,\ 1,1,1,1,\ 1,1,1,1,$ $1,1,1,1,1,1,1,1,1,1,1,1,1,1,1,1,1,1,1,1,1,1,1,1\ ,1,1,1,1,1,1,1,1,1,1,1,1,1,1,1,1,1,1,0,1,1,1,1,1,1,0,1,0,1,$ $0,1,0,1,0,1,0,1,0,1,0,0,1,0,0,0,0,0,0,\ 1,1,0,0,0,0,0,0,0,0,0,0,0,0,0\}$, in the second one $\mu_2=\{1,1,1,\ 1,1,1,1,\ 1,1,1,$ $1,1,1,1,1,1,1,1,1,\ 1,1,1,1,1,1,1,1,1,1,1,1,1,1,1,1,1,1,1,1,1,1,1,1,1,1,1,1,1,1,1,1,1,0,1,\ 1,1,1,1,1,1,1,1,\ 1,1,$ $1\ ,1,1,1,1,1,1,0,0,1,0,1,0,0,1,0,1,1,0,0,1,1,0,0,0,1,0,0,1,0,1,1,0,0,0,0,0,0,0,0\}$, in the third one $\mu_3=\{1,1,1,\ 1,1,$ $1,1,1,1,1,1,1,1,1,1,1,1,1,1,1,1,1,1,1,1,1,1,1,1,1,1,1,1,1,1,1,1,\ 1,1,1,1,1,1,1,1,1,1,1,1,1,1,1,1,1,\ 1,1,0,1,1,1,1,$ $1,1,1,1,0,1,1,1,1,1,1,0,1,1,1,0,1,0,1,0,1,0,1,0,0,0\ ,1,0,0,1,0,0,0,0,0,0,0,0,0,0,0,0,0\}$, in the fourth one $\mu_4=\{1,\ 1,1,$ $1,1,1,1,1,1,1,\ 1,1,1,1,1,1,1,1,1,1,1,1,1,1,1,1,1,1,1,1,1,1,1,1,1,1,\ 1,1,1,1,1,1,1,1,1,1,1,1,1,1,1,1,1,1,1,1,\ 1,\ 1\ ,0,$ $1,1,1,1,1,1,1,1,0,1,0,0,0,1,1,0,1,0,0,0,0,1,0,0,1,0,0,1,0,1,0,0,1,0,1,0,0,0,0,0,0,0,0,0,\ 0\}$, in the fifth one $\mu_5=\{1,1,1,1,1,1,1,1,1,1,1,1,1,1,1,1,1,1,1,1,1,1,1,1,1,1,1,\ 1,1,1,1,1,1,1,1,1,1,1,1,1,1,1,1,1,1,1,1,1,1,1,1,1,1,1,1,\ 1,1,$ $1,1,1,1,1,1,1,1,1,0,1,1,1,0,1,1,1,1,0\ ,0,1,0,1,1,0,0,0,0,0,0,1,0,0,0,0,1,0,0,0,0,0,0,0,0,0,0,0,0\}$, where the index numbers the repetition of the experiment. Using the statistical model obtained for a given distribution of dilutions, these values are recomputed into the estimates $c_l = E_l(C_{sample})$ of the initial concentration of DNA copies in the sample $c_1=1.45\cdot10^5$, $c_2=1.84\cdot10^5$, $c_3=1.74\cdot10^5$, $c_4=1.43\cdot10^5$ and $c_5=1.52\cdot10^5$ [mL$^{-1}$].

For example, a 20 $\mu$L sample containing gene copies at a concentration of $C_{sample}=5\cdot10^7$ mL$^{-1}$ was used. In five repeated experiments the following results were obtained: in the first trial $\mu_1=\{1,1,1,\ 1,1,1,1,1,1,\ 1,1,\ 1,1,$ $1,1,1,1,1,1,1,1,1,1,1,1,1,1,1,1,1,1,1,1,1,1,1,1,1\ ,1,1,1,1,1,1,1,1,1,1,1,1,1,1,1,1,1,1,1,1,1,1,1,1,1,1,1,1,1,1,$ $1,1,1,1,1,1,1,1,1,1,1,1,1,1,1,1,1,1,1,1,\ 1,1,1,1,1,1,0,1,0,0,0,0,0,0\}$, in the second one $\mu_2=\{1,1,\ 1,1,1,1,\ 1,1,$ $1,1,1,1,1,1,1,1,1,1,1,\ 1,1,1,1,1,1,1,1,1,1,1,1,1,1,1,1,1,1,1,1,1,1,1,1,1,1,1,1,1,1,1,1,1,1,1,1,1,1,1,1,\ 1,1,1,1,$ $1,1,1\ ,1,1,1,1,1,1,1,1,1,1,1,1,1,1,1,1,1,1,1,1,1,1,1,1,1,0,1,0,0,0,0,0,0\}$, in the third one $\mu_3=\{1,1,\ 1,1,1,1,$ $1,1,1,1,1,1,1,1,1,1,1,1,1,1,1,1,1,1,1,1,1,1,1,1,1,1,1,1,1,1,1,1,1,1,1,1,1,1,1,1,1,1\ ,1,1,1,1,1,1,1,1,1,1,1,1,1,$ $1,1,1,1,1,1,1,1,1,1,1,1,1,1,1,1,1,1,1,1,1,1,1,1,1,\ 1,1,1,1,1,1,0,0,0,0,0,0,0,0\}$, in the fourth one $\mu_4=\{1,1,1,$ $1,1,1,1,1,1,1,1,1,1,1,1,1,1,1,1,1,1,\ 1,1,1,1,1,1,1,1,1,1,1,1,1,1,1,1,1,1,1,1,1,1,1,1,1,1,1,1,1,1,1,1,1,1,1,1,1,$ $1,1,1,1,1,1,1\ ,1,1,1,1,1,1,1,1,1,1,1,1,1,1,1,1,1,1,1,0,0,0,0,0,0,0,0,0\}$, in the fifth one $\mu_5=\{1,1,$ $1,1,1,1,1,1,1,1,1,1,1,1,1,1,1,1,1,1,1,1,1,1,1,1,1,1,1,1,1,1,1,1,1,1,1,1,1,1,1,1,1,1,1\ ,1,1,1,1,1,1,1,1,1,1,1,1,1,1,$ $1,1,1,1,1,1,1,1,1,1,1,1,1,1,1,1,1,1,1,1,1,1,1,1,1,1,1,1,\ 1,1,1,1,1,1,1,0,0,0,0,0,0,0\}$, where the index numbers the repetition of the experiment. Using the statistical model obtained for a given distribution of dilutions, these values are recomputed into the estimates $c_l = E_l(C_{sample})$ of the initial concentrations of DNA copies in the sample $c_1=2.84\cdot10^7$,

$c_2=3.08 \cdot 10^7$, $c_3=3.42 \cdot 10^7$, $c_4=3.81 \cdot 10^7$ and $c_5=3.31 \cdot 10^7$ [mL$^{-1}$].

## Executive Example 4

In a preferred embodiment, an inventive quantitation assay for a sample with a volume of 20 $\mu$L is designed so as to offer the estimate of the concentration of DNA molecules in the said sample with a relative standard deviation less than $\sigma_{max}$ =15% in the concentration range from $C^-$=50 [1/mL] to $C^{\mp}$= 0.5·10$^7$ [1/mL], i.e., $(C^+/C^-)$ = 10$^5$. The required dynamic range is accomplished by partitioning the sample into 474 partitions with dilution degrees $\frac{C_n}{C_{sample}} = d_n = x^n$, where $x$=0.9886. Using numerical algorithms presented in the description of the invention one computes a corrected estimate of the initial concentration of molecules in the sample $c_{corr} = c \cdot f_{corr}(c)$, where $c = E(C_{sample})$ is the estimate of concentration of molecules in the sample, computed using the conditional probability distribution $P(C_{sample}|K)$, where $K$ is a weighted sum of signals from individual partitions counted with weights $w_n = 1/d_n$.

So prepared statistical tools ($f_{corr}(c)$ and $c(K)$ functions) can be used to determine the initially unknown concentration of target DNA molecules in the sample.

In a preferred embodiment, the number of Epstein-Barr DNA copies is determined using a sample with a volume of 20 $\mu$L. The reaction mixture is placed in a system made of PDMS/polycarbonate and 474 on-demand droplets with dilution degrees $\frac{C_n}{C_{sample}} = d_n = x^n$, where x =0.9886, are generated. The system with droplets is placed in a thermocycler, the PCR reaction is carried out, followed by counting the signals from each droplet. The signals from droplets displaying high fluorescence level are summed up using weights $w_n = 1/d_n$.

For example, a 20 $\mu$L sample containing gene copies at a concentration of $C_{sample}$=5·10$^2$ mL$^{-1}$ was used. In five repeated experiments the following results were obtained: in the first trial $K_1=0.178$, in the second one $K_2=0.404$, in the third one $K_3=0.292$, in the fourth one $K_4=0.155$ and in the fifth one $K_5=0.212$, where the index numbers the repetition of the experiment. Using the statistical model obtained for a given distribution of dilutions, these values are recomputed into the estimates of the initial concentration of DNA copies in the sample $c_1=8.53 \cdot 10^2$, $c_2=1.56 \cdot 10^3$, $c_3=1.27 \cdot 10^3$, $c_4=7.41 \cdot 10^2$ and $c_5=1.02 \cdot 10^3$ [mL$^{-1}$]. After taking the correction into account $c_{corr}(c) = c(K) \cdot f_{corr}(c)$, the following estimates are obtained: $c_{corr1}=7.84 \cdot 10^2$, $c_{corr2}=1.43 \cdot 10^3$, $c_{corr3}=1.17 \cdot 10^3$, $c_{corr4}=6.80 \cdot 10^2$ and $c_{corr5}=9.32 \cdot 10^2$ [mL$^{-1}$].

For example, a 20 $\mu$L sample containing gene copies at a concentration of $C_{sample}$=5·10$^5$ mL$^{-1}$ was used. In five repeated experiments the following results were obtained: in the first trial $K_1=54.60$, in the second one $K_2=59.62$, in the third one $K_3=58.65$, in the fourth one $K_4=62.03$ and in the fifth one $K_5=55.55$, where the index numbers the repetition of the experiment. Using the statistical model obtained for a given distribution of dilutions, these values are recomputed into the estimates of the initial concentration of DNA copies in the sample $c_1=3.88 \cdot 10^5$, $c_2=4.57 \cdot 10^5$, $c_3=4.38 \cdot 10^5$, $c_4=4.89 \cdot 10^5$ and $c_5=3.94 \cdot 10^5$ [mL$^{-1}$]. After taking the correction into account $c_{corr}(c) = c(K) \cdot f_{corr}(c)$, the following estimates are obtained: $c_{corr1}=3.99 \cdot 10^5$, $c_{corr2}=4.79 \cdot 10^5$, $c_{corr3}=4.57 \cdot 10^5$, $c_{corr4}=5.17 \cdot 10^5$ and $c_{corr5}=4.05 \cdot 10^5$ [mL$^{-1}$].

For example, a 20 $\mu$L sample containing gene copies at a concentration of $C_{samples}$=10$^6$ mL$^{-1}$ was used. In five repeated experiments the following results were obtained: in the first trial $K_1=74.99$, in the second one $K_2=78.99$, in the third one $K_3=77.16$, in the fourth one $K_4=75.85$ and in the fifth one $K_5=79.22$, where the index numbers the repetition of the experiment. Using the statistical model obtained for a given distribution of dilutions, these values are recomputed into the estimates of the initial concentration of DNA copies in the sample $c_1=7.48 \cdot 10^5$, $c_2=8.50 \cdot 10^5$, $c_3=8.06 \cdot 10^5$, $c_4=7.80 \cdot 10^5$ and $c_5=8.55 \cdot 10^5$ [mL$^{-1}$]. After taking the correction into account $c_{corr}(c) = c(K) \cdot f_{corr}(c)$, the following estimates are obtained: $c_{corr1}=8.62 \cdot 10^5$, $c_{corr2}=1.01 \cdot 10^6$, $c_{corr3}=9.47 \cdot 10^5$, $c_{corr4}=9.09 \cdot 10^5$ and $c_{corr5}=1.02 \cdot 10^6$ [mL$^{-1}$].

## Executive Example 5

In a preferred embodiment of the invention, an assay is designed to offer the estimate of the concentration of DNA particles in a sample with a relative standard deviation less than $\sigma_{max1}$ = 20% in the concentration range from $C_1^- = 1$ $\mu$L$^{-1}$ to $C_1^+ = 10^3$ $\mu$L$^{-1}$, i.e., $C_1^+/C_1^- = 10^3$, $\sigma_{max2}$ = 10% in the concentration range from $C_2^- = 10^3$ $\mu$L$^{-1}$ to $C_2^+ = 10^6$ $\mu$L$^{-1}$, i.e., $C_2^+/C_2^- = 10^3$, and $\sigma_{max3}$ = 20% in the concentration range from $C_3^- = 10^6$ $\mu$L$^{-1}$ to $C_3^+ = 10^9$ $\mu$L$^{-1}$, i.e., $C_3^+/C_3^- = 10^3$. The required dynamic range is accomplished by partitioning the sample into a geometric series of $N$ = 38 libraries with dilution ratios $C_i/C_S = d_i = x^i$, where x = 0.5 and the first term $d_0 v_0$ = 11.1 $\mu$L, whereas the libraries with indexes from 0 to 9 have cardinalities $N'_1$ = 10, the libraries with indexes from 10 to 27 have cardinalities $N'_2$ = 40, and the libraries with indexes from 28 to 37 have cardinalities $N'_3$ = 10. Using numerical algorithms presented in the description of the invention, the estimate $E(C_S)$ is calculated based on the conditional probability distribution $f(C_S|\mu)$, where $\mu$ is a microstate of the system of compartments used to perform the analytical assay.

The reaction mixture is placed in a system made of PDMS/polycarbonate and N = 38 libraries with dilution ratios $C_i/C_S = d_i = x^i$, where $x = 0.5$, whereas the libraries with indexes from 0 to 9 have cardinalities $N'_1 = 10$, the libraries with indexes from 10 to 27 have cardinalities $N'_2 = 40$, and the libraries with indexes from 28 to 37 have cardinalities $N'_3 = 10$. The system with droplets is placed in a thermocycler, the PCR reaction is carried out, and subsequently the signals from each droplet (microstate of the system) are read out, producing an N-dimensional vector $k$ with coordinates $k_i$ equal to the number of droplets belonging to the $i$-th library, and yielding positive signals.

For example, a sample containing gene copies at a concentration of $C_S = 10^2\,\text{mL}^{-1}$. In five repeated experiments the following results were obtained: in the first $k_1$={10, 10, 10, 10, 10, 10, 10, 10, 10, 9, 26, 18, 7, 7, 2, 2, 1, 0, 1, 0, 0, 0, 0, 0, 0, 0, 0, 0, 0, 0, 0, 0, 0, 0, 0, 0, 0, 0}, $k_2$={10, 10, 10, 10, 10, 10, 10, 10, 10, 9, 25, 18, 7, 4, 2, 2, 1, 0, 0, 0, 0, 0, 0, 0, 0, 0, 0, 0, 0, 0, 0, 0, 0, 0, 0, 0, 0, 0}, $k_3$={10, 10, 10, 10, 10, 10, 10, 10, 10, 8, 26, 17, 11, 4, 5, 3, 0, 0, 0, 0, 0, 0, 0, 0, 0, 0, 0, 0, 0, 0, 0, 0, 0, 0, 0, 0, 0, 0}, $k_4$={10, 10, 10, 10, 10, 10, 10, 10, 10, 10, 28, 16, 11, 8, 2, 2, 0, 0, 0, 0, 0, 0, 0, 0, 0, 0, 0, 0, 0, 0, 0, 0, 0, 0, 0, 0, 0, 0}, $k_5$={10, 10, 10, 10, 10, 10, 10, 10, 10, 10, 25, 14, 11, 8, 3, 0, 1, 0, 0, 0, 0, 0, 0, 0, 0, 0, 0, 0, 0, 0, 0, 0, 0, 0, 0, 0, 0, 0}, where the index numbers the repetition of the experiment. Using the statistical model obtained for a given distribution of dilutions, these values are recomputed into the estimates of the initial concentration of DNA copies in the sample $c_1 = 104 \pm 12$, $c_2 = 96 \pm 12$, $c_3 = 104 \pm 12$, $c_4 = 115 \pm 14$, and $c_5 = 103 \pm 12\,\mu\text{L}^{-1}$.

For example, a sample containing gene copies at a concentration of $C_S = 10^5\,\text{mL}^{-1}$. In five repeated experiments the following results were obtained: $k_1$={10, 10, 10, 10, 10, 10, 10, 10, 10, 10, 40, 40, 40, 40, 40, 40, 40, 40, 40, 36, 23, 14, 11, 4, 1, 1, 0, 1, 0, 0, 0, 0, 0, 0, 0, 0, 0, 0}, $k_2$={10, 10, 10, 10, 10, 10, 10, 10, 10, 10, 40, 40, 40, 40, 40, 40, 40, 40, 39, 34, 27, 16, 9, 4, 2, 3, 2, 0, 0, 0, 0, 0, 0, 0, 0, 0, 0, 0}, $k_3$={10, 10, 10, 10, 10, 10, 10, 10, 10, 10, 40, 40, 40, 40, 40, 40, 40, 40, 40, 34, 24, 20, 8, 3, 3, 1, 0, 1, 0, 0, 0, 0, 0, 0, 0, 0, 0, 0}, $k_4$={10, 10, 10, 10, 10, 10, 10, 10, 10, 10, 40, 40, 40, 40, 40, 40, 40, 40, 40, 39, 36, 27, 15, 10, 10, 3, 0, 0, 1, 1, 0, 0, 0, 0, 0, 0, 0, 0}, $k_5$={10, 10, 10, 10, 10, 10, 10, 10, 10, 10, 40, 40, 40, 40, 40, 40, 40, 40, 39, 37, 30, 19, 10, 4, 3, 3, 3, 2, 0, 0, 0, 0, 0, 0, 0, 0, 0, 0}, where the index numbers the repetition of the experiment. Using the statistical model obtained for a given distribution of dilutions, these values are recomputed into the estimates of the initial concentration of DNA copies in the sample $c_1 = (96 \pm 10) \cdot 10^3$, $c_2 = (98 \pm 10) \cdot 10^3$, $c_3 = (99 \pm 10) \cdot 10^3$, $c_4 = (108 \pm 11) \cdot 10^3$, and $c_5 = (123 \pm 12) \cdot 10^3\,\mu\text{L}^{-1}$.

For example, a sample containing gene copies at a concentration of $C_S = 10^8\,\text{mL}^{-1}$. In five repeated experiments the following results were obtained: $k_1$={10, 10, 10, 10, 10, 10, 10, 10, 10, 10, 40, 40, 40, 40, 40, 40, 40, 40, 40, 40, 40, 40, 40, 40, 40, 40, 40, 10, 7, 8, 2, 1, 2, 1, 0, 0, 0}, $k_2$={10, 10, 10, 10, 10, 10, 10, 10, 10, 10, 40, 40, 40, 40, 40, 40, 40, 40, 40, 40, 40, 40, 40, 40, 40, 40, 40, 10, 8, 7, 5, 2, 0, 1, 0, 0, 0}, $k_3$={10, 10, 10, 10, 10, 10, 10, 10, 10, 10, 40, 40, 40, 40, 40, 40, 40, 40, 40, 40, 40, 40, 40, 40, 40, 40, 40, 10, 9, 6, 1, 3, 2, 0, 0, 0, 0}, $k_4$={10, 10, 10, 10, 10, 10, 10, 10, 10, 10, 40, 40, 40, 40, 40, 40, 40, 40, 40, 40, 40, 40, 40, 40, 40, 40, 40, 9, 9, 6, 5, 2, 1, 1, 0, 0, 0}, $k_5$={10, 10, 10, 10, 10, 10, 10, 10, 10, 10, 40, 40, 40, 40, 40, 40, 40, 40, 40, 40, 40, 40, 40, 40, 40, 40, 39, 10, 10, 7, 4, 4, 0, 1, 2, 1, 0}, where the index numbers the repetition of the experiment. Using the statistical model obtained for a given distribution of dilutions, these values are recomputed into the estimates of the initial concentration of DNA copies in the sample $c_1 = (89 \pm 17) \cdot 10^6$, $c_2 = (103 \pm 21) \cdot 10^6$, $c_3 = (94 \pm 19) \cdot 10^6$, $c_4 = (94 \pm 18) \cdot 10^6$, and $c_5 = (104 \pm 19) \cdot 10^6\,\mu\text{L}^{-1}$.

**Claims**

1. Method for determining an estimate of a concentration of particles $E(C)$, wherein a sample of predetermined volume is divided into a number $(N)$ of compartments, at least part of the particles that are present in any of the $(N)$ compartments provide a measurable signal and the estimated concentration of particles $E(C)$ is a function of measured signals, **characterized in that** the method comprises or consists of

   a) determining the number $(N)$ of separate compartments, wherein the number $(N)$ is smaller or equal to the value of the function

$$N_{MAX} = A \cdot \ln\left(\frac{C^+}{C^-}\right)\Big/\sigma_{MAX}^2$$

   wherein $(A)$ represents a real number being the integer 6,
   wherein $(C^+)$ represents a predetermined upper limit of the interval of concentration $(C)$ to be estimated by the method,
   wherein $(C^-)$ represents a predetermined lower limit of the interval of concentration $(C)$ to be estimated by

the method,
wherein ($\sigma_{MAX}$) represents a predetermined maximum allowable relative standard deviation of the estimate of concentration $E(C)$ of particles, wherein $C^- < C < C^+$, and

    b) determining a modulation factor ($z_i$), wherein ($z_i$) is a function of volumes ($v_i$) and dilution factors ($d_i$) of the sample in at least part of or all of the *(N)* compartments and partitioning the sample into the *(N)* compartments, wherein at least part of or all of the two or more of the *(N)* compartments comprise or consist of different sample volumes ($v_i$) and/or different dilution factors ($d_i$) of the sample,
wherein (*i*) represents an index number of the *(N)* compartments represented by the integers 0 to *N* - 1, and
wherein ($v_i$) represents the volume and ($d_i$) represents the dilution factor of the sample in the compartment *(i)*.

2. Method according to claim 1, wherein the modulation factor ($z_i$) is determinable by the function $z_i = (v_i d_i)/(v_0 d_0)$ or analogue thereof, wherein the analogue conveys information about pairwise differences in the estimated number of particles in compartments,
wherein (*i*), ($v_i$) and ($d_i$) is defined as in claim 1 and
wherein ($v_0$) represents the volume and ($d_0$) represents the dilution factor of the sample in a reference compartment, wherein the reference compartment is different to compartment (*i*), preferably wherein the reference compartment represents the first compartment in the series of (*N*) compartments.

3. Method according to claim 1 or 2, wherein in step b) 1%, preferably 5 %, more preferably 25 %, even more preferably 50 %, even more preferably 75% and most preferably 100 % of the (*N*) compartments differ from each other by the value of the modulation factor ($z_i$).

4. Method according to any one of claims 1 to 3, wherein in step b) the value of the modulation factor ($z_i$) is determined based on a well defined power sequence, an exponential sequence, a polynomial sequence or a geometric sequence or based on a distribution in the set of compartments, preferably predetermined by Gaussian distribution or a combination thereof.

5. Method according to claim 4, wherein in step b) the sample is partitioned into (*N*) compartments with such volumes ($v_i$) and dilution factors ($d_i$) as to fulfil the condition:

$$(v_{i+1}d_{i+1})/(v_i d_i) = z_{i+1}/z_i = x$$

wherein $x > 0$ and $x \neq 1$, preferably wherein the value of (*x*) is represented by about 0.1, 0.5, 0.8, 0.9, 0.91, 0.92, 0.93, 0.94, 0.95, 0.96, 0.97, 0.98, 0.99, 1.01, 1.02, 1.03, 1.04, 1.05, 1.06, 1.07, 1.08, 1.09, 1.1, 1.2, 1.3, 1.4, 1.5, 2, or 10 and
wherein preferably quotient $x = (v_{i+1}d_{i+1})/(v_i d_i)$ is determinable by the function:

$$x = 1 - \left(\frac{\sigma_{max}}{0.8955}\right)^{1/0.513}$$

the number (N) of compartments is set to an integer not smaller than the value determinable by the function:

$$N = \Delta N + \log_x\left(\frac{C^-}{C^+}\right)$$

Wherein ($\Delta N$) is an integer not smaller than the value determinable by the function:

$$\Delta N = 4.5637(1 - x)^{-0.798}$$

And wherein the value for $d_o v_o$ is determinable by the function

$$d_o v_o = ln2 \cdot x^{-\Delta N/2} \left(\frac{1}{C^-}\right)$$

with $(C^+)$, $(C^-)$, $(\sigma_{MAX})$, $(d_o)$ and $(v_o)$ as defined in claims 1 and 2.

6. Method according to any one of claims 1 to 5, wherein in step b) the sample is partitioned into (N) compartments with such volumes $(v_i)$ as to fulfil the condition: $v_i = A \cdot exp^{(B \cdot \frac{i}{N})}$ = wherein (A) and (B) represent independently of each other arbitrary real numbers, and wherein preferably $A = 1/C^+$ and/or $B = [1.95 \blacksquare ln\{(C+/C-)/N\}]^{0.856}$, wherein the sample is preferably divided into (N) compartments

$$N = 2 \cdot \sigma_{MAX}^{-1.9} \cdot \left(\frac{C^+}{C^-}\right)^{0.25} ,$$

preferably

$$N = 2 \cdot \sigma_{MAX}^{-1.94} \cdot \left(\frac{C^+}{C^-}\right)^{0.22} .$$

with $(C^+)$, $(C^-)$, and $(\sigma_{MAX})$ as defined in claim 1.

7. Method according to any one of claims 1 to 6, wherein the method comprises or consists of the following steps:

   c) optionally amplifying the particles of the compartments with one or more suitable reagents and optionally one or more diluents for obtaining a measurable signal indicating the presence of a predetermined threshold number of particles, preferably one, two, three or more particles in a compartment,
   d) measuring the signals in each of the (N) compartments and assigning to at least part, preferably all compartments a value $(k_i)$, wherein *(i)* represents the index number of the compartments represented by integers 0 to $N - 1$ and compartment *(i)* is assigned a first value $(k_i)$, if the compartment *(i)* comprises or consists of the predetermined threshold number of particles or more, and compartment *(i)* is assigned a second value $(k_i)$, if the compartment $(k_i)$ comprises or consists of less than the threshold number of particles indicating the first value and
   e) determining the estimated concentration of particles $E(C)$, wherein the estimated concentration of particles $E(C)$ is determinable by a function of

      i. a *sum K* of the first and/or second values $(k_i)$, wherein the function preferably is corrected using a calibration correction function, and/or
      ii. a *sum K* of weighted first and/or second values $(w_i k_i)$, wherein at least part of the values $(k_i)$, preferably each value $(k_i)$ is modulated with a weight value $(w_i)$, wherein *(i)* represents the index number of the compartments represented by the integers 0 to $N - 1,$ and wherein the weight value $(w_i)$ preferably is inversely proportional to the degree of volume and/or dilution of the number *(i)* of the compartments, and wherein the function is preferably corrected using a calibration correction function, and/or
      iii. a number, vector, matrix, tensor of any order or any other unambiguous representation of a *microstate* $\mu$, wherein the number, vector, matrix, tensor of any order or any other representation comprises or consists of information reflecting at least part of the values $(k_i)$ measured in step e), preferably comprises or consists of a *vector* $k \equiv \{k_i\}$, representing the *microstate* $\mu$, wherein the vector $(k)$ comprises or consists of at least part, preferably all of the values $(k_i)$ measured in step e).

8. Method according to any one of claims 1 to 7, wherein in step b) the sample is partitioned into a number $(N_{LIB})$ of two or more separate sets of compartments (libraries), each library set is indexed with $(j)$ and contains $N'_j > 0$ compartments, each compartment in the same library comprising or consisting of a part of the sample volume with the same value of a modulation factor $(z_j)$, wherein $z_j$ is a function of volumes $(v_j)$ and dilution factors $(d_j)$ of the sample in the library set $(j)$, and wherein the $N_{LIB}$ separate library *sets j* are distinguishable from each other by different values of the modulation factor $(z_j)$, wherein *j* represents the index number of the number $(N_{LIB})$ library sets represented by the integers 0 to $N_{LIB}$ - 1, and

wherein preferably the modulation factor ($z_j$) is determined by the function of $Z_j = (v_j d_j)/(v_o d_o)$ or an analogue thereof, wherein the analogue conveys information about pairwise differences in the estimated number of particles in compartments

wherein ($v_j$) represents the volume and ($d_j$) represents the dilution factor of the sample in the compartments of library set ($j$) and

wherein ($v_0$) represents the volume and ($d_0$) represents the dilution factor of the sample in the compartments of a selected reference library set ($j$).

9. Method according to claim 8, wherein in step b) at least part, preferably all of the number ($N_{LIB}$) library sets ($j$) comprise or consist of $Nj' = N'$ compartments for each library set ($j$), and fulfil the condition:

$$\left(v_{j+1}d_{j+1}\right)/\left(v_j d_j\right) = z_{j+1}/z_j = x$$

wherein $x > 0$ and $x \neq 1$ and wherein preferably the function:

$$x_{max} = 1 - \left(\frac{\sigma_{max}}{0.8955}\right)^{1/0.513}$$

represents the maximum preferred value of the quotient ($x$), wherein the quotient ($x$) can be tuned to the technical preferences for execution of the method, and the number $N'j$ of compartments in each library set ($j$) is set to an integer not smaller than the value determinable by the function:

$$N_j' = \left(\frac{0.8955}{\sigma_{max}}\right)^2 (1-x)^{1.026}$$

and the number ($N_{LIB}$) of the separate library sets ($j$) is set to an integer not smaller than the value determinable by the function

$$N_{LIB} = log_x\left(\frac{C^-}{C^+}\right) + \Delta N_{LIB}$$

wherein

$$\Delta N_{LIB} = 4.5637(1-x)^{-0.798}$$

and wherein $d_0^j v_0^j$ is determinable by the function

$$d_0\, v_0 = ln2 \cdot x^{-\Delta N/2} \cdot \frac{1}{C^-}.$$

with ($C^+$), ($C^-$) and ($\sigma_{QMAX}$), as defined in claim 1 and ($d_0$) and ($v_0$) as defined in claim 13.

10. Method according to claim 8 or 9, wherein at least part or preferably each of the number ($N_{LIB}$) library sets ($j$) is assigned a value ($k_i^j$) being a function of at least part of, preferably all the values ($k_i$) assigned to each of the compartments contained in library set ($j$) in step d), and subsequently mapping the values ($k_i^j$) into a number, vector, matrix, tensor of any order or any other unambiguous representation of a *microstate* $\mu$, wherein the number, vector, matrix, tensor of any order or any other representation comprises or consists of information reflecting at least part of the values ($k_i^j$), preferably comprises or consists of a vector $k_{LIB} \equiv \{k_i^j\}$, representing the *microstate*

μ, wherein the vector ($k_{LIB}$), wherein the vector ($k_{LIB}$) comprises or consists of at least part, preferably all of the values ($k_i^j$), wherein preferably the value ($k_i^j$) comprises or consists of the sum of the values ($k_i$) assigned to each of the compartments contained in library set (*j*) in step d).

11. Method according to any one of claims 1 to 10, wherein the concentration of two or more different particles and/or two or more different parts of essentially the same particles are determined, wherein at least part, preferably all of the different particles and/or different parts of essentially the same particles that are present in any of the (*N*) compartments provide, preferably are amplified to provide two or more distinguishable measurable signals, wherein the two or more distinguishable signals in any of the (*N*) compartments are measured and wherein to each of the (*N*) compartments two or more different values are respectively assigned, wherein the different values independently from each other indicate that a threshold of a number of same particles and/or same parts of essentially the same particles is present in the compartment or not.

12. Apparatus for use in determining a concentration of particles in accordance with any one of claims 1 to 11 **characterized in that** the apparatus is configured to

a) determining a number (*N*) of separate compartments, wherein the number (N) is smaller or equal to the value of the function

$$N_{MAX} = A \cdot \ln\left(\frac{C^+}{C^-}\right) \Big/ \sigma_{MAX}^2$$

wherein (*A*) represents a real number being the integer 6, Wherein (*C+*) represents a predetermined upper limit of the interval of concentration (*C*) to be estimated by the method, Wherein (*C-*) represents a predetermined lower limit of the interval of concentration (*C*) to be estimated by the method, Wherein ($\sigma_{MAX}$) represents a predetermined maximum allowable relative standard deviation of the estimate of concentration *E*(*C*) of particles, wherein $C^- < C < C^+$, and

b) determining a modulation factor ($z_i$), wherein ($z_i$) is a function of volumes ($v_i$) and dilution factors ($d_i$) of the sample in at least part of or all of the (*N*) compartments so that at least part of or all of the two or more of the (*N*) compartments comprise or consist of different sample volumes ($v_i$) and/or different dilution factors ($d_i$) of the sample, wherein (*i*) represents an index number of the (N) compartments represented by the integers 0 to *N* - 1, and wherein ($v_i$) represents the volume and ($d_i$) represents the dilution factor of the sample of predetermined volume in the compartment (*i*).

13. Use of the method according to any one of claims 1 to 11 or the apparatus according to claim 12 for

a) reducing the total number (N) of compartments comprising the predetermined sample volume and/or

b) reducing the total volume of a mixture comprising or consisting of sample, one or more reagents suitable for amplifying at least part of the particles to a measurable signal and optionally one or more diluents in all of the (*N*) compartments and/or

c) predetermining the volume, preferably the minimum or maximum volume of the mixture comprising or consisting of sample, one or more reagents suitable for amplifying at least part of the particles to a measurable signal and optionally one or more diluents in each of the (*N*) compartments and/or

d) predetermining the modulation factor ($z_i$), preferably the minimum or the maximum suitable modulation factor ($z_i$) for partitioning the predetermined sample volume into the (*N*) compartments.

14. Use of a sample holder in a method for determining a concentration of particles in accordance with any one of claims 1 to 11, **characterized in that** the sample holder is configured to,

a) comprising or consisting of a predetermined number (*N*) of compartments wherein the number (*N*) is smaller or equal to the value of the function

$$N_{MAX} = A \cdot \ln\left(\frac{C^+}{C^-}\right) \Big/ \sigma_{MAX}^2$$

wherein (*A*) represents a real number being the integer 6, wherein (*C*$^+$) represents a predetermined upper limit of the interval of concentration (*C*) to be estimated by the method, wherein (*C*$^-$) represents a predetermined lower limit of the interval of concentration (*C*) to be estimated by the method, herein ($\sigma_{MAX}$) represents a predetermined maximum allowable relative standard deviation of the estimate of concentration E(C) of particles, wherein $C^- < C < C^+$, and

b) wherein the (*N*) compartments are configured to comprising the predetermined sample volume with a pre-determined modulation factor (*z*$_i$), wherein (*z*$_i$) is a function of volumes (*v*$_i$) and dilution factors (*d*$_i$) of the sample in at least part of or all of the (*N*) compartments so that at least part of or all of the two or more of the (*N*) compartments can comprise or consist of different sample volumes (*v*$_i$) and/or different dilution factors (*d*$_i$) of the sample, wherein (*i*) represents an index number of the (N) compartments represented by the integers 0 to *N -1,* and wherein (*v*$_i$) represents the volume and (*d*$_i$) represents the dilution factor of the sample in the compartment (*i*).

**15.** Use of a kit comprising the sample holder according to claim 14 and one or more reagents suitable for amplifying at least part of particles comprised in the compartments of the sample holder to a measurable signal and optionally one or more suitable diluents for determining a concentration of particles, preferably for determining the concentration of particles in accordance with a method any one of claims 1 to 11.

**Patentansprüche**

**1.** Verfahren zur Bestimmung eines Schätzwerts einer Konzentration von Partikeln *E(C)*, wobei eine Probe mit vorbestimmtem Volumen in eine Anzahl (*N*) von Kompartimenten unterteilt wird, wenigstens ein Teil der Partikel, die in einem der (*N*) Kompartimente vorliegen, ein messbares Signal bereitstellen, und die geschätzte Konzentration von Partikeln *E(C)* eine Funktion gemessener Signale ist, **dadurch gekennzeichnet, dass** das Verfahren Folgendes umfasst oder aus Folgendem besteht:

a) die Anzahl (*N*) getrennter Kompartimente wird bestimmt, wobei die Anzahl (N) kleiner oder gleich dem Wert der Funktion

$$N_{MAX} = A \cdot \ln\left(\frac{C^+}{C^-}\right)/\sigma_{MAX}^2$$

ist, wobei (*A*) eine reelle Zahl darstellt, die die ganze Zahl 6 ist,
wobei (*C*$^+$) eine vorbestimmte Obergrenze des Intervalls der Konzentration (*C*) darstellt, die durch das Verfahren geschätzt werden soll,
wobei (*C*$^-$) eine vorbestimmte Untergrenze des Intervalls der Konzentration (*C*) darstellt, die durch das Verfahren geschätzt werden soll,
wobei ($\sigma_{MAX}$) eine vorbestimmte, maximal zulässige relative Standardabweichung der Schätzung der Konzentration *E(C)* von Partikeln darstellt, wobei $C^- < C < C^+,$ und

b) ein Modulationsfaktor (*z*$_i$) wird bestimmt, wobei (*z*$_i$) eine Funktion von Volumina (*v*$_i$) und Verdünnungsfaktoren (*d*$_i$) der Probe in wenigstens einem Teil der oder allen der (*N*) Kompartimente ist, und die Probe wird in die (*N*) Kompartimente aufgeteilt, wobei wenigstens ein Teil der oder alle der zwei oder mehreren der (N) Kompartimente verschiedene Probenvolumina (*v*$_i$) und/oder verschiedene Verdünnungsfaktoren (*d*$_i$) der Probe umfassen oder daraus bestehen,

wobei (*i*) eine Indexzahl der (*N*) Kompartimente darstellt, dargestellt durch die ganzen Zahlen 0 bis *N - 1,* und
wobei (*v*$_i$) das Volumen darstellt und (*d*$_i$) den Verdünnungsfaktor der Probe in dem Kompartiment (*i*) darstellt.

**2.** Verfahren nach Anspruch 1, wobei der Modulationsfaktor (*z*$_i$) durch die Funktion $z_i = (v_i d_i)/(v_o d_o)$ oder deren Analogon bestimmbar ist, wobei das Analogon Informationen über paarweise Unterschiede in der geschätzten Anzahl von Partikeln in Kompartimenten übermittelt,
wobei (*i*), (*v*$_i$) und (*d*$_i$) wie in Anspruch 1 definiert sind und
wobei (*v*$_o$) das Volumen darstellt und (*d*$_0$) den Verdünnungsfaktor der Probe in einem Referenzkompartiment darstellt, wobei das Referenzkompartiment von dem Kompartiment (*i*) verschieden ist, vorzugsweise wobei das Referenzkompartiment das erste Kompartiment in der Reihe von (N) Kompartimenten darstellt.

3. Verfahren nach Anspruch 1 oder 2, wobei in Schritt b) 1%, vorzugsweise 5%, bevorzugter 25%, noch bevorzugter 50%, noch bevorzugter 75% und am bevorzugtesten 100% der ($N$) Kompartimente sich durch den Wert des Modulationsfaktors ($z_i$) voneinander unterscheiden.

4. Verfahren nach einem der Ansprüche 1 bis 3, wobei in Schritt b) der Wert des Modulationsfaktors ($z_i$) anhand einer wohldefinierten Potenzfolge, einer Exponentialfolge, einer Polynomfolge oder einer geometrischen Folge oder anhand einer Verteilung in der Menge von Kompartimenten bestimmt wird, vorzugsweise vorbestimmt durch Gaußsche Verteilung oder eine Kombination daraus.

5. Verfahren nach Anspruch 4, wobei in Schritt b) die Probe in ($N$) Kompartimente mit solchen Volumina ($v_i$) und Verdünnungsfaktoren ($d_i$) aufgeteilt wird, dass die folgende Bedingung erfüllt ist:

$$(v_{i+1}d_{i+1})/(v_i d_i) = z_{i+1}/z_i = x$$

wobei $x > 0$ und $x \neq 1$, vorzugsweise wobei der Wert von ($x$) durch ungefähr 0,1, 0,5, 0,8, 0,9, 0,91, 0,92, 0,93, 0,94, 0,95, 0,96, 0,97, 0,98, 0,99, 1,01, 1,02, 1,03, 1,04, 1,05, 1,06, 1,07, 1,08, 1,09, 1,1, 1,2, 1,3, 1,4, 1,5, 2 oder 10 dargestellt ist und
wobei vorzugsweise der Quotient $x = (v_{i+1}d_{i+1})/(v_i d_i)$ durch die folgende Funktion bestimmbar ist:

$$x = 1 - \left(\frac{\sigma_{max}}{0,8955}\right)^{1/0,513}$$

die Anzahl ($N$) von Kompartimenten auf eine ganze Zahl festgelegt wird, die nicht kleiner als der Wert ist, der durch die folgende Funktion bestimmbar ist:

$$N = \Delta N + \log_x \left(\frac{C^-}{C^+}\right)$$

wobei ($\Delta N$) eine ganze Zahl ist, die nicht kleiner als der Wert ist, der durch die folgende Funktion bestimmbar ist:

$$\Delta N = 4,5637(1 - x)^{-0,798}$$

und wobei der Wert für $d_o v_o$ durch die Funktion

$$d_o v_o = ln2 \cdot x^{-\Delta N/2} \left(\frac{1}{C^-}\right)$$

bestimmbar ist, wobei ($C^+$), ($C^-$), ($\sigma_{MAX}$), ($d_o$) und ($v_o$) wie in den Ansprüchen 1 und 2 definiert sind.

6. Verfahren nach einem der Ansprüche 1 bis 5, wobei in Schritt b) die Probe in (N) Kompartimente mit solchen Volumina ($v_i$) aufgeteilt wird, dass die folgende Bedingung erfüllt ist: $v_i = A \cdot exp^{(B \cdot \frac{i}{N})}$, wobei ($A$) und ($B$) unabhängig voneinander beliebige reelle Zahlen darstellen und wobei vorzugsweise $A = 1/C^+$ und/oder $B = [1,95 \cdot ln\{(C^+/C^-)/ N\}]^{0,856}$, wobei die Probe vorzugsweise in (N) Kompartimente unterteilt wird,

$$N = 2 \cdot \sigma_{MAX}^{-1,9} \cdot \left(\frac{C^+}{C^-}\right)^{0,25},$$

vorzugsweise

$$N = 2 \cdot \sigma_{MAX}^{-1,94} \cdot \left(\frac{C^+}{C^-}\right)^{0,22},$$

wobei ($C^+$), ($C^-$) und ($\sigma_{MAX}$) wie in Anspruch 1 definiert sind.

**7.** Verfahren nach einem der Ansprüche 1 bis 6, wobei das Verfahren die folgenden Schritte umfasst oder daraus besteht:

c) die Partikel der Kompartimente werden optional mit einem oder mehreren geeigneten Reagenzien und optional einem oder mehreren Verdünnungsmitteln amplifiziert, um ein messbares Signal zu erhalten, das die Gegenwart einer vorbestimmten Schwellenwertanzahl von Partikeln angibt, vorzugsweise ein, zwei, drei oder mehr Partikel in einem Kompartiment,

d) die Signale in jedem der ($N$) Kompartimente werden gemessen, und wenigstens einem Teil, vorzugsweise allen Kompartimenten wird ein Wert ($k_i$) zugewiesen, wobei ($i$) die Indexzahl der Kompartimente darstellt, dargestellt durch ganze Zahlen 0 bis $N$ - 1, und dem Kompartiment ($i$) ein erster Wert ($k_i$) zugewiesen wird, wenn das Kompartiment ($i$) die vorbestimmte Schwellenwertanzahl von Partikeln oder mehr umfasst oder daraus besteht, und dem Kompartiment ($i$) ein zweiter Wert ($k_i$) zugewiesen wird, wenn das Kompartiment ($k_i$) weniger als die Schwellenwertanzahl von Partikeln, die den ersten Wert angeben, umfasst oder daraus besteht, und

e) die geschätzte Konzentration von Partikeln $E(C)$ wird bestimmt, wobei die geschätzte Konzentration von Partikeln $E(C)$ bestimmbar ist durch eine Funktion

i. einer *Summe K* aus den ersten und/oder zweiten Werten ($k_i$), wobei die Funktion vorzugsweise mittels einer Kalibrierungskorrekturfunktion korrigiert wird, und/oder

ii. einer *Summe K* aus gewichteten ersten und/oder zweiten Werten ($w_i k_i$), wobei wenigstens ein Teil der Werte ($k_i$), vorzugsweise jeder Wert ($k_i$) mit einem Gewichtswert ($w_i$) moduliert wird, wobei ($i$) die Indexzahl der Kompartimente darstellt, dargestellt durch die ganzen Zahlen 0 bis $N$ - 1, und wobei der Gewichtswert ($w_i$) vorzugsweise umgekehrt proportional zum Grad des Volumens und/oder der Verdünnung der Anzahl ($i$) der Kompartimente ist und wobei die Funktion vorzugsweise mittels einer Kalibrierungskorrekturfunktion korrigiert wird, und/oder

iii. einer Zahl, eines Vektors, einer Matrix, eines Tensors einer beliebigen Ordnung oder einer beliebigen anderen eindeutigen Darstellung eines *Mikrozustands* $\mu$, wobei die Zahl, der Vektor, die Matrix, der Tensor einer beliebigen Ordnung oder eine beliebige andere Darstellung Informationen umfasst oder aus Informationen besteht, die wenigstens einen Teil der in Schritt e) gemessenen Werte ($k_i$) wiedergeben, vorzugsweise einen den *Mikrozustand* $\mu$ darstellenden Vektor $k \equiv \{k_i\}$ umfasst oder daraus besteht, wobei der Vektor ($k$) wenigstens einen Teil, vorzugsweise alle der in Schritt e) gemessenen Werte ($k_i$) umfasst oder daraus besteht.

**8.** Verfahren nach einem der Ansprüche 1 bis 7, wobei in Schritt b) die Probe in eine Anzahl ($N_{LIB}$) von zwei oder mehreren getrennten Mengen von Kompartimenten (Bibliotheken) aufgeteilt wird, wobei jede Bibliotheksmenge mit ($j$) indiziert wird und $N'j$ > 0 Kompartimente enthält, wobei jedes Kompartiment in der gleichen Bibliothek einen Teil des Probenvolumens mit dem gleichen Wert eines Modulationsfaktors ($z_j$) umfasst oder daraus besteht, wobei $z_j$ eine Funktion von Volumina ($v_j$) und Verdünnungsfaktoren ($d_j$) der Probe in der Bibliotheksmenge ($j$) ist, und wobei die $N_{LIB}$ getrennten Bibliotheksmengen $j$ durch verschiedene Werte des Modulationsfaktors ($z_j$) voneinander unterscheidbar sind, wobei $j$ die Indexzahl der Anzahl ($N_{LIB}$) Bibliotheksmengen darstellt, dargestellt durch die ganzen Zahlen 0 bis $N_{LIB}$ - 1, und

wobei vorzugsweise der Modulationsfaktor ($z_j$) durch die Funktion $z_j = (v_j d_j)/(v_o d_o)$ oder ein Analogon davon bestimmt wird, wobei das Analogon Informationen über paarweise Unterschiede in der geschätzten Anzahl von Partikeln in Kompartimenten übermittelt,

wobei ($v_j$) das Volumen darstellt und ($d_j$) den Verdünnungsfaktor der Probe in den Kompartimenten der Bibliotheksmenge ($j$) darstellt und

wobei ($v_o$) das Volumen darstellt und ($d_0$) den Verdünnungsfaktor der Probe in den Kompartimenten einer ausgewählten Referenzbibliotheksmenge ($j$) darstellt.

**9.** Verfahren nach Anspruch 8, wobei in Schritt b) wenigstens ein Teil, vorzugsweise alle aus der Anzahl ($N_{LIB}$) Bibliotheksmengen ($j$) $N'j = N'$ Kompartimente für jede Bibliotheksmenge ($j$) umfassen oder daraus bestehen und die folgende Bedingung erfüllen:

$$\left(v_{j+1}d_{j+1}\right)/\left(v_j d_j\right) = z_{j+1}/z_j = x$$

wobei $x > 0$ und $x \neq 1$ und wobei vorzugsweise die Funktion:

$$x_{max} = 1 - \left(\frac{\sigma_{max}}{0{,}8955}\right)^{1/0{,}513}$$

den maximalen bevorzugten Wert des Quotienten ($x$), darstellt, wobei der Quotient ($x$) auf die technischen Präferenzen für die Ausführung des Verfahrens abgestimmt werden kann, und die Anzahl $N'j$ von Kompartimenten in jeder Bibliotheksmenge ($j$) auf eine ganze Zahl festgelegt wird, die nicht kleiner als der Wert ist, der durch die folgende Funktion bestimmbar ist:

$$N_j' = \left(\frac{0{,}8955}{\sigma_{max}}\right)^2 (1 - x)^{1{,}026}$$

und die Anzahl ($N_{LIB}$) der getrennten Bibliotheksmengen ($j$) auf eine ganze Zahl festgelegt wird, die nicht kleiner als der Wert ist, der durch die folgende Funktion bestimmbar ist:

$$N_{LIB} = \log_x\left(\frac{C^-}{C^+}\right) + \Delta N_{LIB}$$

wobei

$$\Delta N_{LIB} = 4{,}5637(1 - x)^{-0{,}798}$$

und wobei $d_0^j v_0^j$ durch die Funktion

$$d_0\, v_0 = \ln 2 \cdot x^{-\Delta N/2} \cdot \frac{1}{C^-}.$$

bestimmbar ist, wobei ($C^+$), ($C^-$) und ($\sigma_{MAX}$) wie in Anspruch 1 definiert sind und ($d_o$) und ($v_0$) wie in Anspruch 13 definiert sind.

10. Verfahren nach Anspruch 8 oder 9, wobei wenigstens einem Teil oder vorzugsweise jeder der Anzahl ($N_{LIB}$) Bibliotheksmengen ($j$) ein Wert ($k_i^j$) zugewiesen wird, der eine Funktion wenigstens eines Teils der, vorzugsweise aller Werte ($k_i$) ist, die in Schritt d) jedem der in der Bibliotheksmenge ($j$) enthaltenen Kompartimente zugewiesen werden, und anschließend die Werte ($k_i^j$) in eine Zahl, einen Vektor, eine Matrix, einen Tensor einer beliebigen Ordnung oder eine beliebige andere eindeutige Darstellung eines *Mikrozustands* $\mu$ abgebildet werden, wobei die Zahl, der Vektor, die Matrix, der Tensor einer beliebigen Ordnung oder eine beliebige andere Darstellung Informationen umfasst oder aus Informationen besteht, die wenigstens einen Teil der Werte ($k_i^j$) wiedergeben, vorzugsweise einen den *Mikrozustand* $\mu$ darstellenden Vektor $k_{LIB} \equiv \{k_i^j\}$ umfasst oder daraus besteht, wobei der Vektor ($k_{LIB}$) wenigstens einen Teil, vorzugsweise alle der Werte ($k_i^j$) umfasst oder daraus besteht, wobei vorzugsweise der Wert ($k_i^j$) die Summe der Werte ($k_i$), die in Schritt d) jedem der in der Bibliotheksmenge ($j$) enthaltenen Kompartimente zugewiesen werden, umfasst oder daraus besteht.

11. Verfahren nach einem der Ansprüche 1 bis 10, wobei die Konzentration von zwei oder mehreren verschiedenen Partikeln und/oder zwei oder mehreren verschiedenen Teilen der im Wesentlichen gleichen Partikel bestimmt wird, wobei wenigstens ein Teil, vorzugsweise alle der verschiedenen Partikel und/oder verschiedenen Teile der im Wesentlichen gleichen Partikel, die in einem der (N) Kompartimente vorliegen, zwei oder mehrere unterscheidbare, messbare Signale bereitstellen, vorzugsweise so amplifiziert werden, dass sie diese bereitstellen, wobei die zwei oder mehreren unterscheidbaren Signale in einem der (N) Kompartimente gemessen werden und wobei jedem der (N) Kompartimente jeweils zwei oder mehrere verschiedene Werte zugewiesen werden, wobei die verschiedenen Werte unabhängig voneinander angeben, dass ein Schwellenwert einer Anzahl gleicher Partikel und/oder gleicher Teile der im Wesentlichen gleichen Partikel in dem Kompartiment vorliegt oder nicht.

12. Vorrichtung zur Verwendung bei der Bestimmung einer Konzentration von Partikeln gemäß einem der Ansprüche 1 bis 11, **dadurch gekennzeichnet, dass** die Vorrichtung so eingerichtet ist, dass sie

  a) eine Anzahl (N) getrennter Kompartimente bestimmt, wobei die Anzahl (N) kleiner oder gleich dem Wert der Funktion

$$N_{MAX} = A \cdot \ln\left(\frac{C^+}{C^-}\right) / \sigma_{MAX}^2$$

  ist, wobei (A) eine reelle Zahl darstellt, die die ganze Zahl 6 ist, wobei (C⁺) eine vorbestimmte Obergrenze des Intervalls der Konzentration (C) darstellt, die durch das Verfahren geschätzt werden soll, wobei (C⁻) eine vorbestimmte Untergrenze des Intervalls der Konzentration (C) darstellt, die durch das Verfahren geschätzt werden soll, wobei ($\sigma_{MAX}$) eine vorbestimmte, maximal zulässige relative Standardabweichung der Schätzung der Konzentration $E(C)$ von Partikeln darstellt, wobei $C^- < C < C^+$, und

  b) einen Modulationsfaktor ($z_i$) bestimmt, wobei ($z_i$) eine Funktion von Volumina ($v_i$) und Verdünnungsfaktoren ($d_i$) der Probe in wenigstens einem Teil der oder allen der (N) Kompartimente ist, so dass wenigstens ein Teil der oder alle der zwei oder mehreren der (N) Kompartimente verschiedene Probenvolumina ($v_i$) und/oder verschiedene Verdünnungsfaktoren ($d_i$) der Probe umfassen oder daraus bestehen, wobei (i) eine Indexzahl der (N) Kompartimente darstellt, dargestellt durch die ganzen Zahlen 0 bis N - 1, und wobei ($v_i$) das Volumen darstellt und ($d_i$) den Verdünnungsfaktor der Probe mit vorbestimmtem Volumen in dem Kompartiment (i) darstellt.

13. Verwendung des Verfahrens nach einem der Ansprüche 1 bis 11 oder der Vorrichtung nach Anspruch 12 zur

  a) Verringerung der Gesamtzahl (N) an Kompartimenten, die das vorbestimmte Probenvolumen umfassen, und/oder
  b) Verringerung des Gesamtvolumens einer Mischung, die Probe, ein oder mehrere Reagenzien, das bzw. die zur Amplifikation wenigstens eines Teils der Partikel zu einem messbaren Signal geeignet ist bzw. sind, und optional ein oder mehrere Verdünnungsmittel in allen der (N) Kompartimente umfasst oder daraus besteht, und/oder
  c) Vorbestimmung des Volumens, vorzugsweise des minimalen oder maximalen Volumens der Mischung, die Probe, ein oder mehrere Reagenzien, das bzw. die zur Amplifikation wenigstens eines Teils der Partikel zu einem messbaren Signal geeignet ist bzw. sind, und optional ein oder mehrere Verdünnungsmittel in jedem der (N) Kompartimente umfasst oder daraus besteht, und/oder
  d) Vorbestimmung des Modulationsfaktors ($z_i$), vorzugsweise des minimalen oder des maximalen geeigneten Modulationsfaktors ($z_i$) zur Aufteilung des vorbestimmten Probenvolumens in die (N) Kompartimente.

14. Verwendung eines Probenträgers bei einem Verfahren zur Bestimmung einer Konzentration von Partikeln nach einem der Ansprüche 1 bis 11, **dadurch gekennzeichnet, dass** der Probenträger so eingerichtet ist, dass er

  a) eine vorbestimmte Anzahl (N) von Kompartimenten umfasst oder daraus besteht, wobei die Anzahl (N) kleiner oder gleich dem Wert der Funktion

$$N_{MAX} = A \cdot \ln\left(\frac{C^+}{C^-}\right) / \sigma_{MAX}^2$$

  ist, wobei (A) eine reelle Zahl darstellt, die die ganze Zahl 6 ist, wobei (C⁺) eine vorbestimmte Obergrenze des Intervalls der Konzentration (C) darstellt, die durch das Verfahren geschätzt werden soll, wobei (C⁻) eine vor-

**44**

bestimmte Untergrenze des Intervalls der Konzentration (*C*) darstellt, die durch das Verfahren geschätzt werden soll, wobei ($\sigma_{UMAX}$) eine vorbestimmte, maximal zulässige relative Standardabweichung der Schätzung der Konzentration *E(C)* von Partikeln darstellt, wobei $C^- < C < C^+$, und

b) wobei die (*N*) Kompartimente so eingerichtet sind, dass sie das vorbestimmte Probenvolumen mit einem vorbestimmten Modulationsfaktor ($z_i$) umfassen, wobei ($z_i$) eine Funktion von Volumina ($v_i$) und Verdünnungsfaktoren ($d_i$) der Probe in wenigstens einem Teil der oder allen der (*N*) Kompartimente ist, so dass wenigstens ein Teil der oder alle der zwei oder mehreren der (*N*) Kompartimente verschiedene Probenvolumina ($v_i$) und/oder verschiedene Verdünnungsfaktoren ($d_i$) der Probe umfassen oder daraus bestehen kann bzw. können, wobei (*i*) eine Indexzahl der (*N*) Kompartimente darstellt, dargestellt durch die ganzen Zahlen 0 bis *N - 1,* und wobei ($v_i$) das Volumen darstellt und ($d_i$) den Verdünnungsfaktor der Probe in dem Kompartiment (*i*) darstellt.

**15.** Verwendung eines Kits, der den Probenträger nach Anspruch 14 und ein oder mehrere Reagenzien, das bzw. die zur Amplifikation wenigstens eines Teils von in den Kompartimenten des Probenträgers enthaltenen Partikeln zu einem messbaren Signal geeignet ist bzw. sind, und optional ein oder mehrere geeignete Verdünnungsmittel zur Bestimmung einer Konzentration von Partikeln umfasst, vorzugsweise zur Bestimmung der Konzentration von Partikeln gemäß einem Verfahren nach einem der Ansprüche 1 bis 11.

## Revendications

**1.** Procédé pour la détermination d'une estimation d'une concentration de particules *E(C)*, un échantillon de volume prédéterminé étant divisé en un nombre (*N*) de compartiments, au moins une partie des particules présentes dans l'un des *(N)* compartiments fournissant un signal mesurable, et la concentration estimée de particules *E(C)* étant une fonction de signaux mesurés, **caractérisé en ce que** le procédé comprend ou se compose de

a) la détermination du nombre (*N*) de compartiments séparés, le nombre (*N*) étant inférieur ou égal à la valeur de la fonction

$$N_{MAX} = A \cdot \ln\left(\frac{C^+}{C^-}\right)/\sigma_{MAX}^2$$

(*A*) représentant un nombre réel qui est l'entier 6,
(*C*$^+$) représentant une limite supérieure prédéterminée de l'intervalle de concentration (*C*) à estimer au moyen du procédé,
(*C*$^-$) représentant une limite inférieure prédéterminée de l'intervalle de concentration (*C*) à estimer au moyen du procédé,
($\sigma_{MAX}$) représentant un écart type relatif maximal admissible prédéterminé de l'estimation de la concentration *E(C)* de particules, avec $C^- < C < C^+$, et

b) la détermination d'un facteur de modulation (z$_i$)*,* (z$_i$) étant une fonction de volumes ($v_i$) et de facteurs de dilution ($d_i$) de l'échantillon dans au moins une partie ou dans l'ensemble des (*N*) compartiments, et la division de l'échantillon en les (*N*) compartiments, au moins une partie ou l'ensemble des deux compartiments ou plus des (*N*) compartiments comprenant ou se composant de différents volumes d'échantillon ($v_i$) et/ou de différents facteurs de dilution ($d_i$) de l'échantillon,
(*i*) représentant un chiffre d'indice des (N) compartiments représenté par les entiers de 0 à *N-1,* et
($v_i$) représentant le volume et ($d_i$) représentant le facteur de dilution de l'échantillon dans le compartiment (*i*).

**2.** Procédé selon la revendication 1, dans lequel le facteur de modulation ($z_i$) est apte à être déterminé au moyen de la fonction $z_i = (v_i d_i)/(v_o d_o)$ ou d'un équivalent à celle-ci, l'équivalent transmettant des informations concernant des différences appariées dans le nombre estimé de particules dans des compartiments,
*(i)*, ($v_i$) et ($d_i$) étant définis tel qu'à la revendication 1, et
($v_0$) représentant le volume et ($d_0$) représentant le facteur de dilution de l'échantillon dans un compartiment de référence, le compartiment de référence étant différent du compartiment (*i*), le compartiment de référence représentant de préférence le premier compartiment dans la série de (*N*) compartiments.

**3.** Procédé selon la revendication 1 ou 2, dans lequel à l'étape b), 1%, de préférence 5%, plus préférablement 25%, encore plus préférablement 50%, encore plus préférablement 75% et le plus préférablement 100% des (*N*) compartiments diffèrent les uns des autres par la valeur du facteur de modulation ($z_i$).

4. Procédé selon l'une des revendications 1 à 3, dans lequel à l'étape b), la valeur du facteur de modulation ($z_i$),est déterminée sur la base d'une série de puissances bien définie, d'une série exponentielle, d'une série polynomiale ou d'une série géométrique ou sur la base d'une distribution dans le groupe de compartiments, de préférence prédéterminée par une distribution de Gauss ou une combinaison de celles-ci.

5. Procédé selon la revendication 4, dans lequel à l'étape b), l'échantillon est divisé en ($N$) compartiments avec des volumes ($v_i$) et des facteurs de dilution ($d_i$) tels que la condition suivante soit remplie :

$$(v_{i+1}d_{i+1})/(v_i d_i) = z_{i+1}/z_i = x$$

avec $x > 0$ et $x \neq 1$, la valeur de ($x$) étant de préférence représentée par environ 0,1, 0,5, 0,8, 0,9, 0,91, 0,92, 0,93, 0,94, 0,95, 0,96, 0,97, 0,98, 0,99, 1,01, 1,02, 1,03, 1,04, 1,05, 1,06, 1,07, 1,08, 1,09, 1,1, 1,2, 1,3, 1,4, 1,5, 2, ou 10, et le quotient $x = (v_{i+1}d_{i+1})/(v_i d_i)$ étant de préférence apte à être déterminé par la fonction :

$$x = 1 - \left(\frac{\sigma_{max}}{0.8955}\right)^{1/0.513}$$

le nombre ($N$) de compartiments étant fixé à un entier qui n'est pas inférieur à la valeur apte à être déterminée par la fonction :

$$N = \Delta N + \log_x\left(\frac{C^-}{C^+}\right)$$

$\Delta N$ étant un entier qui n'est pas inférieur à la valeur apte à être déterminée par la fonction :

$$\Delta N = 4.5637(1 - x)^{-0.798}$$

et la valeur pour $d_o v_o$ étant apte à être déterminée par la fonction :

$$d_o v_o = ln2 \cdot x^{-\Delta N/2}\left(\frac{1}{C^-}\right)$$

avec ($C^+$), ($C^-$), ($\sigma_{MAX}$), ($d_o$) et ($v_o$) tels que définis dans les revendications 1 et 2.

6. Procédé selon l'une des revendications 1 à 5, dans lequel à l'étape b), l'échantillon est divisé en ($N$) compartiments avec des volumes ($v_i$) tels que la condition : $v_i = A \cdot exp^{(B \cdot \frac{i}{N})}$ soit remplie, ($A$) et ($B$) représentant indépendamment l'un de l'autre des nombres réels arbitraires, et avec de préférence $A = 1/C^+$ et/ou $B = [1.95 \cdot ln\{(C^+/C^-)/ N\}]^{0.856}$, l'échantillon étant de préférence divisé en ($N$) compartiments

$$N = 2 \cdot \sigma_{MAX}^{-1.9} \cdot \left(\frac{C^+}{C^-}\right)^{0.25},$$

de préférence

$$N = 2 \cdot \sigma_{MAX}^{-1.94} \cdot \left(\frac{C^+}{C^-}\right)^{0.22}$$

avec ($C^+$), ($C^-$) et ($\sigma_{MAX}$) tels que définis à la revendication 1.

7. Procédé selon l'une des revendications 1 à 6, le procédé comprenant ou se composant des étapes suivantes :

c) en option l'amplification des particules des compartiments avec au moins un réactif approprié et en option au moins un diluant pour obtenir un signal mesurable indiquant la présence d'un nombre seuil prédéterminé de particules, de préférence d'une, de deux, de trois particules ou plus dans un compartiment,

d) la mesure des signaux dans chacun des ($N$) compartiments et l'association d'une valeur ($k_i$) à au moins une partie, de préférence à l'ensemble des compartiments, ($i$) représentant le chiffre d'indice des compartiments représenté par des entiers de 0 à $N$ -1, et une première valeur ($k_i$) étant associée au compartiment ($i$) lorsque le compartiment ($i$) comprend ou se compose du nombre seuil prédéterminé de particules ou plus, et une deuxième valeur ($k_i$) étant associée au compartiment ($i$) lorsque le compartiment ($k_i$) comprend ou se compose de moins que le nombre seuil de particules indiquant la première valeur, et

e) la détermination de la concentration de particules estimée $E(C)$, la concentration de particules estimée $E(C)$ étant apte à être déterminée par une fonction

i. d'une somme $K$ des premières et/ou deuxièmes valeurs ($k_i$), la fonction étant de préférence corrigée en employant une fonction de correction de calibrage et/ou

ii. d'une somme $K$ de premières et/ou de deuxièmes valeurs ($w_i k_i$) pondérées, au moins une partie des valeurs ($k_i$), de préférence chaque valeur ($k_i$) étant modulée par une valeur de pondération ($w_i$), ($i$) représentant le chiffre d'indice des compartiments représenté par les entiers de 0 à $N$-1, et la valeur de pondération ($w_i$) étant de préférence inversement proportionnelle au degré de volume et/ou de dilution du nombre ($i$) des compartiments, et la fonction étant de préférence corrigée en employant une fonction de correction de calibrage, et/ou

iii. d'un nombre, d'un vecteur, d'une matrice, d'un tenseur d'un ordre quelconque ou de toute autre représentation univoque d'un *état microscopique* $\mu$, le nombre, le vecteur, la matrice, le tenseur d'un ordre quelconque ou toute autre représentation comprenant ou se composant d'informations reflétant au moins une partie des valeurs ($k_i$) mesurées à l'étape e), comprenant ou se composant de préférence d'un vecteur $k \equiv \{k_i\}$ représentant *l'état microscopique* $\mu$, le vecteur ($k$) comprenant ou se composant d'au moins une partie, de préférence de l'ensemble des valeurs ($k_i$) mesurées à l'étape e).

8. Procédé selon l'une des revendications 1 à 7, dans lequel à l'étape b), l'échantillon est divisé en un nombre ($N_{LIB}$) de deux groupes de compartiments séparés ou plus (bibliothèques), chaque groupe bibliothèque étant pourvu d'un indice ($j$) et contenant $N'j > 0$ compartiments, chaque compartiment dans une même bibliothèque comportant ou se composant d'une partie du volume d'échantillon avec la même valeur d'un facteur de modulation ($z_j$), $z_j$ étant une fonction de volumes ($v_j$) et de facteurs de dilution ($d_j$) de l'échantillon dans le groupe bibliothèque ($j$), et les $N_{LIB}$ groupes bibliothèques séparés $j$ se distinguant les uns des autres par des valeurs différentes du facteur de modulation ($z_j$), $j$ représentant le chiffre d'indice du nombre ($N_{LIB}$) de groupes bibliothèques représenté par les entiers de 0 à $N_{LIB}$ - 1, et

le facteur de modulation ($z_j$) étant de préférence déterminé par la fonction $z_j = (v_j d_j)/(v_o d_o)$ ou un équivalent à celle-ci, l'équivalent transmettant des informations concernant des différences appariées dans le nombre estimé de particules dans des compartiments,

($v_j$) représentant le volume et ($d_j$) représentant le facteur de dilution de l'échantillon dans les compartiments du groupe bibliothèque ($j$), et

($v_o$) représentant le volume et ($d_0$) représentant le facteur de dilution de l'échantillon dans des compartiments d'un groupe bibliothèque ($j$) de référence sélectionné.

9. Procédé selon la revendication 8, dans lequel à l'étape b), au moins une partie, de préférence l'ensemble des nombres ($N_{LIB}$) de groupes bibliothèques ($j$) comprend ou se compose de $N'_j = N'$ compartiments pour chaque groupe bibliothèque ($j$) et remplit la fonction suivante :

$$\left(v_{j+1} d_{j+1}\right)/\left(v_j d_j\right) = z_{j+1}/z_j = x$$

avec x > 0 et x $\neq$ 1, et la fonction :

$$x_{max} = 1 - \left(\frac{\sigma_{max}}{0.8955}\right)^{1/0.513}$$

représentant de préférence la valeur maximale préférée du quotient (x), le quotient (x) étant apte à être ajusté aux préférences techniques pour l'exécution du procédé, et le nombre $N_j'$ de compartiments dans chaque groupe bibliothèque (j) étant fixé à un entier qui n'est pas inférieur à la valeur apte à être déterminée par la fonction :

$$N_j' = \left(\frac{0.8955}{\sigma_{max}}\right)^2 (1-x)^{1.026}$$

et le nombre ($N_{LIB}$) des groupes bibliothèques séparés (j) étant fixé à un entier qui n'est pas inférieur à la valeur apte à être déterminée par la fonction

$$N_{LIB} = \log_x \left(\frac{C^-}{C^+}\right) + \Delta N_{LIB}$$

avec

$$\Delta N_{LIB} = 4.5637(1-x)^{-0.798}$$

et $d_0^j v_0^j$ étant apte à être déterminé par la fonction

$$d_0\, v_0 = \ln 2 \cdot x^{-\Delta N/2} \cdot \frac{1}{C^-}.$$

avec ($C^+$), ($C^-$) et ($\sigma_{MAX}$) tels que définis à la revendication 1 et ($d_0$) et ($v_0$) tels que définis à la revendication 13.

10. Procédé selon la revendication 8 ou 9, dans lequel une valeur ($k_i^j$) qui est une fonction d'au moins une partie, de préférence de l'ensemble des valeurs ($k_i$) associées à chacun des compartiments contenus dans le groupe bibliothèque (j) à l'étape d) est associée à au moins une partie ou de préférence à chacun du nombre ($N_{LIB}$) de groupes bibliothèques (j), et ensuite représentation des valeurs ($k_i^j$) en un nombre, un vecteur, une matrice, un tenseur d'un ordre quelconque ou en toute autre représentation univoque d'un *état microscopique* $\mu$, le nombre, le vecteur, la matrice, le tenseur d'un ordre quelconque ou toute autre représentation comprenant ou se composant d'informations reflétant au moins une partie des valeurs $k_i^j$ comprenant ou se composant de préférence d'un vecteur $k_i^j$ représentant *l'état microscopique* $\mu$, le vecteur ($k_{LIB}$) comprenant ou se composant d'au moins une partie, de préférence de l'ensemble des valeurs $k_i^j$, la valeur $k_i^j$ comprenant ou se composant de préférence de la somme des valeurs ($k_i$) associées à chacun des compartiments contenus dans le groupe bibliothèque (j) à l'étape d).

11. Procédé selon l'une des revendications 1 à 10, dans lequel la concentration de deux particules différentes ou plus et/ou de deux parties ou plus de particules sensiblement identiques est déterminée, au moins une partie, de préférence l'ensemble des particules différentes et/ou des parties différentes de particules sensiblement identiques présentes dans l'un des (N) compartiments fournissant, de préférence étant amplifié(e) de manière à fournir deux signaux mesurables distinguables ou plus, les deux signaux distinguables ou plus dans l'un des (N) compartiments étant mesurés, et deux valeurs différentes ou plus étant respectivement associées à chacun des (N) compartiments, les différentes valeurs indiquant indépendamment les unes des autres qu'un seuil d'un nombre de particules identiques et/ou de parties identiques de particules sensiblement identiques est présent dans le compartiment ou non.

12. Dispositif pour l'utilisation lors de la détermination d'une concentration de particules selon l'une des revendications 1 à 11, **caractérisé en ce que** le dispositif est réalisé de manière à

a) déterminer un nombre *(N)* de compartiments séparés, le nombre *(N)* étant inférieur ou égal à la valeur de la fonction

$$N_{MAX} = A \cdot \ln\left(\frac{C^+}{C^-}\right) / \sigma_{MAX}^2$$

*(A)* représentant un nombre réel qui est l'entier 6, *(C⁺)* représentant une limite supérieure prédéterminée de l'intervalle de concentration (C) à estimer au moyen du procédé, *(C⁻)* représentant une limite inférieure prédéterminée de l'intervalle de concentration (C) à estimer au moyen du procédé, $(\sigma_{MAX})$ représentant un écart type relatif maximal admissible prédéterminé de l'estimation de la concentration $E(C)$ des particule, avec $C^- < C < C^+$, et

b) déterminer un facteur de modulation *(z_i)*, *(z_i)* étant une fonction de volumes *(v_i)* et de facteurs de dilution *(d_i)* de l'échantillon dans au moins une partie ou dans l'ensemble des *(N)* compartiments, de sorte qu'au moins une partie ou l'ensemble des deux compartiments ou plus des *(N)* compartiments comprend ou se compose de différents volumes d'échantillon *(v_i)* et/ou de différents facteurs de dilution *(d_i)* de l'échantillon, *(i)* représentant un chiffre d'indice des *(N)* compartiments représenté par les entiers de 0 à *N - 1*, et *(v_i)* représentant le volume et *(d_i)* représentant le facteur de dilution de l'échantillon de volume prédéterminé dans le compartiment *(i)*.

**13.** Utilisation du procédé selon l'une des revendications 1 à 11 ou du dispositif selon la revendication 12 pour

a) réduire le nombre total *(N)* de compartiments comprenant le volume d'échantillon prédéterminé, et/ou
b) réduire le volume total d'un mélange comprenant ou se composant d'un échantillon, d'au moins un réactif adapté à l'amplification d'au moins une partie des particules en un signal mesurable, et en option d'au moins un diluant dans l'ensemble des *(N)* compartiments et/ou
c) prédéterminer le volume, de préférence le volume minimum ou maximum du mélange comprenant ou se composant de l'échantillon, d'au moins un réactif adapté à l'amplification d'au moins une partie des particules en un signal mesurable, et en option d'au moins un diluant dans chacun des *(N)* compartiments et/ou
d) prédéterminer le facteur de modulation *(z_i)*, de préférence le facteur de modulation *(z_i)* minimum ou maximum pour diviser le volume d'échantillon prédéterminé en les *(N)* compartiments.

**14.** Utilisation d'un support d'échantillon dans un procédé pour la détermination d'une concentration de particules selon l'une des revendications 1 à 11, **caractérisée en ce que** le support d'échantillon est réalisé de manière à

a) comprendre ou à se composer d'un nombre prédéterminé *(N)* de compartiments, le nombre *(N)* étant inférieur ou égal à la valeur de la fonction

$$N_{MAX} = A \cdot \ln\left(\frac{C^+}{C^-}\right) / \sigma_{MAX}^2$$

*(A)* représentant un nombre réel qui est l'entier 6, *(C⁺)* représentant une limite supérieure prédéterminée de l'intervalle de concentration *(C)* à estimer au moyen du procédé, *(C⁻)* représentant une limite inférieure prédéterminée de l'intervalle de concentration *(C)* à estimer au moyen du procédé, $(\sigma_{MAX})$ représentant un écart type relatif maximal admissible prédéterminé de l'estimation de la concentration $E(C)$ de particules, avec $C^- < C < C^+$, et

b) les *(N)* compartiments étant réalisés de manière à comprendre le volume d'échantillon prédéterminé avec un facteur de modulation *(z_i)* prédéterminé, *(z_i)* étant une fonction de volumes *(v_i)* et de facteurs de dilution *(d_i)* de l'échantillon dans au moins une partie ou dans l'ensemble des (N) compartiments, de sorte qu'au moins une partie ou l'ensemble des deux compartiments ou plus des *(N)* compartiments peut comprendre ou se composer de différents volumes d'échantillon *(v_i)* et/ou de différents facteurs de dilution *(d_i)* de l'échantillon, *(i)* représentant un chiffre d'indice des *(N)* compartiments représenté par les entiers de 0 à *N - 1*, et *(v_i)* représentant le volume et *(d_i)* représentant le facteur de dilution de l'échantillon dans le compartiment *(i)*.

**15.** Utilisation d'un ensemble présentant le support d'échantillon selon la revendication 14 et au moins un réactif adapté à l'amplification d'au moins une partie de particules contenues dans les compartiments du support d'échantillon en un signal mesurable et en option au moins un diluant approprié pour déterminer une concentration de particules, de préférence pour déterminer la concentration de particules au moyen d'un procédé selon l'une des revendications 1 à 11.

Fig. 1a)

Fig. 1b)

Fig. 1c)

Fig. 2a)

**Fig. 2b)**

**Fig. 3a)**

**Fig. 3b)**

**Fig. 3c)**

**Fig. 3d)**

Fig. 4a)

Fig. 4b)

Fig. 4c)

**Fig. 5a)**

**Fig. 5b)**

Fig. 6

Fig. 7a)

Fig. 7b)

Fig. 7c)

Fig. 8a)

Fig. 8b)

Fig. 9a)

Fig. 9b)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012100198 A2
- WO 2012109600 A2
- PL 2011050002 W
- PL 395776
- PL 395777
- PL 395778
- PL 398979

- WO 2012049316 A1
- PL 397028
- PL 397027
- PL 397026
- PL 399673
- PL 399908

**Non-patent literature cited in the description**

- *Advan. Physiol. Educ.,* 2005, vol. 29, 151-159
- **SHEN, F. ; SUN, B. ; KREUTZ, J. E. ; DAVYDOVA, E. K. ; DU, W. ; REDDY P. L. ; JOSEPH, L. J. ; IS-MAGILOV, R. F.** Multiplexed Quantification of Nucleic Acids with Large Dynamic Range Using Multivolume Digital RT-PCR on a Rotational SlipChip Tested with HIV and Hepatitis C Viral Load. *J. Am. Chem. Soc.*

- **JOSEPH, L. J. ; ISMAGILOV, R. F.** Multiplexed Quantification of Nucleic Acids with Large Dynamic Range Using Multivolume Digital RT-PCR on a Rotational SlipChip Tested with HIV and Hepatitis C Viral Load. *J. Am. Chem. Soc.*
- **R.F. ISMAGILOV ; F. SHEN ; R. F. ISMAGILOV et al.** *JACS,* 2011, vol. 133, 17705-17712